# EUROPEAN PATENT APPLICATION

(11) **EP 2 090 305 A1**
(43) Date of publication of application: **19.08.2009**
(21) Application number: 07850305.9
(22) Date of filing: 07.12.2007
(51) Int. Cl.: A61K 31/282, A61K 9/127, A61K 33/24, A61K 47/02, A61K 47/20, A61K 47/22, A61K 47/24, A61K 47/28, A61P 35/00

(54) **LIPOSOME ENCAPSULATING AMMINE-PLATINUM COMPLEX AT HIGH CONCENTRATION, AND METHOD FOR PRODUCTION OF THE LIPOSOME**

(30) Priority: 08.12.2006 JP 2006332686
(71) Applicant: Katayama Chemical Industries Co., Ltd., Osaka-shi, Osaka 5410045 (JP)
(72) Inventor: HIRAI, Masahiko, Mino-shi Osaka 562-8686 (JP); IGARASHI, Koichi, Mino-shi Osaka 562-8686 (JP); CHIKUMA, Masahiko, Kyoto-shi Kyoto 606-0803 (JP); OIE, Kazunori, Mino-shi Osaka 562-8686 (JP)
(74) Representative: UEXKÜLL & STOLBERG
(86) International application number: PCT/JP2007/073727
(87) International publication number: WO 2008/072584

(57) **Abstract**

The present invention provides a method of producing a liposome encapsulating an ammine platinum complex. The method includes A) providing a water-soluble ammine platinum complex, platinum complex raw materials or the combination thereof; B) providing a liposome, liposome raw materials or the combination; and C) preparing a mixture of the water-soluble ammine platinum complex, platinum complex raw materials or the combination thereof and the liposome, liposome raw materials or the combination thereof and subjecting it to a liposome-forming/maintaining condition, wherein the salt of the platinum complex forms when the liposome is in a water-soluble form.

## Description

### TECHNICAL FIELD

The present invention relates to a liposome encapsulating an ammine platinum complex (e.g. cisplatin) and a method for producing the same. In particular, it relates to a liposome encapsulating a poor water-soluble ammine platinum complex and a method for producing the same.

### BACKGROUND ART

A liposome has a hollow spherical lipid bilayer membrane structure. It is thus possible to encapsulate a water-soluble medicament inside the lipid bilayer. For that reason, a liposome has been studied and widely used in fields such as pharmaceuticals, agrichemicals, and cosmetics. In particular, liposomes carrying a ligand on the external membrane surface have been studied intensively as a DDS material for delivering a drug or gene selectively to a target site such as cancers or tumors.

However, most of the drugs used in the treatment of cancers and tumors are poorly water-soluble and thus, the amount of the drug encapsulated in a liposome is very small. For example, in the case of cisplatin, since such is not soluble in a system other than aqueous system and has a small saturation concentration of approximately 2 mg/ml in aqueous system at room temperature, there is a disadvantage that the amount of the liposome needed to be administered is inevitably raised in order to administer an effective amount of the drug to a patient in the treatment of cancers or tumors.

Patent Document 1 describes liposome preparations containing an anti-cancer drug such as cisplatin or carboplatin, an antifebrile drug such as aspirin, acetaminophen or indomethacin, a hormone such as cortisone acetate, and the like. However, the liposome preparations described in Patent Document 1 demand a heated step in the production process. It is known that the liposomes are generally unstabilized under heat. Accordingly, the liposomes prepared by the method described in Patent Document 1 are unstable. According to Patent Document 1, the maximum content of cisplatin encapsulated in the liposome is 8.9 µg/mg lipid. Thus, the liposomes prepared by the method described in Patent Document 1 could encapsulate only a small amount of cisplatin.

Nonpatent Literature 1 describes a liposome encapsulating cisplatin. However, the liposome could encapsulate only about 14.0 µg/mg of cisplatin in the lipid.

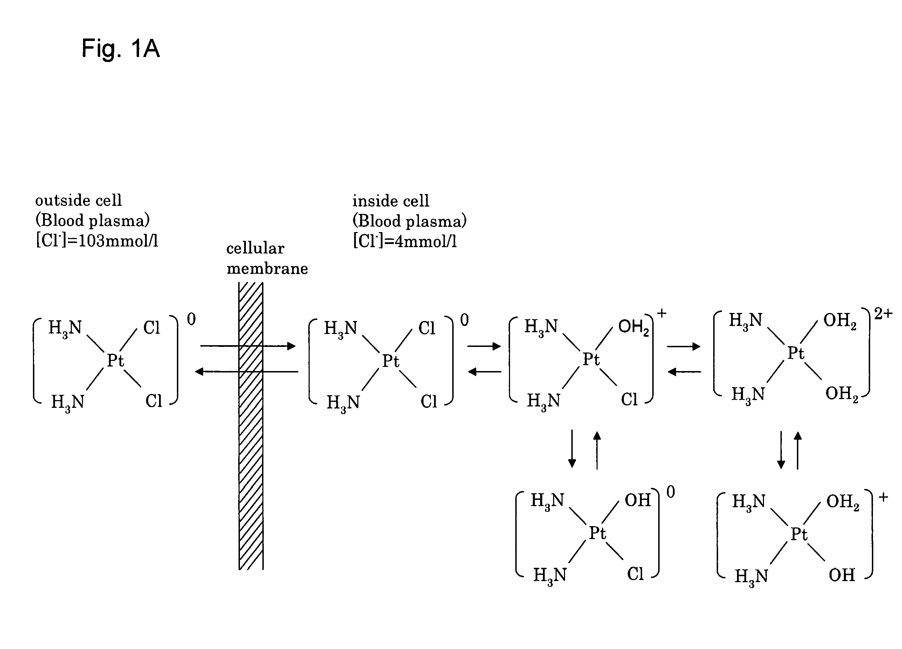

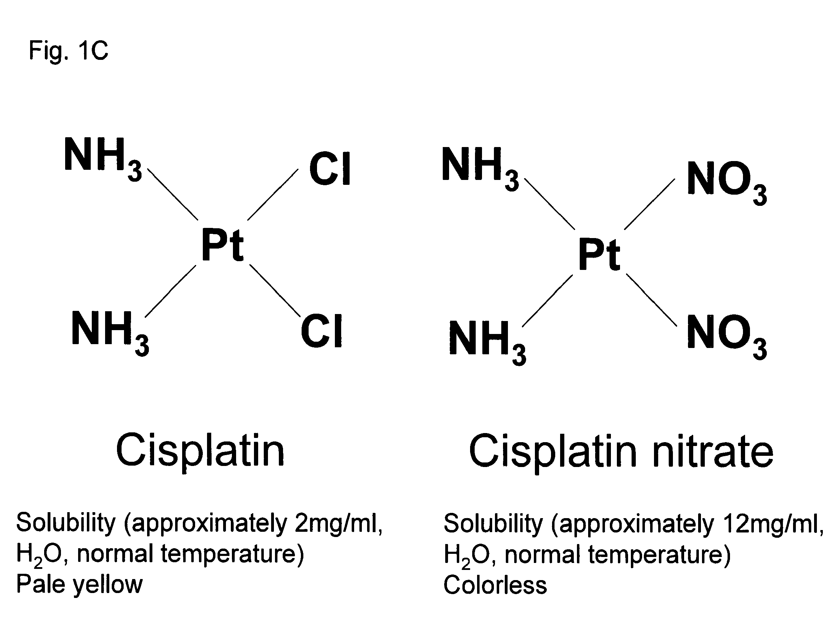

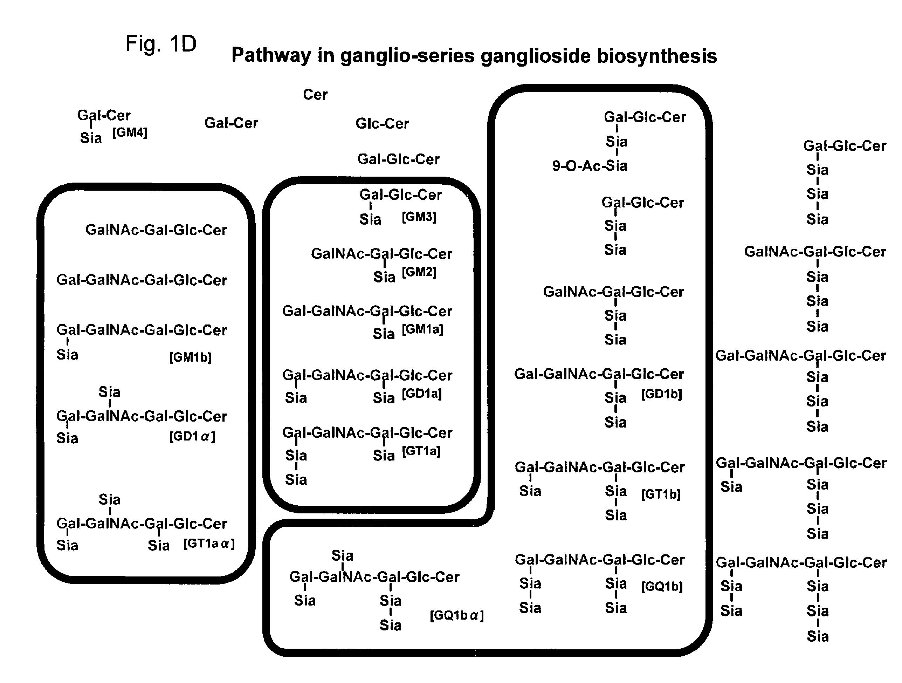

Cisplatin is an anti-cancer drug utilizing the reactivity of a metal complex. Cisplatin, a platinum metal complex, permeates through a cancer cell membrane into a cell in an uncharged state. Chloride ion concentration is 103 mmol/l in blood but it lowers to 4 mmol/l in a cell, and thus, the chloride ions of cisplatin are replaced with water molecules. Therefore, cisplatin is in equilibrium in a cell, as shown in Fig. 1A.

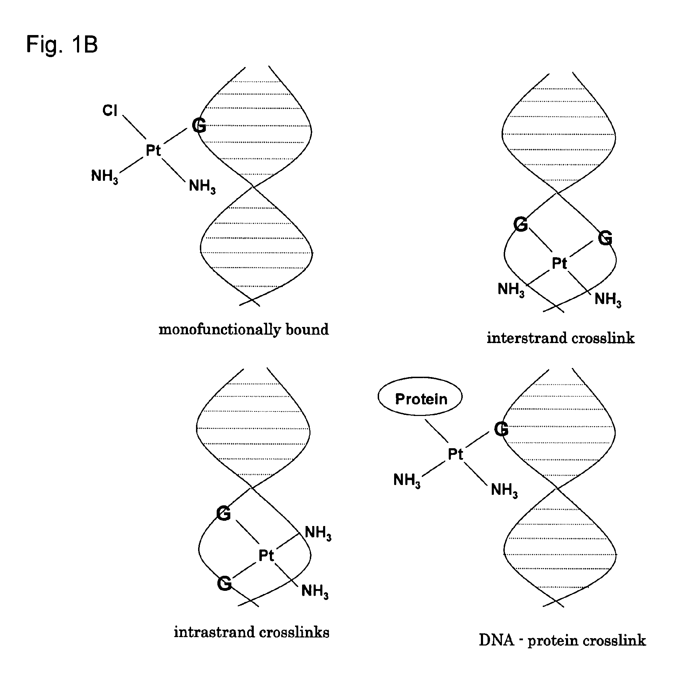

Cisplatin in an equilibrium state binds to DNA in a cell in a manner shown in Fig. 1B, exhibiting an anti-cancer activity by inhibiting DNA replication. However, cisplatin is associated with severe adverse effects such as renal toxicity and hearing loss. Therefore, the development of various cisplatin derivatives and preparations as well as administration methods have been attempted to maintain the anti-cancer activity while reducing these adverse effects.

However, a drug such as cisplatin which has low water solubility is difficult to encapsulate in a liposome at high concentration, and thus, presently, attempts have not been successful in delivering a sufficient amount of the drug to a target site.

Therefore, there exists a need for developing and providing a technique of encapsulating a poorly water-soluble pharmaceutical in liposome in high amounts.
[Patent Document 1]
   Japanese Laid-Open Publication No. 5-255070
[Nonpatent Literature 1]
   Mary S. Newman, Gail T. Colbern, Peter K. Working et al., "Comparative pharmacokinetics, tissue distribution, and therapeutic effectiveness of cisplatin encapsulated in long-circulating, pegylated liposome (SPI-077) in tumor-bearing mice," Cancer Chemother. Pharmacol. (1999) 43: p. 1-7.

### SUMMARY OF THE INVENTION

### Problems to be Solved by the Invention

An object of the present invention is to provide a liposome containing a poorly water-soluble ammine platinum complex which is prepared by encapsulating a poorly water-soluble ammine platinum complex in a liposome in a high amount such that the encapsulated ammine platinum complex can be administered orally in a small amount.

### Means to Solve the Problems

To solve the above problems, the inventors have studied intensively and as a result, have unexpectedly found that, when a water-soluble salt, such as a nitrate of an ammine platinum complex, and a lipid suspension for producing a liposome are mixed in the presence of an ion forming a poorly water-soluble complex salt such as a chloride ion, such results in the formation of a poorly water-soluble complex being contained in the liposome.

To achieve this object, the present invention provides, for example, the following means:

### (Item 1)

A method for producing a liposome encapsulating an ammine platinum complex, including the following steps of:
A) providing a water-soluble ammine platinum complex, a platinum complex raw material or a combination thereof;
B) providing a liposome, a liposome raw material or a combination thereof; and
C) preparing a mixture of the water-soluble ammine platinum complex, the platinum complex raw material or the combination thereof and the liposome, the liposome raw material or the combination thereof and subjecting the mixture under a liposome-forming/maintaining condition, wherein
   a salt formed by the platinum complex is in a water-soluble form at the time point where the liposome exists.

### (Item 2)

The method according to any of the preceding items, further including the step of D) subjecting the mixture obtained in step C) in the presence of a solution containing an ion forming a poorly water-soluble salt with the platinum complex.

### (Item 3)

The method according to any of the preceding items, wherein the water-soluble ammine platinum complex has two ammine groups.

### (Item 4)

The method according to any of the preceding items, wherein the water-soluble ammine platinum complex is cis-diammine dinitratoplatinum (II).

### (Item 5)

The method according to any of the preceding items, including the steps of:
A) dissolving the water-soluble ammine platinum complex in a first buffer solution and thus preparing a platinum complex solution;
B) providing the liposome raw material, including the steps of:
   B-i) obtaining a lipid membrane by suspending a liposome-forming lipid in a methanol-chloroform solution under agitation, evaporating the agitated solution, and vacuum-drying a precipitate; and
   B-ii) suspending the lipid membrane in a second buffer solution and forming a lipid suspension; and
   C) mixing the platinum complex solution with the lipid suspension and subjecting the mixture under the liposome-forming/maintaining condition, wherein the first and second buffer solutions do not contain any ion forming a poorly water-soluble salt with the platinum complex.

### (Item 6)

The method according to any of the preceding items, further including the step of ultrasonicating the lipid suspension after the step B-ii).

### (Item 7)

The method according to any of the preceding items, wherein the liposome-forming/maintaining condition in the step C) is a condition selected from the group consisting of subjecting the mixture of the platinum complex solution and the lipid suspension to ultrafiltration and leaving the mixture to stand overnight.

### (Item 8)

The method according to any of the preceding items, including the steps of:
A) dissolving the water-soluble ammine platinum complex in a first buffer solution and thus preparing the platinum complex solution;
B) providing the liposome; and
C) mixing the platinum complex solution with the liposome and subjecting the mixture under the liposome-forming/maintaining condition, wherein
   the first buffer solution does not contain any ion forming a poorly water-soluble salt with the platinum complex.

### (Item 9)

The method according to any of the preceding items, wherein the liposome in step B) has a composition sufficient for allowing the water-soluble ammine platinum complex to permeate through a liposomal membrane into the liposome and remain therein.

### (Item 10)

The method according to any of the preceding items, wherein the liposome-forming/maintaining condition is a condition sufficient for preserving the liposome without destruction, and at the same time allowing the water-soluble ammine platinum complex to migrate through the liposomal membrane into the liposome and remain therein.

### (Item 11)

The method according to any of the preceding items, wherein the lipid constituting the liposome or the liposome raw material are selected from the group consisting of dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, dipalmitoyl phosphatidylglycerol and combinations thereof.

### (Item 12)

The method according to any of the preceding items, wherein the mixture in the step C) is adjusted to a pH in a range of 6 to 10.

### (Item 13)

The method according to any of the preceding items,
wherein the condition in which the salt formed by the platinum complex is in a water-soluble form is that the mixture is subjected in the presence of a solution which does not contain any ion forming a poorly water-soluble salt with the platinum complex that is selected from the group consisting of a chloride ion (Cl⁻), a bromide ion (Br⁻), an iodide ion (I⁻), a thiocyanate ion (SCN⁻) and a cyanide ion (CN⁻).

### (Item 14)

The method according to any of the preceding items, wherein the chloride ion (Cl⁻) is contained in a range of 0 to 4 mM.

### (Item 15)

The method according to any of the preceding items, wherein the mixture in the step C) contains a buffering agent selected from the group consisting of an N-tris(hydroxymethyl)-3-aminopropane sulfonate buffering agent, a carbonate buffering agent, a phosphate buffering agent, a 2-[4-(2-hydroxyethyl)-1-piperadinyl]ethane sulfonate buffering agent, a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropanesulfonic acid) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent and combinations thereof.

### (Item 16)

The method according to any of the preceding items,
wherein the water-soluble ammine platinum complex, the platinum complex raw material or the combination thereof and the liposome, the liposome raw material or the combination thereof in the step C) are mixed at a ratio in a range of 1:9 to 9:1.

### (Item 17)

The method according to any of the preceding items, wherein the ion forming a poorly water-soluble salt with the platinum complex in step D) is selected from the group consisting of a chloride ion (Cl⁻), a bromide ion (Br⁻), an iodide ion (I⁻), a thiocyanate ion (SCN⁻) and a cyanide ion (CN⁻).

### (Item 18)

The method according to any of the preceding items, wherein the ion forming a poorly water-soluble salt with the platinum complex is a chloride ion (Cl⁻).

### (Item 19)

The method according to any of the preceding items, wherein the chloride ion (Cl⁻) is provided from NaCl, HCl or CaCl₂.

### (Item 20)

The method according to any of the preceding items, wherein the ion forming a poorly water-soluble salt with the platinum complex in step D) is provided from a buffer solution selected from the group consisting of an N-tris(hydroxymethyl)-3-aminopropane sulfonate buffer solution, a carbonate buffer solution, a phosphate buffer, a 2-[4-(2-hydroxyethyl)-1-piperadinyl]ethane sulfonate buffer solution, a tris(hydroxy)aminomethane buffer solution, a 3-(N-morpholino)propane sulfonate buffer solution, an N-tris(hydroxymethyl)1-2-aminoethane sulfonate buffer solution, an N-2-hydroxyethylpiperazine-N'-2ethane sulfonate buffer solution, an N-tris(hydroxymethyl)methyl-2-hydroxy-3-aminopropane sulfonate buffer solution, a piperazine-N,N'-bis(2-hydroxypropanesulfonic acid) buffer solution, an N-2-hydroxyethylpiperazine-N'-2-hydroxypropane-3-propane sulfonate buffer solution, a tris(hydroxymethylmethylglycine) buffer solution, an N,N-bis(2-hydroxyethyl)glycine buffer solution, a 2-(cyclohexylamino)propylethane sulfonate buffer solution, a 3-N-cyclohexylamino-2-hydroxypropane sulfonate buffer solution and a 3-cyclohexylaminopropane sulfonate buffer solution.

### (Item 21)

The method according to any of the preceding items, wherein the chloride ion (Cl⁻) is provided from a buffer solution selected from the group consisting of an N-tris(hydroxymethyl)-3-aminopropane sulfonate buffer solution (pH: 8.4), a carbonate buffer solution (pH: 8.5), a phosphate buffer (pH: 8.0) and a 2-[4-(2-hydroxyethyl)-1-piperadinyl]ethane sulfonate buffer solution (pH: 7.2).

### (Item 22)

The method according to any of the preceding items, wherein the step D) further includes the steps of:
i) subjecting the liposome formed to hydrophilization treatment;
ii) binding a target specific substance to the liposome;
iii) hydrophilizing the modified target specific substance-bound liposome; and
iv) filtering a solution containing the hydrophilized liposome.

### (Item 23)

The method according to any of the preceding items, wherein the target specific substance is selected from the group consisting of antibodies, sugar chains, lectins, complementary nucleic acids, receptors, ligands, aptamers and antigens.

### (Item 24)

The method according to the any of the preceding items, wherein the ammine platinum complex is poorly water-soluble.

### (Item 25)

A method for producing a liposome encapsulating cis-diamine dichloroplatinum (II) according the any of the preceding items, including the following steps of:
(A1) dissolving the cis-diammine dinitratoplatinum (II) in an N-tris(hydroxymethyl)-3-aminopropane sulfonate buffer solution to give a solution containing the cis-diammine dinitratoplatinum (II), wherein the N-tris(hydroxymethyl)-3-aminopropane sulfonate buffer solution does not contain a chloride ion (Cl⁻) or contains a chloride ion (Cl⁻) in a range of 0 to 4 mM;
(A2) adjusting the pH of the solution containing the cis-diammine dinitratoplatinum (II) to 6 to 10;
(B1) preparing a lipid by mixing dipalmitoyl phosphatidylcholine, cholesterol, ganglioside, dicetyl phosphate, dipalmitoyl phosphatidylethanolamine and sodium cholate;
(B2) suspending the lipid in a methanol-chloroform solution under agitation, evaporating the agitated solution and vacuum-drying a precipitate to give a lipid membrane;
(B3) suspending the lipid membrane in an N-tris(hydroxymethyl)-3-aminopropane sulfonate buffer solution (pH: 6 to 10) to prepare a lipid suspension, wherein the N-tris(hydroxymethyl)-3-aminopropane sulfonate buffer solution does not contain a chloride ion (Cl⁻) or contains a chloride ion (Cl⁻) in a range of 0 to 4 mM;
(B4) agitating the lipid suspension at 30°C to 40°C and then ultrasonicating the suspension after nitrogen substitution; and
(C1) mixing the pH-adjusted cis-diammine dinitratoplatinum (II) solution and the ultrasonicated lipid suspension at a ratio in a range of 1:9 to 9:1 and subjecting the mixture to ultrafiltration at a molecular weight cutoff of 500 to 300,000.

### (Item 26)

A method for producing a liposome encapsulating cis-diamine dichloroplatinum (II) according the any of the preceding items, including the following steps of:
(A1) dissolving the cis-diammine dinitratoplatinum (II) in an N-tris(hydroxymethyl)-3-aminopropane sulfonate buffer solution to give a solution containing the cis-diammine dinitratoplatinum (II), wherein the N-tris(hydroxymethyl)-3-aminopropane sulfonate buffer solution does not contain a chloride ion (Cl⁻) or contains a chloride ion (Cl⁻) in a range of 0 to 4 mM;
(A2) adjusting the pH of the solution containing the cis-diammine dinitratoplatinum (II) to 8.4;
(B1) providing a liposome; and
(C1) mixing the pH-adjusted cis-diammine dinitratoplatinum (II) solution and the liposome at a ratio in a range of 1:9 to 9:1 and subjecting the mixture to ultrafiltration at a molecular weight cutoff of 500 to 300,000.

### (Item 27)

A liposome encapsulating an ammonia-containing ammine platinum complex, wherein the liposome can be produced by mixing a water-soluble ammine platinum complex, a platinum complex raw material or a combination thereof and a liposome, a liposome raw material or a combination thereof and subjecting the mixture to liposome-forming/maintaining conditions.

### (Item 28)

The liposome according to any of the preceding items, wherein the liposome can be produced by mixing the water-soluble ammine platinum complex and the liposome and subjecting the mixture to liposome-forming/maintaining conditions.

### (Item 29)

The liposome according to any of the preceding items, wherein the liposome can be produced by mixing the water-soluble ammine platinum complex and the liposome raw material and subjecting the mixture to liposome-forming/maintaining conditions.

### (Item 30)

A liposome encapsulating an ammonia-containing ammine platinum complex, wherein the ammonia-containing ammine platinum complex is contained in an amount of 0.3 pg or more per mg of the lipid.

### (Item 31)

The liposome according to any of the preceding items, wherein the ammonia-containing ammine platinum complex is contained in an amount of 9 µg or more per mg of the lipid.

### (Item 32)

The liposome according to any of the preceding items, wherein the ammonia-containing ammine platinum complex is contained in an amount of 17 µg or more per mg of the lipid.

### (Item 33)

A liposome encapsulating an ammonia-containing ammine platinum complex, wherein an ammonia-containing ammine platinum complex is contained in an amount of 100 pg or more per mg of the phospholipid.

### (Item 34)

A liposome encapsulating an ammonia-containing ammine platinum complex, wherein an ammonia-containing ammine platinum complex is contained in an amount of 39 pg or more per mg of the liposome.

### (Item 35)

A liposome encapsulating an ammonia-containing ammine platinum complex, wherein an ammonia-containing ammine platinum complex is contained in an amount of 3×10⁻¹⁰ pg or more per liposome.

### (Item 36)

The liposome according to any of the preceding items, wherein the ammine platinum complex has two ammonia molecules.

### (Item 37)

The liposome according to any of the preceding items, wherein the ammine platinum complex is cis-diamine dichloroplatinum (II) in a water-soluble form.

### (Item 38)

The liposome according to any of the preceding items, wherein the ammine platinum complex is cis-diamine dichloroplatinum (II).

### (Item 39)

The liposome according to any of the preceding items, wherein the liposome encapsulating an ammine platinum complex includes lipids selected from the group consisting of dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, dipalmitoyl phosphatidylglycerol and combinations thereof.

### (Item 40)

The liposome according to any of the preceding items, wherein the liposome encapsulating an ammine platinum complex further includes a target specific substance.

### (Item 41)

The liposome according to any of the preceding items, wherein the target specific substance is selected from the group consisting of antibodies, sugar chains, lectins, complementary nucleic acids, receptors, ligands, aptamers and antigens.

### (Item 42)

A composition for the treatment of a cancer or a tumor, including a liposome encapsulating an ammonia-containing ammine platinum complex, wherein the liposome is produced by mixing a water-soluble ammine platinum complex, a platinum complex raw material or a combination thereof and a liposome, a liposome raw material or a combination thereof and subjecting the mixture under liposome-forming/maintaining conditions.

### (Item 43)

A composition for the treatment of a cancer or a tumor, including a liposome encapsulating an ammonia-containing ammine platinum complex, in which the ammine platinum complex is contained in an amount of 0.3 µg or more per mg of the lipid.

### (Item 44)

The composition according to the Item, wherein the ammine platinum complex has two ammonia molecules.

### (Item 45)

The composition according to any of the preceding items, wherein the ammine platinum complex is cis-diamine dichloroplatinum (II) in a water-soluble form.

### (Item 46)

The composition according to any of the preceding items, wherein the ammine platinum complex is cis-diamine dichloroplatinum (II).

### (Item 47)

The composition according to any of the preceding items, wherein the liposome encapsulating an ammine platinum complex includes lipids selected from the group consisting of dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, palmitoyl phosphatidylcholine, dipalmitoyl phosphatidylglycerol and combinations thereof.

### (Item 48)

The composition according to any of the preceding items, wherein the liposome encapsulating an ammine platinum complex further includes a target specific substance.

### (Item 49)

The composition according to any of the preceding items, wherein the target specific substance is selected from the group consisting of antibodies, sugar chains, lectins, complementary nucleic acids, receptors, ligands, aptamers and antigens.

### (Item 50)

A method of treating a cancer or a tumor, including the step of administering a liposome encapsulating an ammonia-containing ammine platinum complex to a mammal in need of treating a cancer or a tumor in an effective amount thereof, wherein the liposome is prepared by mixing a water-soluble ammine platinum complex, a platinum complex raw material or a combination thereof and a liposome, a liposome raw material or a combination thereof and subjecting the mixture under liposome-forming/maintaining conditions.

### (Item 51)

A method of treating a cancer or a tumor, including the step of administering a liposome encapsulating an ammonia-containing ammine platinum complex to a mammal in need of treating a cancer or a tumor in an effective amount thereof, wherein the ammine platinum complex in the liposome is contained in an amount of 0.3 pg or more per mg of the lipid.

### (Item 52)

The method according to any of the preceding items, wherein the liposome encapsulating an ammonia-containing ammine platinum complex is administered intravenously, subcutaneously, orally, locally, or intraperitoneally.

### (Item 53)

The method according to any of the preceding items, wherein the liposome encapsulating an ammonia-containing ammine platinum complex is administered at least at a dose of 1.5 µg/g body weight of the platinum complex.

### (Item 54)

The method according to any of the preceding items, wherein the cancer or tumor is selected from the group consisting of testis tumors, bladder cancers, pelvis renalis tumors, ureter tumors, prostate cancers, ovarian cancers, head and neck cancers, non-small cell lung cancers, esophageal cancers, uterine cancers, neuroblastoma and stomach cancers.

### (Item 55)

The method according to any of the preceding items, wherein the liposome encapsulating an ammine platinum complex further includes a target specific substance.

### (Item 56)

The method according to any of the preceding items, wherein the target specific substance is selected from the group consisting of antibodies, sugar chains, lectins, complementary nucleic acids, receptors, ligands, aptamers and antigens.

### (Item 57)

A liposome encapsulating an ammonia-containing ammine platinum complex for use as a medicament, wherein the liposome is prepared by mixing a water-soluble ammine platinum complex, a platinum complex raw material or a combination thereof and a liposome, a liposome raw material or a e combination thereof and subjecting the mixture under liposome-forming/maintaining conditions.

### (Item 58)

A liposome encapsulating an ammonia-containing ammine platinum complex for use as a medicament, wherein the ammine platinum complex is contained 0.3 pg or more per mg of the lipid.

In a certain embodiment, the present invention may be described as follows.

Hereinafter, item A corresponds to any one of A1 to An, and n is a number smaller by 1 than the corresponding number.

### (Item A1)

A method for producing a liposome encapsulating an ammine platinum complex, including the following steps of:
A) providing a solution containing a water-soluble platinum complex having an ammine group;
B) mixing the solution containing a water-soluble platinum complex with a liposome-forming lipid suspension to give a mixture solution;
C) subjecting the mixture solution to liposome-forming conditions; and
D) subjecting the mixture solution to a solution containing an ion forming a poorly water-soluble salt.

### (Item A2)

The method according to item A, wherein the water-soluble ammine platinum complex contains two ammine groups.

### (Item A3)

The method according to any of the preceding items A, wherein the water-soluble platinum complex is cis-diammine dinitratoplatinum (II).

### (Item A4)

The method according to any of the preceding items A, wherein the solution containing a water-soluble platinum complex in the step A) is adjusted to a pH in the range of 6 to 10.

### (Item A5)

The method according to any of the preceding items A, wherein the solution containing a water-soluble platinum complex in the step A) does not contain any ion forming a poor water-soluble salt with the platinum complex.

### (Item A6)

The method according to any of the preceding items A, wherein the ion forming a poorly water-soluble salt is selected from the group consisting of a chloride ion (Cl⁻), a bromide ion (Br⁻), an iodide ion (I⁻), a thiocyanate ion (SCN⁻) and a cyanide ion (CN⁻).

### (Item A7)

The method according to any of the preceding items A, wherein the ion forming a poorly water-soluble salt is a chloride ion (Cl⁻).

### (Item A8)

The method according to any of the preceding items A, wherein the chloride ion (Cl⁻) is contained in the range of 0 to 4 mM.

### (Item A9)

The method according to any of the preceding items A, wherein the solution containing a water-soluble platinum complex in step A) contains a buffering agent selected from the group consisting of an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethanesulfonic acid (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropanesulfonic acid) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent and a 3-cyclohexylaminopropane sulfonate buffering agent.

### (Item A10)

The method according to any of the preceding items A, wherein the solution containing a water-soluble platinum complex contains an N-tris(hydroxymethyl)-3-aminopropane sulfonate buffering agent.

### (Item A11)

The method according to any of the preceding items A, wherein the solution containing a water-soluble platinum complex is adjusted to pH 8.4.

### (Item A12)

The method according to any of the preceding items A, wherein the solution containing a water-soluble platinum complex and the lipid suspension in step B) are mixed at a ratio in the range of 1:9 to 9:1.

### (Item A13)

The method according to any of the preceding items A, wherein the lipid suspension in step B) is produced by the steps of:
i) suspending a lipid in methanol-chloroform solution under agitation, evaporating the agitated solution and vacuum-drying a precipitate to give a lipid membrane; and
ii) suspending the lipid membrane in a suspended buffer solution.

### (Item A14)

The method according to any of the preceding items A, wherein the lipid suspension contains, as the lipid, dipalmitoyl phosphatidylcholine, cholesterol, ganglioside, dicetyl phosphate, dipalmitoyl phosphatidylethanolamine and sodium cholate at a molar ratio of 35:40:15:5:5:167.

### (Item A15)

The method according to any of the preceding items A, wherein the solution containing the water-soluble platinum complex and the lipid suspension are mixed at a ratio of 7:3.

### (Item A16)

The method according to any of the preceding items A, wherein the methanol-chloroform solution contains methanol and chloroform at a ratio of 1:1.

### (Item A17)

The method according to any of the preceding items A, wherein the suspended buffer solution does not contain any ion forming a poorly water-soluble salt with the platinum complex.

### (Item A18)

The method according to any of the preceding items A, wherein the ion forming a poorly water-soluble salt is selected from the group consisting of a chloride ion (Cl⁻), a bromide ion (Br⁻), an iodide ion (I⁻), a thiocyanate ion (SCN⁻) and a cyanide ion (CN⁻).

### (Item A19)

The method according to any of the preceding items A, wherein the ion forming a poorly water-soluble salt is a chloride ion (Cl⁻).

### (Item A20)

The method according to any of the preceding items A, wherein the chloride ion (Cl⁻) is contained in a range of 0 to 4 mM.

### (Item A21)

The method according to any of the preceding items A, wherein the suspended buffer solution contains a buffering agent selected from the group consisting of an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethanesulfonic acid (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropanesulfonic acid) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent and a 3-cyclohexylaminopropane sulfonate buffering agent.

### (Item A22)

The method according to any of the preceding items A, wherein the suspended buffer solution is an N-tris(hydroxymethyl)-3-aminopropane sulfonate buffering agent (pH: 8.4).

### (Item A23)

The method according to any of the preceding items A, further including the step of ultrasonicating the lipid suspension after step B).

### (Item A24)

The method according to any of the preceding items A, wherein the ultrasonication step consists of agitating the lipid solution at 30°C to 40°C and ultrasonicating it after nitrogen substitution.

### (Item A25)

The method according to any of the preceding items A, wherein the mixture solution in step C) is subjected to liposome-forming conditions for period of time sufficient for the preparation of the liposome.

### (Item A26)

The method according to any of the preceding items A, wherein the conditions for forming a liposome from the mixture solution in the step C) are selected from the group consisting of subjecting the mixture solution to ultrafiltration and leaving the mixture solution to stand overnight.

### (Item A27)

The method according to any of the preceding items A, wherein the conditions for forming a liposome from the mixture solution consists of subjecting the mixture solution to ultrafiltration at a molecular weight cutoff of 10,000.

### (Item A28)

The method according to any of the preceding items A, wherein step D) is carried out after the confirmation of liposome formation in step C).

### (Item A29)

The method according to any of the preceding items A, wherein the ion forming a poorly water-soluble salt in step D) is selected from the group consisting of a chloride ion (Cl⁻), a bromide ion (Br⁻), an iodide ion (I⁻), a thiocyanate ion (SCN⁻) and a cyanide ion (CN⁻)

### (Item A30)

The method according to any of the preceding items A, wherein the ion forming a poorly water-soluble salt is a chloride ion (Cl⁻).

### (Item A31)

The method according to any of the preceding items A, wherein the chloride ion (Cl⁻) in step D) is provided from at least one buffer solution selected from the group consisting of an N-tris(hydroxymethyl)-3-aminopropane sulfonate buffer solution (pH: 8.4), a carbonate buffer solution (pH: 8.5), a phosphate buffer (pH: 8.0), a 2-[4-(2-hydroxyethyl)-1-piperadinyl]ethanesulfonic acid (HEPES) buffer solution (pH: 7.2), a tris(hydroxy)aminomethane buffer solution, a 3-(N-morpholino)propane sulfonate buffer solution, an N-tris(hydroxymethyl)1-2-aminoethane sulfonate buffer solution, an N-2-hydroxyethylpiperazine-N'-2ethane sulfonate buffer solution, an N-tris(hydroxymethyl)methyl-2-hydroxy-3-aminopropane sulfonate buffer solution, a piperazine-N,N'-bis(2-hydroxypropanesulfonic acid) buffer solution, an N-2-hydroxyethylpiperazine-N'-2-hydroxypropane-3-propane sulfonate buffer solution, a tris(hydroxymethylmethylglycine) buffer solution, an N,N-bis(2-hydroxyethyl)glycine buffer solution, a 2-(cyclohexylamino)propylethane sulfonate buffer solution, a 3-N-cyclohexylamino-2-hydroxypropane sulfonate buffer solution and a 3-cyclohexylaminopropane sulfonate buffer solution.

### (Item A32)

The method according to any of the preceding items A, wherein the chloride ion (Cl⁻) is provided from at least one buffer solution selected from the group consisting of an N-tris(hydroxymethyl)-3-aminopropane sulfonate buffer solution (pH: 8.4), a carbonate buffer solution (pH: 8.5), a PBS (pH: 8.0) and a HEPES buffer solution (pH: 7.2).

### (Item A33)

The method according to any of the preceding items A, wherein the chloride ion (Cl⁻) is provided from NaCl, HCl or CaCl₂.

### (Item A34)

The method according to any of the preceding items A, wherein the chloride ion (Cl⁻) is provided from NaCl.

### (Item A35)

The method according to any of the preceding items A, wherein step D) includes the steps of:
i) subjecting the liposome formed to hydrophilization treatment;
ii) binding a target specific substance to the liposome;
iii) hydrophilizing the modified target specific substance-bound liposome; and
iv) filtering a solution containing the hydrophilized liposome.

### (Item A36)

The method according to any of the preceding items A, wherein the target specific substance is selected from the group consisting of antibodies, sugar chains, lectins, complementary nucleic acids, receptors, ligands, aptamers and antigens.

### (Item A37)

The method according to any of the preceding items A, wherein the ammine platinum complex is poorly water-soluble.

### (Item A38)

A method for producing a liposome encapsulating an ammine platinum complex, including the following steps of:
A-1) preparing a solution containing a water-soluble platinum complex having two ammine groups;
A-2) adjusting the pH of the solution containing a water-soluble platinum complex-containing solution in the range of 6 to 10;
B-1) mixing the pH-adjusted cis-diammine dinitratoplatinum (II) solution with an ultrasonicated lipid suspension at a ratio in the range of 1: 9 to 9:1 to give a mixture solution;
C-1) subjecting the mixture solution to ultrafiltration; and
D-1) subjecting the mixture solution to the presence of the chloride ion (Cl⁻) after ultrafiltration, wherein the ammine platinum complex is poorly water-soluble.

### (Item A39)

A method for producing a liposome encapsulating cis-diamine dichloroplatinum (II), including the following steps of:
(A1) dissolving the cis-diammine dinitratoplatinum (II) in an N-tris(hydroxymethyl)-3-aminopropane sulfonate buffer solution to give a solution containing a cis-diammine dinitratoplatinum (II), wherein the N-tris(hydroxymethyl)-3-aminopropane sulfonate buffer solution does not contain a chloride ion (Cl⁻) or contains a chloride ion (Cl⁻) in a range of 0 to 4 mM;
(A2) adjusting the pH of the solution containing the cis-diammine dinitratoplatinum (II) to 8.4;
(B1) mixing dipalmitoyl phosphatidylcholine, cholesterol, ganglioside, dicetyl phosphate, dipalmitoyl phosphatidylethanolamine and sodium cholate at a molar ratio of 35:40:15:5:5:167 to prepare a lipid;
(B2) suspending the lipid in a methanol-chloroform (1:1) solution under agitation, evaporating the agitated solution, and vacuum-drying the precipitate to give a lipid membrane;
(B3) suspending the lipid membrane in an N-tris(hydroxymethyl)-3-aminopropane sulfonate buffer solution (pH: 8.4) to prepare a lipid suspension, wherein the N-tris(hydroxymethyl)-3-aminopropane sulfonate buffer solution does not a contain chloride ion (Cl⁻) or contains a chloride ion (Cl⁻) in a range of 0 to 4 mM;
(B4) agitating the lipid suspension at 30°C to 40°C and then ultrasonicating the suspension after nitrogen substitution;
(B5) mixing the pH-adjusted cis-diammine dinitratoplatinum (II) solution with the ultrasonicated lipid suspension at a ratio in a range of 7:3 to give a mixture solution;
(C1) subjecting the mixture solution to ultrafiltration at a molecular weight cutoff of 10,000; and
(D1) exposing the mixture solution after ultrafiltration to at least one buffer solution selected from the group consisting of an N-tris(hydroxymethyl)-3-aminopropane sulfonate buffer solution (pH: 8.4), a carbonate buffer solution (pH: 8.5), a PBS (pH: 8.0) and a HEPES buffer solution (pH: 7.2).

### (Item A40)

A liposome encapsulating an ammine platinum complex, wherein an ammonia-containing ammine platinum complex is contained in an amount of 20 µg or more per mg of the lipid.

### (Item A41)

The liposome according to any of the preceding items A, wherein the ammine platinum complex has two ammonia molecules.

### (Item A42)

The liposome according to any of the preceding items A, wherein the ammine platinum complex is cis-diamine dichloroplatinum (II).

### (Item A43)

The liposome according to any of the preceding items A, wherein the liposome encapsulating an ammine platinum complex contains dipalmitoyl phosphatidylcholine, cholesterol, ganglioside, dicetyl phosphate, dipalmitoyl phosphatidylethanolamine and sodium cholate.

### (Item A44)

The liposome according to any of the preceding items A, wherein the liposome encapsulating an ammine platinum complex contains dipalmitoyl phosphatidylcholine, cholesterol, ganglioside, dicetyl phosphate, dipalmitoyl phosphatidylethanolamine and sodium cholate at a molar ratio of 35:40:15:5:5:167.

### (Item A45)

The liposome according to any of the preceding items A, wherein the liposome encapsulating an ammine platinum complex further includes a target specific substance.

### (Item A46)

The liposome according to any of the preceding items A, wherein the target specific substance is selected from the group consisting of antibodies, sugar chains, lectins, complementary nucleic acids, receptors, ligands, aptamers and antigens.

It should be noted that the steps A and B, in the above description, do not necessarily mean the same steps and are understood appropriately variably according to the present invention.

Therefore, these and other advantages of the present invention will be obvious from the detailed description below with reference to attached drawings.

### Effect of the invention

It is possible to encapsulate a poorly water-soluble ammine platinum complex (e.g., cisplatin), which cannot be encapsulated or has low encapsulation efficiency heretofore, effectively in a liposome and to encapsulate the poorly water-soluble ammine platinum complex (e.g., cisplatin) in a liposome at a high concentration or in a large amount that has not been accomplished in the past. In this manner, it becomes possible to obtain a pharmaceutical effect (e.g. anti-cancer activity) similar to that obtained traditionally using higher dosages of the liposme by administering a liposome preparation containing a poorly water-soluble ammine platinum complex at a dosage far lower than that used in traditional methods.

Hereinafter, preferable embodiments of the present invention will be described, but it should be acknowledged that those skilled in the art can perform the embodiments appropriately and also easily understand the action and effect exhibited by the present invention based on the description of the present invention and well known prior art.

Hereinafter, embodiments of each invention will be described in detail.

### BRIEF DESCRIPTION OF THE DRAWINGS

Fig. 1A is a schematic view illustrating the behavior of cisplatin in a cell. Cisplatin is present in the means of cisplatin chloride in blood plasma where the Cl⁻ ion concentration is 103 mmole, but inside the cell, where the Cl⁻ ion concentration is lower at 4 mmole, cisplatin is in equilibrium with the structures formed by the dissociation of the cisplatin and Cl⁻ ions via the coordination of water molecules.
Fig. 1B is a schematic view illustrating cisplatin binding to DNA. The top left drawing shows cisplatin binding to the guanine base via a single bond. The top right drawing shows cisplatin binding between the double-stranded chains of DNA. The bottom left drawing shows cisplatin binding to a DNA via two single bonds. The bottom right drwing shown cisplatin binding to a guanine base via a single bond and also to a protein.
Fig. 1C shows the chemical structure of cis-diamine dinitratoplatinum (II) (CDDP-3) and cis-diamine dichloroplatinum (II) (CDDP).
Fig. 1D shows the biosynthetic pathway of a ganglio-series ganglioside.
Fig. 2A is a graph showing the results of measuring the particle diameter of the cis-diamine dichloroplatinum (II)-containing liposome prepared in Example 1.
Fig. 2B is a graph showing the results of measuring the particle diameter of the sugar chain-bound cis-diamine dichloroplatinum (II)-containing liposome prepared in Example 3.
Fig. 3 is a schematic chart showing the method of preparing a cisplatin-containing liposome.
Fig. 4A is a UV spectrum obtained when 5 mg/ml of cis-diamine dinitratoplatinum (II) was dissolved in a TAPS buffer solution (pH: 8.4) containing 150 mM sodium chloride and the mixture was left still at 25°C (after 0 minute, 5 minutes, 10 minutes, 15 minutes, 30 minutes and 45 minutes). 1: 0 minutes, 2: after 5 minutes, 3: after 10 minutes, 4: after 15 minutes, 5: after 30 minutes, and 6: after 45 minutes.
Fig. 4B is a UV spectrum of a control which was prepared by dissolving 1 mg of cis-diamine dichloroplatinum (II) in 1 ml of a TAPS buffer solution.
Fig. 5a is a ¹⁹⁵PtNMR spectrum of cisplatin. Cisplatin has a peak at a chemical shift of -2160 ppm.
Fig. 5b is a ¹⁹⁵PtNMR spectrum of (cis-diammine dinitratoplatinum (II)) nitrate. The (cis-diammine dinitratoplatinum (II)) nitrate has a peak at a chemical shift of -1620 ppm.
Fig. 5c is a ¹⁹⁵PtNMR spectrum of (cis-diammine dinitratoplatinum (II)) nitrate-containing liposome (NaCl⁺). The chemical shift of the (cis-diammine dinitratoplatinum (II)) nitrate encapsulated in the liposome was identical with cisplatin occurs at -2160 ppm when the external solution contains NaCl.
Fig. 5d is a ¹⁹⁵Pt-NMR spectrum obtained when liposome-encapsulated cis-diammine dinitratoplatinum (II) was not converted to cis-diamine dichloroplatinum (II) (4.75 mM, heavy water).
Fig. 6 is a graph showing the results of measuring the particle diameter of the antibody-bound cis-diamine dichloroplatinum (II)-containing liposome prepared in Example 5.
Fig. 7 is a photograph of cis-diamine dichloroplatinum (II)-containing liposome I taken under a transmission electron microscope. The liposome prepared by the encapsulation of cis-diammine dinitratoplatinum (II) in liposome I and the subsequent replacement of the solvent with a TAPS buffer solution containing 150 mM sodium chloride was observed under H-7100S (HITACHI). The bar therein represents 100 nm.
Fig. 8 is a ¹⁹⁵PtNMR spectrum of cisplatin. INOVA-600 (Varian) was used for measurement.
Fig. 8a is a ¹⁹⁵PtNMR spectrum of cis-diamine dichloroplatinum (II) (6.6 mM, heavy water).
Fig. 8b is a ¹⁹⁵PtNMR spectrum of cis-diammine dinitratoplatinum (II) (13.7 mM, heavy water).
Fig. 8c is a ¹⁹⁵PtNMR spectrum of cis-diammine dinitratoplatinum (II) encapsulated in liposome and the external solution was replaced with a buffer solution containing 150 mM sodium chloride for the conversion of cis-diammine dinitratoplatinum (II) to cis-diamine dichloroplatinum (II) (4.75 mM, heavy water).
Fig. 8d is a ¹⁹⁵Pt-NMR spectrum of liposome-encapsulated cis-diammine dinitratoplatinum (II) when it was not changed to cis-diamine dichloroplatinum (II) (4.75 mM, heavy water).

### BEST MODE FOR CARRYING OUT THE INVENTION

Hereinafter, the present invention will be described. Throughout herein, an article in the singular (e.g. "a", "an", or "the" in English) should be understood to include a concept of its plural form, unless specified otherwise. The terms used herein should be understood to have the same meaning as those used commonly in the art, unless specified otherwise. If there is a contradiction, the term should be understood as defined herein (including definitions).

### (Definition of terms)

Hereinafter, the terms used particularly herein will be defined.
As described herein, an "ammine platinum complex" is a platinum complex containing ammonia. Examples of the ammine platinum complexes include, but are not limited to: cis-diammine dichloroplatinum (II) <known as cisplatin>, cis-diammine dinitratoplatinum (II), cis-diammine-1,1-cyclobutane-dicarboxylatoplatinum (II) <known as carboplatin>, cis-diammine (glycolato) platinum (II) <known as nedaplatin>, oxalato (1R,2R-cyclohexane diammine) platinum (II) <known as oxaliplatin>, cis-diammine dibromoplatinum (II) <having an anti-cancer activity>, p-hydroxoplatinum (II) <having an anti-cancer activity>, and the like. Examples of the poorly water-soluble ammine platinum complexes include: cis-diammine dichloroplatinum (II), cis-diammine-1,1-cyclobutane-dicarboxylatoplatinum (II), cis-diammine dibromoplatinum (II), and the like. As described herein, "cis-diammine dichloroplatinum (II) (CDDP)" may be referred to as cisplatin. Cisplatin has the following structure:

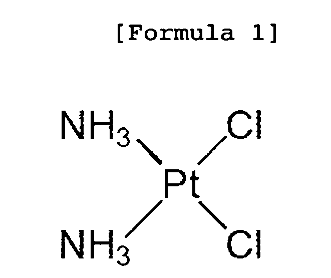

Cisplatin is effective in the treatment of various cancers and tumors. Examples of the cancers and tumors include, but are not limited to: testicular tumors, bladder cancers, renal pelvic/ureteral tumors, prostate cancers, ovarian cancers, head and neck cancers, non-small cell lung cancers, esophagus cancers, uterocervical cancers, neuroblastomas, stomach cancers, small cell lung cancers, osterosarcomas, blastocyte tumors, and the like. Herein, the cancers include all neoplastic diseases such as tumors and leukemia.

"Cis-diamine dichloroplatinum (II) in a water-soluble form" as described herein is cisplatin in a water-soluble form. Examples of the cis-diamine dichloroplatinum (II) in a water-soluble form include cis-diammine dinitratoplatinum (II) (CDDP-3) and the like.

As described herein, the "cis-diammine dinitratoplatinum (II) (CDDP-3)" may also be referred to as cisplatin nitrate. It has the following structure:

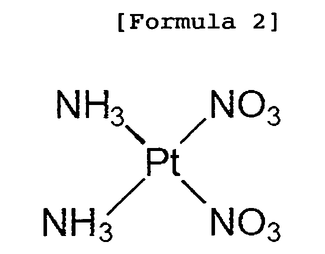

As described herein, the "cis-diammine-1,1-cyclobutane-dicarboxylatoplatinum (II)" will be referred to as carboplatin. It has the following structure:

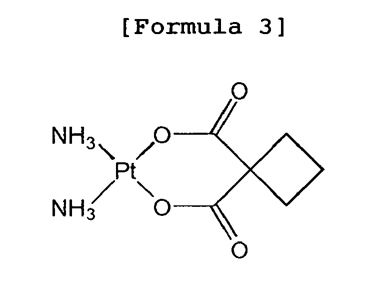

Carboplatin is effective in the treatment of various cancers and tumors. Examples of the cancers and tumors include, but are not limited to: testicular tumors, bladder cancers, renal pelvic/ureteral tumors, prostate cancers, ovarian cancers, head and neck cancers, non-small cell lung cancers, esophagus cancers, uterocervical cancers, neuroblastomas, stomach cancers, small cell lung cancers, osterosarcomas, blastocyte tumors, and the like. Herein, the cancers include all neoplastic diseases such as tumors and leukemia.

As described herein, the "cis-diammine (glucolato)platinum (II)" will be referred to as nedaplatin. It has the following structure:

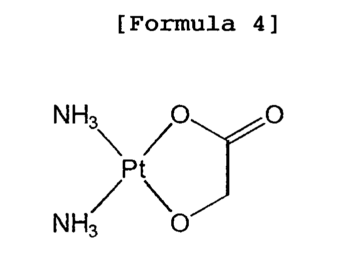

Nedaplatin is effective in the treatment of various cancers and tumors. Examples of the cancers and tumors include, but are not limited to: testicular tumors, bladder cancers, renal pelvic/ureteral tumors, prostate cancers, ovarian cancers, head and neck cancers, non-small cell lung cancers, esophagus cancers, uterocervical cancers, neuroblastomas, stomach cancers, small cell lung cancers, osterosarcomas, blastocyte tumors, and the like. Herein, the cancers include all neoplastic diseases such as tumors and leukemia.

As described herein, the "1R,2R-diammine cyclohexane) oxalato-platinum (II)" is also referred to as oxaliplatin. It has the following structure:

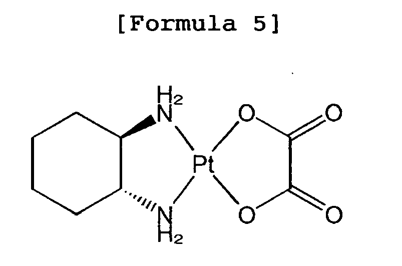

Oxaliplatin is effective in the treatment of various cancers and tumors. Examples of the cancers and tumors include, but are not limited to: testicular tumors, bladder cancers, renal pelvic/ureteral tumors, prostate cancers, ovarian cancers, head and neck cancers, non-small cell lung cancers, esophagus cancers, uterocervical cancers, neuroblastomas, stomach cancers, small cell lung cancers, osterosarcomas, blastocyte tumors, and the like. Herein, the cancers include all neoplastic diseases such as tumors and leukemia.

As described herein, "cis-diammine dibromoplatinum (II)" has the following structure:

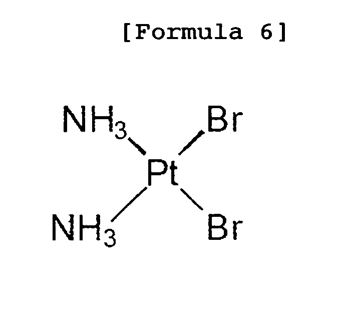

The cis-diammine dibromoplatinum (II) has an anti-cancer activity.

As described herein, the "µ-hydroxoplatinum (II) binuclear complex" includes, for example, the compound of the following structure:

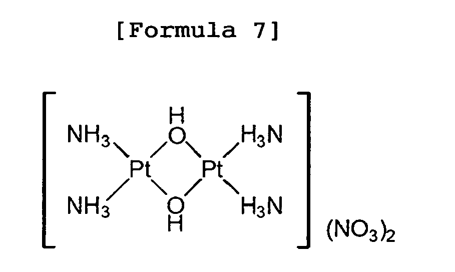

The p-hydroxoplatinum (II) binuclear complex has anti-cancer activity.

In these platinum complexes, the binding between Pt and ammine ligands is strong. Thus, the platinum complexes are present in aqueous solution in a state where the binding portion between Pt and O are dissociated.

In the present invention, it is believed to be important that the platinum complex in a water-soluble form is in contact with the liposome at the time point when the liposome exists as a liposome. Thus, it would be understood that the effect of the present invention would be achieved as long as a platinum complex, even those other than cisplatin, forms a salt having low water solubility in its water-soluble form. Ionic species forming a water-soluble or poorly water-soluble salt with the platinum complexes and other platinum complexes are known in the art or can be selected properly, and thus, it would be understood that it is possible to perform the present invention, even if a platinum complex is that other than cisplatin. In addition, the criterion for selection of the water-soluble complex can be expressed by a solubility of 0.5 g/100 g water to 10 g/100 g water (e.g. any range possible is a combination of 0.5 g/100 g water, 1.0 g/100 g water, 1.5 g/100 g water, 2.0 g/100 g water, 2.5 g/100 g water, 3.0 g/100 g water, 3.5 g/100 g water, 4.0 g/100 g water, 4.5 g/100 g water, 5.0 g/100 g water, 5.5 g/100 g water, 6.0 g/100 g water, 6.5 g/100 g water, 7.0 g/100 g water, 7.5 g/100 g water, 8.0 g/100 g water, 8.5 g/100 g water, 9.0 g/100 g water, 9.5 g/100 g water, or 10 g/100 g water or more (upper limit) and 10 g/100 g water, 9.5 g/100 g water, 9.0 g/100 g water, 8.5 g/100 g water, 8.0 g/100 g water, 7.5 g/100 g water, 7.0 g/100 g water, 6.5 g/100 g water, 6.0 g/100 g water, 5.5 g/100 g water, 5.0 g/100 g water, 4.5 g/100 g water, 4.0 g/100 g water, 3.5 g/100 g water, 3.0 g/100 g water, 2.5 g/100 g water, 2.0 g/100 g water, 1.5 g/100 g water, or 1.0 g/100 g water or less (lower limit) (solubility of water-soluble complexes)), and the criterion for the selection of the poorly water-soluble complex can be expressed by a solubility of 0.0 g/100 g water to 0.5 g/100 g water (e.g., any range possible is a combination of 0.0 g/100 g water, 0.05 g/100 g water, 0.1 g/100 g water, 0.15 g/100 g water, 0.2 g/100 g water, 0.25 g/100 g water, 0.3 g/100 g water, 0.35 g/100 g water, 0.4 g/100 g water, 0.45 g/100 g water, or 0.5 g/100 g water or more (upper limit) and 0.5 g/100 g water, 0.45 g/100 g water, 0.4 g/100 g water, 0.35 g/100 g water, 0.25 g/100 g water, 0.2 g/100 g water, 0.15 g/100 g water, 0.1 g/100 g water, or 0.05 g/100 g water or less (lower limit) (solubility of poor water-soluble complexes)).

As described herein, the "platinum complex raw material" is a raw material giving a water-soluble ammine platinum complex when mixed with a buffer solution. Thus, any material may be used, as long as a water-soluble ammine platinum complex can be formed by mixing, and a plurality of raw materials may be used. Examples of the platinum complex raw materials for use herein include, but are not limited to: cis-[Pt(NH₃)₂I₂], cis-[Pt(NH₃)₂Cl₂], cis-[Pt(NH₃)₂Br₂] and K₂PtCl4. For example, cis-[Pt(NH₃)₂I₂], cis-[Pt(NH₃)₂Cl₂], cis-[Pt(NH₃)₂Br₂], and K₂PtCl₄ are converted to nitrates by the addition of silver nitrate (AgNO₃) in an amount of 2 equivalents (1.98 equivalents).

For example, in the case of cisplatin, the solubility of cisplatin in water is 2 mg/ml (room temperature). The solubility of cisplatin nitrate is 20 mg/ml. Namely, cisplatin can be used as a platinum complex in an amount of not less than its solubility (room temperature). Ionic species forming a water-soluble or a poorly water-soluble salt with the platinum complex such as cisplatin are known in the art or can be selected properly, and thus, even if the platinum complex is that other than cisplatin, it is possible to select a platinum complex properly by considering its solubility, similarly to cisplatin. In this case, it is possible to form nitrates similarly, by adjusting the equivalent amount of silver nitrate added. In addition, concentrations of a bromide ion and an iodide ion are the same as the concentration of a chloride ion respectively; therefore, with any of these ions, the present invention can be carried out without problems. This is because the bromide ion or the iodide ion, which are higher in binding property than the chloride ion, can form a poorly water-soluble substance similarly, even when the present invention is carried out under the same conditions. Since it is believed that there is no difference in liposomal membrane permeability of each ion, the present invention can be carried out by taking these elements into consideration similarly.

The "ammine group," as described herein, is a term used when an ammonia molecule is bound to another group.

The "liposome," as described herein, refers to a closed vesicle composed of a lipid layer normally formed in the membrane shape and an internal aqueous layer. It is also possible to incorporate a cholesterol, glucolipid, and the like in addition to the phospholipids typically used. Since the liposome is a closed vesicle internally encapsulating water, it is also possible to retain a water-soluble agent in such vesicle. Accordingly, such a liposome is used for delivering a pharmaceutical or gene that cannot penetrate a cell membrane into a cell. It is also preferably biocompatible and thus, is expected as a nanoparticle-carrying material for DDS. In the present invention, the liposome may have structural units having a functional group imparting ester binding activity (e.g. glucolipid, ganglioside, or phosphatidylglycerol) or structural units group having a functional group imparting peptide binding activity (e.g. phosphatidylethanolamine) for attaching a modifying group.

The liposome, as used herein may be prepared by any production method, as long as the liposome allows the penetration of the water-soluble ammine platinum complex. In the present invention, a liposome may be used by mixing liposomes prepared by a plurality of production methods (e.g. those from improved cholate method and those from freeze drying method), since a water-soluble ammine platinum complex can be migrated through the liposomal membrane and stay in the liposome. Such a liposome can be determined properly by those skilled in the art according to the application, by examining the encapsulating content and the encapsulating efficiency of the water-soluble platinum complex in the liposome in combination with the constitution of the lipid components, any leakage after encapsulation, liposome stability, and the like. The criteria for determination are as follows.

The encapsulating content is determined by measuring an amount of the platinum in the liposome by atomic absorption spectroscopy (FAAS) and may be the amount of the platinum according to its application.

The encapsulating efficiency can be calculated as a ratio of the amount of the platinum encapsulated in the liposome with respect to an amount of the initial platinum complex. For example, a platinum complex of 0.5% or more may be encapsulated according to its application.

The leakage after encapsulation can be evaluated by determining the amount of the platinum in the liposome quantitatively by atomic absorption spectroscopy (FAAS) over time and comparing the amount with the amount immediately determined after encapsulation.

The liposome stability can be confirmed by measuring particle diameter distribution over time.

The liposomal membrane does not permit the penetration of substances when it is too rigid, while the liposome itself becomes unstable when it is too soft. The rigidity of the liposomal membrane is determined, for example, by the kinds of the liposome constituent components as well as the blending ratio thereof. The rigidity and the fluidity of the liposomal membrane influences the barrier effectiveness of the liposome.

The water-soluble substance remains in the aqueous phase of the liposome, since the lipid bilayer functions as a barrier for the water-soluble substance. The fluidity and the permeability of the membrane are completely different between the gel phase and a liquid crystalline phase. The fluidity in the liquid crystalline phase generally increases as the content of lipids having low phase-transition temperature is increased and also as the temperature is raised. The leakage of the substance enclosed in the aqueous phase in the liposome varies according to the phase transition and the fluidity of the membrane.

Liposomes composed of saturated lipids have high barrier function (hard to permeate, but resistant to leakage) in the gel phase. Liposomes composed of saturated lipids lose their barrier function at or above the phase-transition temperature, but an addition of cholesterol or a small amount of unsaturated fatty acid to the saturated lipid in the liquid crystalline phase leads to recovery of barrier function.

Liposomes composed of unsaturated fatty acids exhibit barrier function in the liquid crystalline phase, but the fluidity and the membrane permeability of small molecules increases, as the temperature is raised. An addition of cholesterol to an unsaturated fatty acid film also enhances the barrier function of the membrane. Specifically, an increase of cholesterol content leads to a deterioration in permeability, but the substance in the internal aqueous phase can be stably maintained.

When cholesterol is contained as a membrane component, the phase transition state and the fluidity of the membrane change significantly. In the unsaturated lipid membrane, cholesterol enhances the interaction among lipid molecules, leading to deterioration in fluidity and membrane permeability. On the other hand, an addition of cholesterol to the saturated lipid leads to elimination of the phase transition, giving a membrane fluidity even at the gel-phase temperature.

These effects are considered to be generated by the formation of a complex between cholesterol and the lipid molecules. Since a presence of cholesterol leads to the elimination of the drastic fluidity change in the phase transition, consequently decrease in the fluidity will take place at or above the phase-transition temperature and, on the other hand increase in the fluidity will take place at or below the phase-transition temperature.

In the liposome used in the present invention when cholesterol is included, it can be contained in a ratio such that the rigidity of the liposomal membrane allows the substance to be enclosed to adequately penetrate the membrane and to remain sufficiently in the liposome (e.g. the cholesterol may be approximately 30 to 50 % by mol, approximately 30 % by mol or more, approximately 35 % by mol or more, approximately 40 % by mol or more, or approximately 45 % by mol or more; and approximately 50 % by mol or less, approximately 45 % by mol or less, approximately 40 % by mol or less, approximately 35 % by mol or less, approximately 50 % by mol or more or approximately 30 % by mol with respect to the phospholipid and any possible range combinations thereof).

The liposome can be prepared by any method known in the art. Examples thereof include an ultrasonication method, an ethanol injection method, a French press method, an ether injection method, a cholate method, a freeze drying method and a reverse phase evaporization method (see e.g., "New development in liposome Application -For development of an artificial cell-, Kazunari Akiyoshi and Kaoru Tsujii Ed., NTS p33 to 45 (2005)" and "Liposomes, Shoshichi Nojima, p. 21-40 Nankodo (1988)").

Among them, for example, the cholate dialysis method is exemplified. In the cholate dialysis method, a production is carried out by a) preparation of a mixed micelle of lipids and a surfactant, and b) dialysis of the mixed micelle. Next in a preferable embodiment of a sugar-chain liposome according to the present invention, a protein is used preferably as the linker, and a glycoprotein in which a protein is bound to a sugar chain is coupled with the liposome in the following two-phase reaction: a) periodate oxidation of the ganglioside portion on the liposomal membrane, and b) coupling of the oxidized liposome with the glycoprotein in a reductive amination reaction. In this way, it is possible to bind a glycoprotein having a desirable sugar chain to the liposome and to obtain various kinds of glycoprotein-liposome conjugates having a desired sugar chain. It is very important to study the particle diameter distribution, for examination of purity and stability of the liposome. As such methods, gel filtration chromatography (GPC), scanning electron microscopy (SEM), dynamic light scattering (DLS), and the like can be used. As an example, it is possible to prepare a liposome containing dipalmitoyl phosphatidylcholine, cholesterol, ganglioside, dicetyl phosphate, dipalmitoyl phosphatidylethanolamine and sodium cholate at a rate of 35:40:15:5:5:167.

Liposomes can by prepared by a freeze-drying method. The freeze-drying method is reported, for example, in H. Kikuchi, N. Suzuki et al., Biopharm. Drug Dispos., 17, 589-605 (1999) and the like. For example, a liposome can be prepared in the following method. A liposome solution is frozen at -40 to -50° C and then is freeze dried. The freeze-dried powder is rehydrated with a solution of the substances to be encapsulated. The substance not to be encapsulated is removed by ultrafiltration or dialysis. Sugars and the like are optionally added to the first liposome solution. In addition, the particle diameter is adjusted by a French press method or a membrane filtration method, in accordance with the desired purpose.

The term "liposome raw material," as used herein refers to a lipid capable of producing a liposome when mixed with a buffer solution. Examples thereof include not only the micellar suspensions used in the Examples, but any liposome raw material may be used, as long as it is a lipid which produces a liposome when in contact with a water-soluble ammine platinum complex and allows permeation of the water-soluble ammine platinum complex. The liposome raw materials used in the production method can be determined properly by those skilled in the art according to the application of the liposome to be produced, by examining an encapsulating content and an encapsulating efficiency of the water-soluble platinum complex in the liposome in combination with the constituting lipid components, the leakage after encapsulation, and liposome stability.

The term "lipid," as used herein, refers to a long-chain aliphatic hydrocarbon or a derivative thereof. The "lipid" is a generic term indicating compounds composed of compounds such as fatty acids , alcohols, amines, amino alcohols and aldehydes. Examples of the liposome-forming lipids or liposome-constituting lipids used herein include: phosphatidylcholines, phosphatidylethanolamines, phosphatidic acids, long-chain alkylphosphate salts, glucolipids (gangliosides and the like), phosphatidylglycerols, sphingomyelins, cholesterols and the like.

Examples of the phosphatidylcholines include dimyristoyl phosphatidylcholine, dipalmitoyl phosphatidylcholine, distearoyl phosphatidylcholine and the like.

Examples of the phosphatidylethanolamines include dimyristoyl phosphatidylethanolamine, dipalmitoyl phosphatidylethanolamine, distearoyl phosphatidylethanolamine and the like.

Examples of the phosphatidic acids include dimyristoyl phosphatidic acid, dipalmitoyl phosphatidic acid, and distearoyl phosphatidic acid. Examples of the long-chain alkylphosphates include dicetyl phosphate and the like.

Examples of the glucolipids include galactosyl ceramides, glycosyl ceramides, lactosyl ceramides, phosphatides, globosides, gangliosides and the like. Examples of the gangliosides include ganglioside GM1 (Galβ1, 3GalNAcβ1, 4(NeuAa2,3)Galβ1, 4Glcβ1, 1'Cer), ganglioside GD1a, ganglioside GT1b, and the like.

Preferable examples of the phosphatidylglycerols include dimyristoyl phosphatidylglycerol, dipalmitoyl phosphatidylglycerol, and distearoyl phosphatidylglycerol.

Among them, phosphatidic acids, long-chain alkylphosphates, glucolipids and cholesterols, which have an effect of enhancing the liposome stability, are preferably contained as constituent lipids. For example, the lipid constituting the liposome used in the present invention is preferably a lipid containing one or more lipids (molar ratio: 0 to 30%) selected from the group consisting of phosphatidylcholines (molar ratio: 0 to 70%), phosphatidylethanolamines (molar ratio: 0 to 30%), phosphatidic acids, and long-chain alkylphosphates; one or more lipids (molar ratio: 0 to 40%), selected from the group consisting of glucolipids, phosphatidylglycerols and sphingomyelins; and cholesterols (molar ratio: 0 to 70%). The lipid preferably contains a ganglioside, a glucolipid or a phosphatidylglycerol, since these lipids facilitate binding a linker such as albumin.

Examples of lipids or liposome raw materials constituting the liposome used in the present invention include, but are not limited to: dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dimyristoyl phosphatidylcholine, distearoyl phosphatidylcholine, dioleoyl phosphatidylcholine, dimyristoyl phosphatidylserine, dipalmitoyl phosphatidylserine, distearoyl phosphatidylserine, dioleoyl phosphatidylserine, dimyristoyl phosphatidylinositol, dipalmitoyl phosphatidylinositol, distearoyl phosphatidylinositol, dioleoyl phosphatidylinositol, dimyristoyl phosphatidylethanolamine, distearoyl phosphatidylethanolamine, dioleoyl phosphatidylethanolamine, dimyristoyl phosphatidic acid, dipalmitoyl phosphatidic acid, distearoyl phosphatidic acid, dioleoyl phosphatidic acid, galactosyl ceramides, glycosyl ceramides, lactosyl ceramides, phosphatides, globosides, GM1 (Galβ1, 3GalNAcβ1, 4(NeuAa2,3)Galβ1, 4Glcβ1, 1'Cer), ganglioside GD1a, ganglioside GD1b, dimyristoyl phosphatidylglycerol, dipalmitoyl phosphatidylglycerol, distearoyl phosphatidylglycerol, dioleoyl phosphatidylglycerol and the like. Preferable are dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, and dipalmitoyl phosphatidylglycerol.

The liposome encapsulating an ammine platinum complex according to the present invention is prepared by the following method. Specifically, the method includes the steps of A) providing a water-soluble ammine platinum complex, a platinum complex raw material or a combination thereof; B) providing a liposome, a liposome raw material or a combination thereof; and C) preparing a mixture of the water-soluble ammine platinum complex, the platinum complex raw material or the combination thereof and the liposome, the liposome raw material or the combination thereof and subjecting the mixture to liposome-forming/maintaining conditions, wherein a salt formed by the platinum complex is in a water-soluble form at the time point where the liposome exists. The production method according to the present invention may include additionally the step of D) subjecting the mixture obtained in step C) to the presence of a solution containing an ion forming a poorly water-soluble salt with the platinum complex.

Preferably, step D) may include the steps of i) subjecting the liposome formed to a hydrophilization method; ii) binding a target specific substance to the liposome; iii) hydrophilizing the modified target specific substance-bound liposome; and iv) filtering a solution containing the hydrophilized liposome.

In the present invention, the water-soluble ammine platinum complex may preferably have two ammine groups, more preferably cis-diammine dinitratoplatinum (II).

The preferable production method may include the following steps of: A) dissolving the water-soluble ammine platinum complex in a first buffer solution and thus preparing a platinum complex solution; B) providing the liposome raw material; and C) mixing the platinum complex solution with the lipid suspension and subjecting the mixture to the liposome-forming/maintaining conditions. In the method, those buffers that do not contain any ion that forms a poorly water-soluble salt with the platinum complex may be used as the first and second buffer solutions.

In one embodiment, step B) of the production method may include the steps of B-i) obtaining a lipid membrane by suspending a liposome-forming lipid in a methanol-chloroform solution under agitation, evaporating the agitated solution, and vacuum-drying the precipitate and B-ii) suspending the lipid membrane in a second buffer solution and forming a lipid suspension.

In a more preferable embodiment, as step A), the production method according to the present invention may include the steps of (A1) dissolving cis-diammine dinitratoplatinum (II) in an N-tris(hydroxymethyl)-3-aminopropane sulfonate buffer solution to give a solution containing the cis-diammine dinitratoplatinum (II), wherein the N-tris(hydroxymethyl)-3-aminopropane sulfonate buffer solution does not contain a chloride ion (Cl⁻) or contains a chloride ion (Cl⁻) in a range of 0 to 4 mM; and (A2) adjusting the pH of the solution containing the cis-diammine dinitratoplatinum (II) to 6 to 10 (preferably 8.4).

Step B) may also include the steps of (B1) preparing a lipid by mixing dipalmitoyl phosphatidylcholine, cholesterol, ganglioside, dicetyl phosphate, dipalmitoyl phosphatidylethanolamine and sodium cholate (preferably, for example, at a molar ratio of 35:40:15:5:5:167); (B2) suspending the lipid in a methanol-chloroform (preferably 1:1) solution under agitation, evaporating the agitated solution and vacuum-drying the precipitate to give a lipid membrane; (B3) suspending the lipid membrane in an N-tris(hydroxymethyl)-3-aminopropane sulfonate buffer solution (pH: 8.4) to prepare a lipid suspension, wherein the N-tris(hydroxymethyl)-3-aminopropane sulfonate buffer solution does not contain a chloride ion (Cl⁻) or contains a chloride ion (Cl⁻) in a range of 0 to 4 mM; and (B4) agitating the lipid suspension at 30° C to 40° C and then ultrasonicating the suspension after nitrogen substitution.

Step C) may also include the steps of (C1) mixing the pH-adjusted cis-diammine dinitratoplatinum (II) solution with the ultrasonicated lipid suspension in a range of 1:9 to 9:1 (preferably, 7:3) and subjecting the suspension to ultrafiltration in the molecular weight cutoff of 500 to 300,000 (preferably, 10,000).

Another preferable production method is a method including the steps of A) dissolving the water-soluble ammine platinum complex in a first buffer solution and thus preparing a platinum complex solution; B) providing the liposome; and C) mixing the platinum complex solution with the liposome and subjecting the mixture under liposome-forming/maintaining conditions. In the method, those that do not contain any ion that forms a poorly water-soluble salt with the platinum complex may be used as the first buffer solution. The liposome can have a composition, for example, sufficient for allowing the water-soluble ammine platinum complex to migrate through the liposomal membrane into the liposome and remain therein. The liposome-forming/maintaining conditions may be, for example, a condition sufficient for maintaining the liposome without destruction and at the same time allowing the migration of the water-soluble ammine platinum complex through the liposomal membrane into the liposome and allowing such to remain therein.

In more another preferable embodiment, step A) of the production method according to the present invention may include the steps of (A1) dissolving cis-diammine dinitratoplatinum (II) in an N-tris(hydroxymethyl)-3-aminopropane sulfonate buffer solution to give a solution containing cis-diammine dinitratoplatinum (II), wherein the N-tris(hydroxymethyl)-3-aminopropane sulfonate buffer solution does not contain chloride ion (Cl⁻) or contains a chloride ion (Cl⁻) in a range of 0 to 4 mM; and (A2) adjusting the pH of the solution containing cis-diammine dinitratoplatinum (II) to be 6 to 10 (preferably, 8.4).

Step B) of the production method according to the present invention may include the steps of (B1) providing a liposome; and (C1) mixing the pH-adjusted cis-diammine dinitratoplatinum (II) solution with the liposome at a ratio in the range of 1:9 to 9:1 and ultrafiltrating the suspension at the molecular weight cutoff of 500 to 300,000 (preferably 10,000).

In one embodiment, the liposome encapsulating an ammine platinum complex according to the present invention may be prepared by the following method. Specifically, the method includes the steps of A) providing a solution containing a water-soluble platinum complex having two ammine group; B) mixing the solution containing the water-soluble platinum complex with a liposome-forming lipid suspension to give a mixture solution; C) subjecting the mixture solution to the liposome-forming conditions; and D) subjecting the mixture solution to the presence of a solution containing an ion forming a poorly water-soluble salt. Preferably, the water-soluble platinum complex according to the present invention may be cis-diammine dinitratoplatinum (II).

A more preferable production method includes the following steps of: A-1) preparing a solution containing a water-soluble platinum complex having two ammine group; A-2) adjusting the pH of the solution containing the water-soluble platinum complex in the range of 6 to 10; B-1) mixing the pH-adjusted cis-diammine dinitratoplatinum (II) solution with the ultrasonicated lipid suspension at a ratio in the range of 1:9 to 9:1 to give a mixture solution; C-1) subjecting the mixture solution to ultrafiltration; and D-1) subjecting the mixture solution in the presence of a chloride ion (Cl⁻) after ultrafiltration.

The liposome encapsulating cis-diamine dichloroplatinum (II) according to the present invention can be prepared by the following method. Specifically, the method includes the steps of (A1) dissolving cis-diammine dinitratoplatinum (II) in N-tris(hydroxymethyl)-3-aminopropane sulfonate buffer solution containing substantially no chloride ion (Cl⁻) to give a solution containing the cis-diammine dinitratoplatinum (II); (A2) adjusting the pH of the solution containing the cis-diammine dinitratoplatinum (II) to be 8.4; (B1) mixing dipalmitoyl phosphatidylcholine, cholesterol, ganglioside, dicetyl phosphate, dipalmitoyl phosphatidylethanolamine and sodium cholate at a molar ratio of 35:40:15:5:5:167 to prepare a lipid; (B2) suspending the lipid in a methanol-chloroform (1:1) solution under agitation, evaporating the agitated solution and vacuum-drying the precipitate to give a lipid membrane; (B3) suspending the lipid membrane in N-tris(hydroxymethyl)-3-aminopropane sulfonate buffer solution (pH: 8.4) containing substantially no chloride ion (Cl⁻) to give a lipid suspension; (B4) agitating the lipid suspension at 30°C to 40°C and then ultrasonicating the suspension after nitrogen substitution; (B5) mixing the pH-adjusted cis-diammine dinitratoplatinum (II) solution with the ultrasonicated lipid suspension at a ratio in the range of 7:3 to give a mixture solution; (C1) subjecting the mixture solution to ultrafiltration at the molecular weight cutoff of 10,000; and (D1) exposing the mixture solution to at least one buffer solution selected from the group consisting of an N-tris(hydroxymethyl)-3-aminopropane sulfonate buffer solution (pH: 8.4), a carbonate buffer solution (pH: 8.5), PBS (pH: 8.0) and a HEPES buffer solution (pH: 7.2) after ultrafiltration.

The term "mixture," as used herein, refers to those containing a water-soluble ammine platinum complex, a platinum complex raw material or a combination thereof (herein, also referred to as a water-soluble ammine platinum complex or the like) and a liposome, a liposome raw materials or a combination thereof (herein, also referred to as liposome or the like).

The term "mixture solution," as used herein, refers to a solution prepared by mixing a solution containing a water-soluble platinum complex with a liposome-forming lipid suspension.

The liposome, liposome raw material or the combination thereof; the platinum complex, platinum complex raw materials or the combination thereof; and optionally a buffer solution may be mixed in any order. For example, the order may be, but is not limited to, 1) mixing the liposome raw materials and the platinum complex and subsequently adding the buffer solution, 2) mixing the liposome raw materials and the buffer solution and subsequently adding the platinum complex, or 3) dissolving the platinum complex in the buffer solution and subsequently adding the liposome raw materials. The order can vary because the present invention can be achieved as long as the liposome-forming/maintaining conditions is provided, i.e. as long as the water-soluble ammine platinum complex can be brought into contact with the liposome at the time point where the liposome exists, though they can be mixed in any order. When a platinum complex strongly acidic in aqueous solution is used, the pH of the mixture liquid is preferably in a range of 6 to 10.

Thus, the present invention can be achieved as long as the liposome-forming/maintaining conditions are satisfied.

Without wishing to be bound by theory, the platinum complex strongly acidic in an aqueous solution (including cisplatin nitrate) should be resuspended in an alkaline buffer solution, when a mixture of lipid components and cisplatin nitrate is re-dissolved in aqueous solution, and the conditions should be considered because the lipid decomposes under acidic condition. As for the amount of the alkali buffer solution needed, when 80 mg of cisplatin nitrate is dissolved in 4 ml of TAPS (pH: 8.4), approximately 350 µl of 1 N NaOH is added.

In the production method according to the present invention, it is possible to prepare the liposome encapsulating an ammine platinum complex according to the present invention by subjecting the mixture under the liposome-forming/maintaining conditions wherein a salt formed by the platinum complex is in a water-soluble form at the time point where the liposome exists. The pH of the mixture should be in the range of pH 6 to 10 because it is possible to form and/or retain a liposome at a pH close to neutral.

The term "liposome-forming/maintaining conditions," as used herein, means conditions for forming and/or maintaining a liposome.

The term "liposome-forming" conditions, as used herein, means conditions for forming a liposome in the mixture (e.g., mixture solution). The liposome-forming conditions may be, for example, ultrafiltration of the mixture, leaving the mixture to stand overnight, or the like. More preferably, the liposome-forming conditions may be ultrafiltration of the mixture at a molecular weight cutoff of 500 to 300,000 (preferably, 10,000).

The term "liposome-maintaining" conditions, as used herein, means conditions for maintaining the liposome in the mixture (e.g., mixture solution). Without wishing to be bound by theory, for example, the liposome-maintaining conditions are based on the following. That a liposome deteriorates and is not maintained at a pH of 6.0 or less. When adding a surfactant to the liposome, the liposome deteriorates and thus is not maintained. The liposome also deteriorates and thus is not maintained by physicochemical forces such as ultrasonic waves. A high temperature of 60°C or higher is also destructive and disrupts the liposome. When the external solution contains Cl⁻, Br⁻, I-, SCN⁻, CN⁻ or a nucleobase (guanine or cytosine), the platinum complex cannot exist in the water-soluble platinum complex state or is inactivated (possibly caused by binding thereof to the N7-nitrogen in the guanine base.) Thus these conditions should be considered.

The term "conditions wherein the salt formed by the platinum complex is water-soluble," as used herein, means conditions wherein the salt formed by the platinum complex is in the water-soluble form in the mixture. For example, such conditions are that the liquid mixture is subjected to a solution that does not contain any ion forming a poorly water-soluble salt with the platinum complex.

The term "does not contain any ion forming a poorly water-soluble salt with the platinum complex, " as used herein, means there is substantially no ion in the solution with which the platinum complex can form a poorly water-soluble salt. Examples of the ions giving a poorly water-soluble salt include a chloride ion (Cl⁻), bromide ion (Br⁻), iodide ion (I⁻), thiocyanate ion (SCN⁻), cyanide ion (CN⁻) and the like. The solution may not contain any ion forming a poorly water-soluble salt with the platinum complex or may contain such an ion in an amount of forming the poorly water-soluble salt with platinum complex even when the ion is present

The term "substantially containing no poorly water-soluble salt," as used herein, means that the ion may be contained at a concentration not forming a poorly water-soluble salt. For example, if the ion forming a poorly water-soluble salt is a chloride ion (Cl⁻), the concentration may be a concentration not higher than that in the body (approximately 0 to 4 mM). The concentration at which the poorly water-soluble salt is formed can be determined experimentally by a method including the following steps of:
1) preparing an aqueous solution (6 mM) containing an ammine platinum complex;
2) preparing solutions containing the ion forming a poorly water-soluble salt at various concentrations (final concentrations: 0 to 200 mM);
3) adding the solutions containing the ion forming a poorly water-soluble salt obtained in the step 2) to the ammine platinum complex-containing aqueous solution in step 1) until the ion forms a poorly water-soluble salt; and
4) calculating the concentration at which the poorly water-soluble salt is formed from the addition amount of the ion-containing solution.

The concentration of other salts at which no poorly water-soluble salt is formed is defined similarly.

The term "water-soluble," as used herein, means a property of being soluble in water. The ammine platinum complex encapsulated in the liposome prepared by the method of producing the ammine platinum complex-containing liposome according to the present invention is preferably poorly water-soluble. The term "poorly water-soluble," as used herein, is a property of being insoluble or scarcely soluble in water.

The "first buffer solution" herein means a buffer solution for dissolving a water-soluble ammine platinum complex, materials for the platinum complex, or the combination thereof.

In the production method according to the present invention, a buffer solution that does not contain any ion forming a poorly water-soluble salt with the platinum complex is used as the first buffer solution. The first buffer solution preferably does not contain any ion forming a poorly water-soluble salt or contains it in an amount not forming a poorly water-soluble salt with the platinum complex even when the ion is present. Examples of the ions forming a poorly water-soluble salt include a chloride ion (Cl⁻), bromide ion (Br⁻), iodide ion (I⁻), thiocyanate ion (SCN⁻) , cyanide ion (CN⁻) and the like. The first buffer solution for is, for example, a buffer solution containing an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethane sulfonate (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropane sulfonate) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent or the like. Preferably, the first buffer solution may be a buffer solution containing an N-tris(hydroxymethyl)-3-aminopropane sulfonate buffering agent (preferably, pH 8.4).

The "platinum complex solution," as described herein, means a water-soluble platinum complex-containing solution prepared by dissolving a water-soluble ammine platinum complex in the first buffer solution. For example, in the case of cisplatin nitrate, the concentration of platinum in the platinum complex solution can be set in the range of 15 mM to 300 mM, based on the solubility of cisplatin nitrate (molecular weight: 353). Such a water-soluble platinum complex-containing solution is known in the art, and can be determined easily by traditional experiments and the technical common knowledge in the art. Accordingly, such a solution is prepared easily by those skilled in the art.

The term "water-soluble platinum complex-containing solution," as used herein, means any solution containing a water-soluble platinum complex. The water-soluble platinum complex-containing solution may be adjusted to a pH in the range of 6 to 10 (preferably, 8.4). The water-soluble platinum complex-containing solution may be a solution not containing any ion forming a poorly water-soluble salt with the platinum complex.

The water-soluble platinum complex-containing solution for use in the present invention may contain, for example, an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethane sulfonate (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropane sulfonate) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent, or the like. Preferably, the water-soluble platinum complex-containing solution may contain an N-tris(hydroxymethyl)-3-aminopropane sulfonate buffering agent (pH: 8.4). The "platinum complex solution" or the "water-soluble platinum complex-containing solution" may contain buffering agents other than those above, as long as the ammine platinum complex is in the water-soluble form only when the liposome is present. Such a buffering agent can have a buffering ability at pH 6 to 10 and can maintain the liposome and the water-soluble platinum complex if it does not contain anions having a binding property with the water-soluble complex.

The term "lipid suspension," as used herein, is a suspension of a lipid having a liposome-forming ability, and any liquid containing suspended lipids forming a liposome when subjected to liposome-forming conditions. The lipid suspension, when the lipid is suspended in the membrane state, is also called "lipid membrane suspension." In addition, when the lipid is dissolved in a solvent, the solution is also called the "lipid solution." The lipid solution may contain broadly a liquid in the lipid suspension state.

As will be described below, the composition of such a "suspension of lipid having a liposome-forming ability" and the range thereof can be determined arbitrarily by those skilled in the art. The reasons are as follows: The lipid suspension for use in the present invention is prepared by using lipids commonly used in the preparation of the liposomes. Examples of the lipids commonly used in preparation of the liposomes include phosphatidylcholine, cholesterol and the like. Examples of the lipids alternatively used in preparation of the liposomes include, but are not limited to: phosphatidylethanolamine, ganglioside, sodium cholate, dicetyl phosphate, phosphatidylserine, phosphatidylinositol, diphosphatidylglycerol, cardiolipin, sulfoxyribosyl diglyceride, digalactosyl diglyceride, galactosyl diglyceride and the like. Preferably, the lipid suspension for use in the present invention may have a composition in the following range:
Phosphatidylcholine: 0 to 50 mM
Phosphatidylethanolamine: 0 to 3.3 mM
Cholesterol: 0 to 26.1 mM
Ganglioside: 0 to 9.3 mM
Sodium cholate: 0 to 108.9 mM
Dicetyl phosphate: 0 to 3.29 mM.

The suspension of lipid having a liposome-forming ability can be prepared by the steps of: B-i) suspending a lipid, liposome raw materials, or combinations thereof in a methanol-chloroform solution (preferably, 1:1) under agitation, evaporating the agitated solution and vacuum-drying the precipitate to give a lipid membrane; and B-ii) suspending the lipid membrane in a second buffer solution (suspended buffer solution). The lipid suspension may be ultrasonicated. The lipid suspension may contain, as the lipid, dipalmitoyl phosphatidylcholine, cholesterol, ganglioside, dicetyl phosphate, dipalmitoyl phosphatidylethanolamine and sodium cholate (preferably, at a molar ratio of 35:40:15:5:5:167). In the present method, the solution containing the water-soluble ammine platinum complex and the lipid suspension are mixed at a ratio in a range of 1:9 to 9:1 (preferably, 7:3).

As described herein, the "second buffer solution" is a buffer solution for solubilization of the lipid suspension. The second buffer solution may be called "suspension buffer solution" herein.

In the production method according to the present invention, buffers that do not contain any ion forming a poorly water-soluble salt with the platinum complex may be used as the second buffer solution. The suspended buffer solution is a solution containing no ion forming a poorly water-soluble salt or containing the ion only in an amount such that it is not forming the poorly water-soluble salt with a platinum complex even when the ion is present. Examples of the ions forming a poorly water-soluble salt include a chloride ion (Cl⁻), bromide ion (Br⁻), iodide ion (I⁻), thiocyanate ion (SCN⁻), cyanide ion (CN⁻) and the like. The second buffer solution for use may be, for example, a buffer solution containing an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethane sulfonate (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropane sulfonate) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent or the like. Preferably, the second buffer solution may be a buffer solution containing an N-tris(hydroxymethyl)-3-aminopropane sulfonate buffering agent (preferably, pH 8.4).

The "second buffer solution" or the "suspended buffer solution" may contain buffering agents other than those above, as long as the ammine platinum is in the water-soluble form only when the liposome is present. Such a buffering agent can have a buffering ability at pH 6 to 10 and can maintain the liposome and the water-soluble platinum complex if it does not contain anions having a binding property with the water-soluble complex.

As described herein, the first and second buffer solutions may have the same composition or different compositions because these buffer solutions are not particularly limited, as long as they are a buffer solution in which the platinum complex remains in the water-soluble form when the liposome is present.

The phrase "in the presence of a solution containing an ion forming a poorly water-soluble salt with the platinum complex," as used herein, means subjecting an environment providing an ion forming a poorly water-soluble salt with the platinum complex.

In a preferable embodiment, the phrase "in the presence of a solution containing an ion forming a poorly water-soluble salt with the platinum complex" may mean in the presence of chloride ion (Cl⁻).

In the present production method, the ion forming a poorly water-soluble salt with the platinum complex (e.g., chloride ion (Cl⁻)) can be provided from a N-tris(hydroxymethyl)-3-aminopropane sulfonate buffer solution (pH: 8.4), a carbonate buffer solution (pH: 8.5), a PBS (pH: 8.0) or a HEPES buffer solution (pH: 7.2), a tris(hydroxy)aminomethane buffer solution, a 3-(N-morpholino)propane sulfonate buffer solution, an N-tris(hydroxymethyl)1-2-aminoethane sulfonate buffer solution, an N-2-hydroxyethylpiperazine-N'-2-ethane sulfonate buffer solution, an N-tris(hydroxymethyl)methyl-2-hydroxy-3-aminopropane sulfonate buffer solution, a piperazine-N,N'-bis(2-hydroxypropane sulfonate) buffer solution, an N-2-hydroxyethylpiperazine-N'-2-hydroxypropane-3- propane sulfonate buffer solution, a tris(hydroxymethylmethylglycine) buffer solution, an N,N-bis(2-hydroxyethyl)glycine buffer solution, a 2-(cyclohexylamino)propylethane sulfonate buffer solution, a 3-N-cyclohexylamino-2-hydroxypropane sulfonate buffer solution, a 3-cyclohexylaminopropane sulfonate buffer solution or the like, but is not limited thereto. A buffering agent can have a buffering ability at pH 6 to 10 and can maintain the liposome and the water-soluble platinum complex if it does not contain anions having a binding property with the water-soluble complex. General preparative methods and characteristics of the buffer solution are known in the art, and can be determined easily by traditional experiments and the technical common knowledge in the art. Thus, those skilled in the art can select a buffer solution containing an ion forming a poorly water-soluble salt with the platinum complex properly, taking into consideration the intended poorly water-soluble salt of an ammine platinum complex, the pH, the ion contained in the buffer solution, and the like.

In the present method, a chloride ion (Cl⁻) is provided from NaCl, HCl, or CaCl₂, but is not limited thereto. Preferably, the chloride ion (Cl⁻) is provided from NaCl.

In a preferable embodiment, it is possible to make the liposome according to the present invention bind a target specific substance (e.g., antibody, sugar chain, lectin, complementary nucleic acid, receptor, ligand, aptamer, antibody, or the like) by making it contain a ganglioside, glucolipid or phosphatidylglycerol and binding a linker such as a peptide.

The term "target specific substance," as used herein, means a substance specifically recognizing a disease (in particular, tumor). Examples of the "target specific substances" for use in the present invention include, but are not limited to: antibodies, sugar chains, lectins, complementary nucleic acids, receptors, ligand aptamers, antibodies, and the like. The target specific substance may be any substance as long as it provides the liposome with target specificity without destruction of the liposome. Those skilled in the art can determine the target specific substance to be bound to the liposome properly depending on the target substance. It is possible to modify the liposomal membrane surface with any substance by using a suitable crosslinking agent.

The term "antibody," as used herein, means an immunoglobulin molecule having a specific amino acid sequence induced by an antigen, which is an immunogen. Antibodies are produced by B cells and are present in the blood and body fluids. Characteristically, an antibody reacts with an antigen specifically. Antibodies may be present naturally, not being generated as a result of the stimulus caused by exposure to antigens. Fundamentally, an antibody has a molecular structure composed of two light chains and two heavy chains, and it is also present as a dimer, trimer or pentamer. Examples thereof include, but are not limited to, Ig A, Ig E, Ig M, Ig G, and the like.

As described herein, the "sugar chain" means a compound having one or more monosaccharides (monosaccharides and/or the derivatives thereof) connected to each other. When two or more monosaccharides are bound to each other, the monosaccharides are bound to each other via a glycoside bond formed by dehydration condensation. Examples of the sugar chains include, but are not limited to: degraded wide sugar chains or induced from degraded polysaccharides and composite biological molecules such as glycoproteins, proteoglycan, glycosaminoglycans, glucolipids and the like in addition to polysaccharides contained in the living body (glucose, galactose, mannose, fucose, xylose, N-acetylglucosamine, N-acetylgalactosamine, sialic acid, as well as the complexes and derivatives thereof). Thus, in the present specification, the term sugar chains are used interchangeably as "polysaccharides," "sugars," and "carbohydrates." Also as described herein, the "sugar chain" may mean both a sugar chain and a sugar chain-containing other substance, unless specified otherwise. Typically, sugar chains are linear polymers consisting of approximately 20 kinds of monosaccharides (glucose, galactose, mannose, fucose, xylose, N-acetylglucosamine, N-acetyl galactosamine, sialic acid, and the complexes and derivatives thereof) and are bound to various intracellular and extracellular proteins and lipids. Sugar chains have different functions according to the monosaccharide sequence and are normally branched in a complicated manner, and it is expected that there are several hundred or more kinds of various structures of sugar chains in human body and further, it is believed that there are several tens of thousands or more kinds of useful structures in the human body. They are supposedly involved in the higher functions of proteins and lipids serving in the living body such as intercellular molecule/cell recognition, but many of the mechanisms have not yet been elucidated. Sugar chains are attracting attention currently in the field of life science as the third life-related chain, following nucleic acids and proteins. In particular, there are high expectations of the role sugar chain play as a ligand (information molecule), and studies ranging from application to development of high-functional materials have been progressing.

The "sugar" or the "monosaccharide" as described herein means a polyhydroxyaldehyde or a polyhydroxyketone having at least one hydroxyl group and at least one aldehyde group or ketone group that constitutes a basic unit of the sugar chain. The sugar, as used herein, is also called a carbohydrate, and these terms are used interchangeably. The sugar chain, as used herein, means a chain or a sequence of one or more sugars, while the sugar or monosaccharide means a unit constituting the sugar chain, unless specified otherwise. Herein, the sugar each having a repetition number n of 2, 3, 4, 5, 6, 7, 8, 9 or 10 are called diose, triose, tetrose, pentose, hexose, heptose, octose, nonose and decose respectively. Generally, a linear polyvalent alcohol aldehyde or ketone corresponds to an aldose or a ketose, respectively. In the present invention, sugars in any form may be used.

In the present invention, the IUPAC nomenclature system and abbreviated names used for describing the sugars are complied with. For example, β-D-galactose is expressed by Gal;

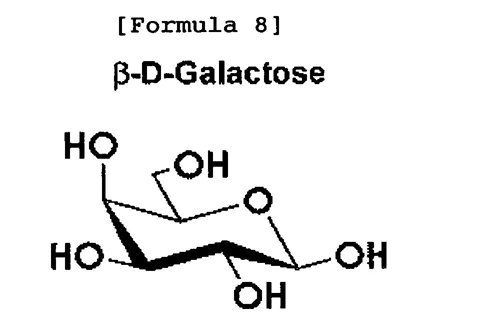

N-acetyl-a-D-galactosamine is expressed by GalNAc;

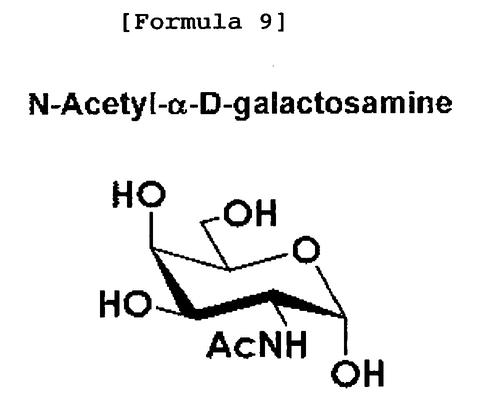

a-D-mannose is expressed by Man;

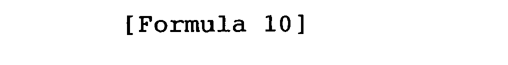

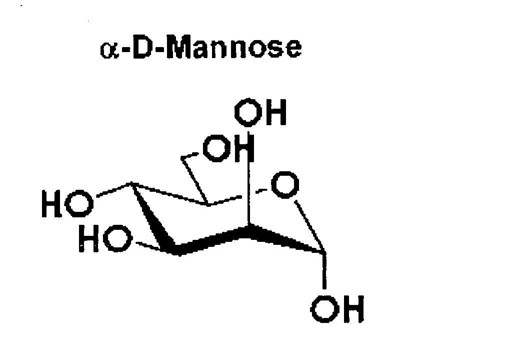

β-D-glucose is expressed by Glc;

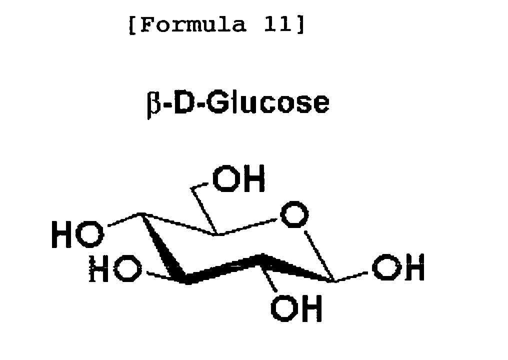

N-acetyl-β-D-glucosamine is expressed by GlcNAc;

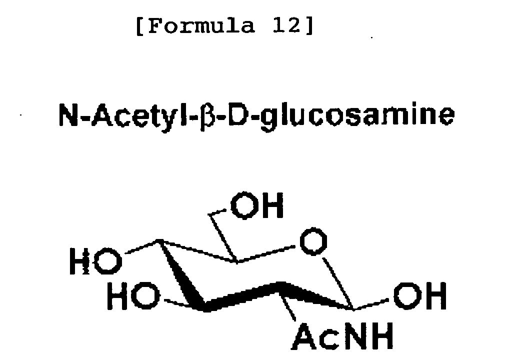

a-L-fucose is expressed by Fuc;

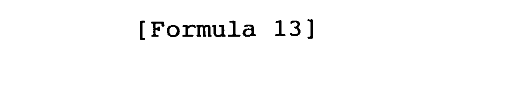

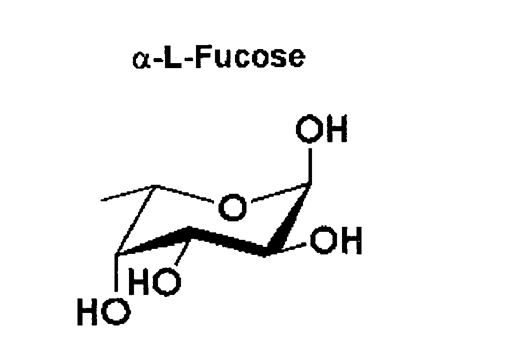

a-N-acetylneuraminic acid is expressed by Neu5Ac;

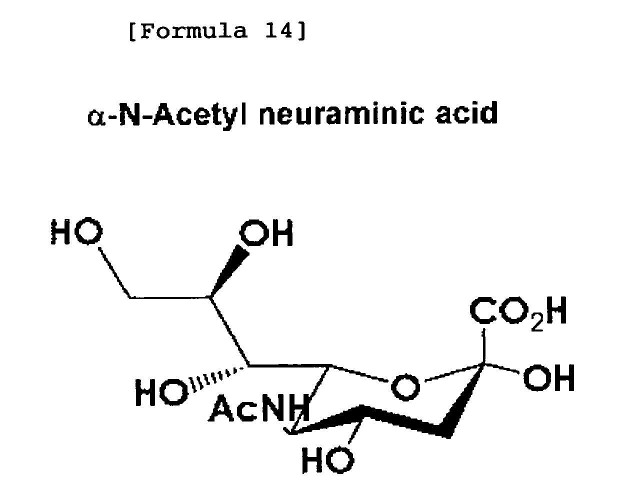

Ceramide is expressed by Cer;

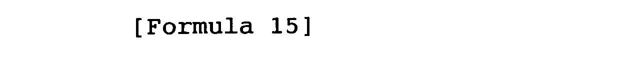

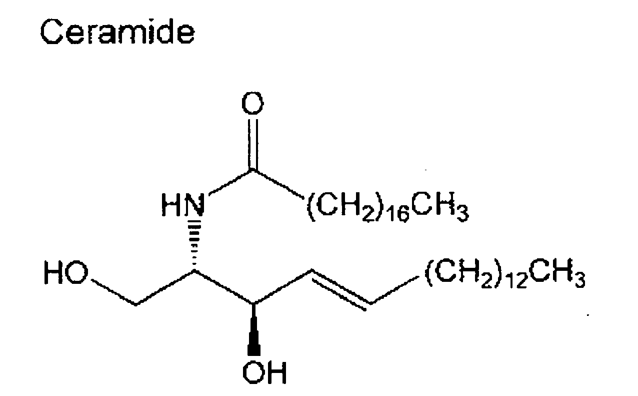

L-serine is expressed by Ser:
CH₂(OH)CH(COOH)NH₂.

Cer is normally classified as a lipid, but in the present specification, since it is classified as a sugar component constituting the sugar chain, it is also regarded as a sugar, unless specified otherwise. Ser is normally classified as an amino acid, but in the present specification, since it is classified as a sugar component constituting the sugar chain, it is also regarded as a sugar, unless specified otherwise. Two cyclic anomers are expressed by a and β anomers. For the reason of expression, they are also expressed by a and b anomers. Thus in the present specification, a and a, and also β and b are used interchangeably in an expression of anomers.

In the present specification, galactose may be any isomer, but is typically β-D-galactose, and thus, the term means β-D-galactose, unless specified otherwise.

In the present specification, acetyl galactosamine may be any isomer but is typically N-acetyl-a-D-galactosamine, and thus, the term means N-acetyl-a-D-galactosamine, unless specified otherwise.

In the present specification, mannose may be any isomer but is typically a-D-mannose, and thus, the term means a-D-mannose, unless specified otherwise.

In the present specification, glucose may be any isomer but is typically β-D-glucose, and thus, the term means β-D-glucose, unless specified otherwise.

In the present specification, acetylglucosamine may be any isomer but is typically N-acetyl-β-D-glucosamine, and thus, the term means N-acetyl-β-D-glucosamine, unless specified otherwise.

In the present specification, fucose may be any isomer but is typically a-L-fucose, and thus, the term means a-L-fucose, unless specified otherwise.

In the present specification, acetylneuraminic acid may be any isomer but typically a-N-acetylneuraminic acid, and thus, the term means a-N-acetylneuraminic acid, unless specified otherwise.

In the present specification, serine may be any isomer but is typically L-serine, and thus, the term menas L-serine unless specified otherwise.

In the present specification, it should be noted that the symbol, name, and abbreviated name (e.g. , Glc) and the like of a sugar are different between when the sugar is a monosaccharide and when it is an unit included in a sugar chain. In the sugar chain, a saccharide unit is bound to another saccharide unite, with a hydrogen atom or a hydroxyl group removed from the other saccharide unit by dehydration condensation. Accordingly, it is understood that each abbreviated symbol of the sugar has all hydroxyl groups when it represents a monosaccharide, but it is in a state where the hydroxyl group and that of another sugar are condensed to leave the remaining oxygen atom when it is used in the sugar chain.

When a sugar is bound to albumin covalently, the sugar's reductive terminal is aminated, and the sugar can be bound to other components such as albumin via the amine group, but, in such a case, it should be noted that the hydroxyl group of the reductive terminal is substituted with an amine group.

Generally, monosaccharides bind to each other to form a disaccharide or a polysaccharide generally through glycoside bonds. The direction of the bond with respect to the ring plane is expressed either with a or β. Particular carbon atoms forming a bond between two carbons are also shown.

In the present specification, a sugar chain is expressed by the following formula:

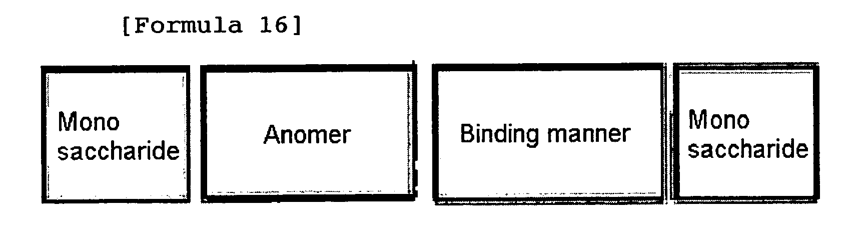

Thus, for example, a β glycoside bond between galactose C-1 and glucose C-4 is expressed by Galβ1, 4Glc. Thus, for example, sialyl Lewis X (SLX) is represented by Neu5Aca2, 3Galβ1, 4(Fuca1,3)GlcNAc. N-acetyllactosamine (G4GN) is represented by Galβ1, 4GlcNAc. a-1,6-Mannobiose (A6) is represented by Manal,6 Man.

A branched sugar chain is expressed in parenthesis, as it is arranged immediately left of the monosaccharide to be bound, as shown in the following formula: 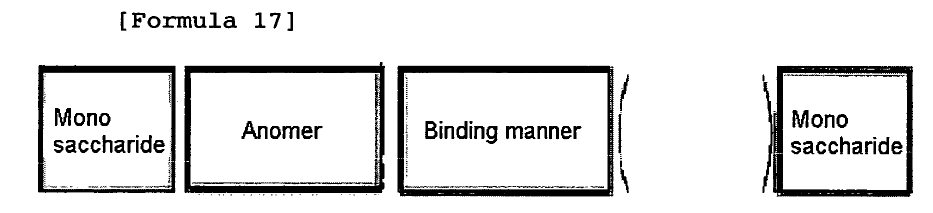 wherein the branched sugar chain in parenthesis is expressed by the following formula:

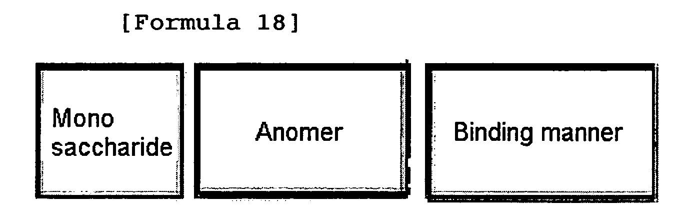

Thus, for example, when galactose C-1 and glucose C-4 are bound each other with β-glycoside binding, and further glucose C-3 and fucose C-1 are bound each other with a-glycoside binding, it is represented by Galβ1, 4(Fuca1,3)Glc. A monosaccharide is expressed basically by putting a number as small as possible to the (latent) carbonyl atom group. Normally, according to general standard of organic chemical nomenclature, the numbering described above is used, even when an atom group superior to the (latent) carbonyl atom group is introduced into the molecule.

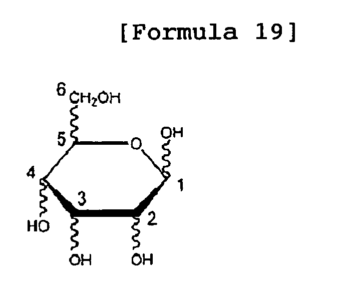

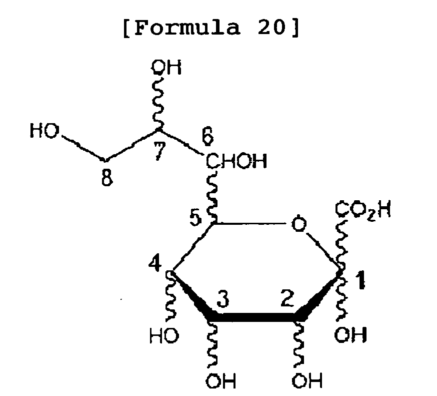

Examples of the sugar chains used herein include, but are not limited to, sialyl Lewis X, N-acetyllactosamine, a1,6-mannobiose and the combinations of two or more of them. Without wishing to be bound by theory, the reason for use of the combinations of two or more of these sugars is that each sugar chain described above has specificity to the lectin locally present in the organ or the cell of the intended target site and the specificity is expressed even in the presence of multiple sugar chains.

The term "lectin," as used herein, means a substance capable of binding to the sugar chain of the cell membrane composite sugars (glycoproteins and glucolipids) and having actions such as cell agglutination, division induction, functional activation and cytotoxicity. If the sugar chain is considered to be an information-providing molecule in cell, it can be said that the lectin is an information-receiving molecule. Cells or organs having a certain property have corresponding lectin patterns. Lectins are involved in infection, body protection, immunity, fertilization, targeting to the targeting cell, cell differentiation, intercellular adhesion, quality control of neogenetic glycoproteins, intracellular selective transportation, and the like. Lectins bind to various kinds of sugar chains and are under strict regulation to have their inherent physical chemical properties of rapid binding and dissociation. They are also called sugar chain-recognizing proteins. Plant lectins have been studied for many years, and there are as many as approximately 300 kinds of known lectins. Recently, animal lectins have also been under intensive study, and new lectins are found constantly. Various sugar chain-recognizing functions based on the lectin group (approximately 100 kinds) in the main lectin families present on animal cell membranes have been studied. In particular, the function as a receptor (information-receiving protein or target molecule) receiving the structural information of the sugar chain ligands having various structures has been attracting attention.

The term "complementary nucleic acid," as used herein, means a nucleic acid defined in the broadest sense in the art, and also, a nucleic acid that can form a base pair with its corresponding nucleic acid by the base-pairing rules of nucleic acids. Examples thereof include, but are not limited to, DNAs, RNAs, and the like.

The term "receptor," as used herein, means an "acceptor" present in a cell and having a structure recognizing a stimulus from outside the cell and transmit such into the cell. Examples thereof include, but are not limited to, cell surface acceptors, intracellular acceptors and the like.

The term "ligand," as used herein, means a molecule that is bound very tightly to a substance. Examples thereof include, but are not limited to, proteins, nucleic acids, chemical substances and the like.

The term "aptamer," as used herein, means a nucleic acid having a relatively small molecular weight that can recognize the structures of various substances (proteins, chemical materials, and the like) and bind thereto. Examples thereof include, but are not limited to, RNA aptamers, DNA aptamers and the like.

The term "antigen," as used herein, means a substance that accelerats the production of an antibody. Examples thereof include, but are not limited to, polysaccharides, proteins, the complexes thereof, viruses, cells, and the like.

The term "hydrophilization," as used herein, means binding of a hydrophilic compound to the liposome surface. Examples of the compounds used for hydrophilization include low-molecular weight hydrophilic compounds, preferably low-molecular weight hydrophilic compounds having at least one OH group, and more preferably low-molecular weight hydrophilic compounds having at least two OH groups. In addition, low-molecular weight hydrophilic compounds having at least one amino group additionally, i.e., hydrophilic compounds having at least one OH group and at least one amino group in the molecule can be included. Typical examples of the hydrophilic compounds include amino alcohols such as tris(hydroxyalkyl)aminoalkanes including tris(hydroxymethyl)aminomethane and the like, and typical examples thereof include tris(hydroxymethyl)aminoethane, tris(hydroxyethyl)aminoethane, tris(hydroxypropyl)aminoethane, tris(hydroxymethyl)aminomethane, tris(hydroxyethyl)aminomethane, tris(hydroxypropyl)aminomethane, tris(hydroxymethyl)aminopropane, tris(hydroxyethyl)aminopropane, tris(hydroxypropyl)aminopropane and the like.

The term "alkyl," as used herein, means a monovalent group formed by eliminating a hydrogen atom from an aliphatic hydrocarbon (refers to "alkane," herein) such as methane, ethane or propane and normally represented by the formula CₙH₂ₙ₊₁ (wherein, n is a positive integer). The alkyl may be a straight chain or a branched chain. The term "substituted alkyl," as used herein, is an alkyl group of which one or more H atoms are substituted with substituent groups specified below. Examples thereof include C1 to C2 alkyl, C1 to C3 alkyl, C1 to C4 alkyl, C1 to C5 alkyl, C1 to C6 alkyl, C1 to C7 alkyl, C1 to C8 alkyl, C1 to C9 alkyl, C1 to C10 alkyl, C1 to C11 alkyl or C1 to C12 alkyl, C1 to C2 substituted alkyl, C1 to C3 substituted alkyl, C1 to C4 substituted alkyl, C1 to C5 substituted alkyl, C1 to C6 substituted alkyl, C1 to C7 substituted alkyl, C1 to C8 substituted alkyl, C1 to C9 substituted alkyl, C1 to C10 substituted alkyl, C1 to C11 substituted alkyl or C1 to C12 substituted alkyl. More specifically, the alkane may be C1 to C2 alkane, C1 to C3 alkane, C1 to C4 alkane, C1 to C5 alkane, C1 to C6 alkane, C1 to C7 alkane, C1 to C8 alkane, C1 to C9 alkane, C1 to C10 alkane, C1 to C11 alkane or C1 to C12 alkane; or alternatively, C1 to C2 substituted alkane, C1 to C3 substituted alkane, C1 to C4 substituted alkane, C1 to C5 substituted alkane, C1 to C6 substituted alkane, C1 to C7 substituted alkane, C1 to C8 substituted alkane, C1 to C9 substituted alkane, C1 to C10 substituted alkane, C1 to C11 substituted alkane or C1 to C12 substituted alkane. The C1 to C10 alkyl means a straight-chain or branched alkyl having 1 to 10 carbon atoms, and typical examples thereof include methyl (CH₃-), ethyl (C₂H₅-), n-propyl (CH₃CH₂CH₂-), isopropyl ((CH₃)₂CH-), n-butyl (CH₃CH₂CH₂CH₂-), n-pentyl (CH₃CH₂CH₂CH₂CH₂-), n-hexyl (CH₃CH₂CH₂CH₂CH₂CH₂-), n-heptyl (CH₃CH₂CH₂CH₂CH₂CH₂CH₂-), n-octyl (CH₃CH₂CH₂CH₂CH₂CH₂CH₂CH₂-), n-nonyl (CH₃CH₂CH₂CH₂CH₂CH₂CH₂CH₂CH₂-), n-decyl (CH₃CH₂CH₂CH₂CH₂CH₂CH₂CH₂CH₂CH₂-), -C(CH₃)₂CH₂CH₂CH(CH₃)₂, -CH₂CH(CH₃)₂, and the like. In addition, the C1 to C10 substituted alkyl is, for example, a C1 to C10 alkyl of which one or a plurality of hydrogen atoms are substituted with substituent groups. R preferably represents a C1 to C6 alkyl group, particularly preferably a C1 to C6 alkyl group.

If the substance according to the present invention or the functional group defined above is substituted with a substituent group R, there may be single or multiple substituent groups R, and the groups R may be independently selected from the group consisting of hydrogen, alkyl, cycloalkyl, alkenyl, cycloalkenyl, alkynyl, alkoxy, carbocyclic group, heterocyclic group, halogen, hydroxy, thiol, cyano, nitro, amino, carboxy, acyl, thiocarboxy, amide, substituted amide, substituted carbonyl, substituted thiocarbonyl, substituted sulfonyl and substituted sulfinyl.

### Application of the technique of a production method for an ammine platinum complex-encapsulating liposome

The technique of the method for producing an ammine platinum complex-encapsulating liposome according to the present invention can be used in the production of liposome preparations for the treatment of clinical conditions (in particular, tumors and cancers).

Examples of the preparations to be encapsulated in the liposome using the method of encapsulating the ammine platinum complex according to the present invention in the liposome include, but are not limited to: medicines for treatment of testicular tumors, bladder cancers, renal pelvic/ureteral tumors, prostate cancers, ovarian cancers, head and neck cancers, non-small cell lung cancers, esophagus cancers, uterocervical cancers, neuroblastomas, stomach cancers, small cell lung cancers, osterosarcomas, blastocyte tumors, and the like. Typical examples of the medicines include, but are not limited to, cis-diammine dichloroplatinum (II), cis-diammine-1,1-cyclobutane-dicarboxylatoplatinum (II), cis-diammine (glucolato)platinum (II), (1R,2R-diammine cyclohexane)oxalatoplatinum (II), cis-diammine dibromoplatinum (II) and the like.

### Application of ammine platinum complex-encapsulating liposome

The ammine platinum complex-encapsulating liposome according to the present invention can be used for the treatment of clinical conditions (in particular, tumors and cancers).

Examples of the clinical conditions treated with the ammine platinum complex-encapsulating liposome according to the present invention include, but are not limited to: testicular tumors, bladder cancers, renal pelvic/ureteral tumors, prostate cancers, ovarian cancers, head and neck cancers, non-small cell lung cancers, esophagus cancers, uterocervical cancers, neuroblastomas, stomach cancers, small cell lung cancers, osterosarcomas, blastocyte tumors, and the like.

### Description of the preferred embodiments

Hereinafter, preferable embodiments of the present invention will be described. Embodiments described below are provided only for more profound understanding of the present invention, and the scope of the present invention shall not be restricted by the following description. It is thus obvious that those skilled in the art can modify the present invention properly within the scope of the present invention with reference to the description of the present description.

### Production method of an ammine platinum complex-encapsulating liposome

An aspect of the present invention provides a method of producing a liposome encapsulating an ammine platinum complex. The method includes the following steps of: A) providing a water-soluble ammine platinum complex, platinum complex raw materials or a combination thereof; B) providing a liposome, liposome raw materials or a combination thereof; and C) preparing a mixture of the water-soluble ammine platinum complex, the platinum complex raw materials or the combination thereof and the liposome, the liposome raw materials or the combination thereof and subjecting the mixture under liposome-forming/maintaining conditions, wherein a salt formed by the platinum complex is in a water-soluble form at the time point where the liposome exists. The production method may include additionally the step of D) subjecting the mixture obtained in the step C) to the presence of a solution containing an ion forming a poorly water-soluble salt with the platinum complex.

In an embodiment, the water-soluble ammine platinum complex for use in the present invention may have two ammine groups. It may be preferably cis-diammine dinitratoplatinum (II).

In another embodiment, the production method according to the present invention may include the steps of A) dissolving the water-soluble ammine platinum complex in a first buffer solution and thus preparing a platinum complex solution; B) providing the liposome raw material; and C) mixing the platinum complex solution with a lipid suspension and subjecting the mixture to the liposome-forming/maintaining conditions. In the method, buffers that do not contain any ion that forms a poorly water-soluble salt with the platinum complex may be used as the first buffer solution and the second buffer solution.

In an embodiment, the step B) of the present production method may include the steps of B-i) suspending a lipid having a liposome-forming ability in a methanol-chloroform solution under agitation, evaporating the agitated solution, and vacuum-drying a precipitate to obtain a lipid membrane; and B-ii) suspending the lipid membrane in a second buffer solution and forming a lipid suspension.

In another embodiment, the first and the second buffer solutions in the present production method may have the same composition or different compositions. These buffer solutions may be any buffer solution, as long as they can keep the platinum complex in the water-soluble form when the liposome is present.

In another embodiment, the production method according to the present invention may include ultrasonicating the lipid suspension after the step B-ii) step.

In another embodiment, the liposome-forming/maintaining condition of the step C) in the production method of the present invention may include the steps of ultrafiltering the mixture of the platinum complex solution and the lipid suspension and leaving the solution to stand overnight. The steps of ultrafiltrating the mixture of the platinum complex solution and the lipid suspension and leaving the mixture to stand overnight may be performed continuously.

In another embodiment, the first buffer solution for use in the production method according to the present invention may be a buffer solution that does not contain any ion forming a poorly water-soluble salt with platinum complex. The first buffer solution preferably does not contain any ion forming a poorly water-soluble salt or contains it in an amount not forming a poor water-soluble salt with the platinum complex even when the ion is present. The ion forming a poorly water-soluble salt may be, for example, a chloride ion (Cl⁻) , bromide ion (Br⁻), iodide ion (I⁻), thiocyanate ion (SCN⁻), cyanide ion (CN⁻) or the like. Examples of the first buffer solution include, but are not limited to: buffer solutions containing an N-tris(hydroxymethyl)-3-aminopropane sulfonate buffering agent (pH: 8.4), a carbonate buffering agent (pH: 8.5), phosphate buffering agent (pH: 8.0), a 2-[4-(2-hydroxyethyl)-1-piperadinyl]ethane sulfonate (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropane sulfonate) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent or the like. Preferably, the first buffer solution is a buffer solution containing an N-tris(hydroxymethyl)-3-aminopropane sulfonate buffering agent (preferably, pH 8.4). In the present invention, a buffer solution other than that above may be used as the first buffer solution, as long as the platinum complex forms a poorly water-soluble salt and as long as it is a buffer solution that does not contain any ion forming a poorly water-soluble salt or contains it in an amount not forming a poorly water-soluble salt with the platinum complex even when an ion is present.

In a preferable embodiment, the first buffer solution may be adjusted to a pH in the range of 6 to 10 (preferably 8.4).

In another embodiment, the second buffer solution for use in the production method according to the present invention may be a buffer solution that does not contain any ion forming a poorly water-soluble salt with the platinum complex. The second buffer solution is preferably a buffer solution that does not contain any ion forming a poorly water-soluble salt or contains it in an amount not forming a poorly water-soluble salt with the platinum complex even when an ion is present.

As described herein, examples of "the ions forming a poorly water-soluble salt" with the platinum complex include a chloride ion (Cl⁻), bromide ion (Br⁻), iodide ion (I⁻), thiocyanate ion (SCN⁻), cyanide ion (CN⁻), and the like. The second buffer solution for use may be, for example, a buffer solution containing an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethane sulfonate (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropane sulfonate) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent, or the like, but is not limited thereto. Preferably, the second buffer solution is a buffer solution containing N-tris(hydroxymethyl)-3-aminopropane sulfonate buffering agent (preferably, pH 8.4). In the present invention, a buffer solution other than that above may be used as the second buffer solution, as long as the platinum complex forms a poor water-soluble salt and as long as the buffer solution does not contain any ion forming a poorly water-soluble salt or contains it in an amount not forming a poorly water-soluble salt with the platinum complex even when an ion is present.

Examples of the solubility are shown below.
1. When there are 2 equivalents of chloride ion (Cl⁻), bromide ion (Br⁻), iodide ion (I⁻) or thiocyanate ion (SCN⁻) with respect to the water-soluble platinum complex (in the state that two water molecules are coordinated), two coordinated water molecules in the water-soluble platinum complex are substituted with the ions, forming a scarcely-soluble salt. When an equivalent of an ion is contained, one water molecule is substituted with the ion. When there is no ion, it takes the structure of the two water molecules to remain coordinated.
2. In the case of chloride ion (Cl⁻), it is believed that the water molecules are substituted at an intracellular chloride ion concentration of 0 to 4 mM and the platinum complex binds to DNA, and thus, the chloride ion range in issue is expressed by mM. Among the bromide ion (Br⁻), iodide ion (I⁻) and thiocyanate ion (SCN⁻) , Br⁻ and I⁻ coordinated complexes are said to have a difference in the active strength or have an activity. Thus, the platinum complex is considered to have a structure coordinated as water molecules in the intracellular environment.

The poorly water-soluble substance coordinated with Cl⁻, Br⁻ or I⁻ is known to have anti-cancer activity. It is said that the ions are binding to the platinum atom more tightly in the order of Cl⁻ < Br⁻ < I⁻. It is believed that Br⁻ and I⁻ tend to bind with the platinum more easily than Cl⁻, forming a scarcely-soluble substance. It is thus believed that it is possible to form the poorly water-soluble substance under the same condition as that for Cl⁻. Thus, it is possible to perform the present invention, by taking these conditions into consideration.

In addition, in the present invention, it is intended that when the liposome is formed, the poorly water-soluble platinum complex once formed may be released less easily out of the liposome and thus, the delivery efficiency can be raised.

In a preferable embodiment, the second buffer solution may be adjusted to a pH in the range of 6 to 10 (preferably, 8.4).

In another preferable embodiment, the production method according to the present invention may include the following steps of: A) dissolving the water-soluble ammine platinum complex in a first buffer solution and thus preparing a platinum complex solution; B) providing the liposome; and C) mixing the platinum complex solution with the liposome and subjecting the mixture to the liposome-forming/maintaining conditions. In the production method, the first buffer solution contains no ion forming a poor water-soluble salt with the platinum complex.

In a preferable embodiment of the production method according to the present invention, the liposome can have a sufficient composition for allowing the water-soluble ammine platinum complex to migrate into the liposome through the liposomal membrane and remain therein.

In another preferable embodiment, the liposome-forming/maintaining conditions may be sufficient conditions for maintaining the liposome without degradation and at the same time allowing the water-soluble ammine platinum complex to migrate through the liposomal membrane into the liposome and remain therein.

In an embodiment, examples of the lipids constituting the liposome or the liposome raw materials for use in the production method according to the present invention include, but are not limited to: dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dimyristoyl phosphatidylcholine, distearoyl phosphatidylcholine, dioleoyl phosphatidylcholine, dimyristoyl phosphatidylserine, dipalmitoyl phosphatidylserine, distearoyl phosphatidylserine, dioleoyl phosphatidylserine, dimyristoyl phosphatidylinositol, dipalmitoyl phosphatidylinositol, distearoyl phosphatidylinositol, dioleoyl phosphatidylinositol, dimyristoyl phosphatidylethanolamine, distearoyl phosphatidylethanolamine, dioleoyl phosphatidylethanolamine, dimyristoyl phosphatidic acid, dipalmitoyl phosphatidic acid, distearoyl phosphatidic acid, dioleoyl phosphatidic acid, galactosyl ceramide, glycosyl ceramide, lactosyl ceramide, phosphatide, globoside, GM1 (Galβ1, 3GalNAcβ1, 4(NeuAa2,3)Galβ1, 4Glcβ1, 1'Cer), ganglioside GD1a, ganglioside GD1b, dimyristoyl phosphatidylglycerol, dipalmitoyl phosphatidylglycerol, distearoyl phosphatidylglycerol, dioleoyl phosphatidylglycerol and the like. Preferable may be dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, and dipalmitoyl phosphatidylglycerol.

The liposome for use in the present production method preferably may contain dipalmitoyl phosphatidylcholine, cholesterol, ganglioside, dicetyl phosphate, dipalmitoyl phosphatidylethanolamine and sodium cholate at a molar ratio of 35:40:15:5:5:167 as the lipids in the lipid suspension.

The liposome for use in the present production method may be any liposome, as long as the water-soluble ammine platinum complex can penetrate the liposomal membrane. A commercially available liposome may be used, and examples thereof include the products available from NOF Corporation and the like. General preparative method and characteristics of the liposomes are known in the art and can be determined easily by traditional experiments and technical common knowledge. Examples thereof include an ultrasonication method, an ethanol injection method, a French press method, an ether injection method, a cholate method, a freeze drying method and a reverse phase evaporization method (see e.g. "New development in liposome Application -For development of an artificial cell-, Kazunari Akiyoshi and Kaoru Tsujii Ed., NTS p33 to 45 (2005)" and "Liposomes, Shoshichi Nojima, p. 21-40 Nankodo (1988)").

In an embodiment of the present production method, a liposome may be used by mixing liposomes prepared by a plurality of production methods (e.g. those from the improved cholate method and those from freeze drying method).

The lipids constituting the liposome or the liposome raw materials for use in the present invention may be those other than above. They may be any compound, as long as the water-soluble ammine platinum complex is able to pass through the membrane of the provided liposome or the formed liposome into the liposome and remain therein.

In another embodiment, the mixture may be preferably adjusted to a pH in the range of 6 to 10 (preferably, pH 8.4) in step C) of the production method according to the present invention.

In yet another embodiment of the production method according to the present invention, the condition wherein the salt formed by the platinum complex in a water-soluble form, is a condition of subjecting the salt to the presence of a solution containing substantially no ion forming a poorly water-soluble salt with the platinum complex. The ion forming a poorly water-soluble salt may be, for example, a chloride ion (Cl⁻), bromide ion (Br⁻), iodide ion (I⁻), thiocyanate ion (SCN⁻), cyanide ion (CN⁻) or the like (preferably, chloride ion (Cl⁻)). The solution preferably does not contain any ion forming a poorly water-soluble salt with the platinum complex or contains the ion only in an amount at which the platinum complex does not form a poorly water-soluble salt even when an ion is present. The ion forming a poorly water-soluble salt with the platinum complex may be added, for example in the case of chloride ion (Cl⁻), in the range of 0 to 4 mM. It is believed that in the case of chloride ion (Cl⁻), since the concentration of 0 to 4 mM is not at a concentration higher than that existing in the body, it would not cause the precipitation of the poorly water-soluble salt.

In an embodiment of the production method according to the present invention, in tep C), the water-soluble ammine platinum complex, platinum complex raw materials or the combination thereof, and the liposome, liposome raw materials or the combination thereof may be mixed at a ratio in the range of 1:9 to 9:1.

The liposome, liposome raw material or the combination thereof; the platinum complex, platinum complex raw materials or the combination thereof; and optionally a buffer solution may be mixed in any order. For example, the order may be, but is not limited to: 1) mixing the liposome raw materials and the platinum complex and subsequently adding the buffer solution, 2) mixing the liposome raw materials and the buffer solution and subsequently adding the platinum complex, or 3) dissolving the platinum complex in the buffer solution and subsequently adding the liposome raw materials. The order is not critical because the present invention can be achieved as long as the liposome-forming/maintaining conditions are satisfied, i.e. as long as the water-soluble ammine platinum complex can be brought into contact with the liposome at the time point where the liposome exists, though they can be mixed in any order. When a platinum complex strongly acidic in aqueous solution is used, the pH of the mixture liquid is preferably in a range of 6 to 10.

In one embodiment, the platinum complex suspension and the lipid suspension may be mixed at a ratio in the range of 1:9 to 9:1.

In a preferable embodiment, the platinum complex suspension and the lipid suspension may be mixed at a ratio of 7:3.

In another embodiment, the platinum complex solution and the liposome may be mixed at a ratio in the range of 1:9 to 9:1 (preferably 7:3).

In a further embodiment, in step C) of the production method according to the present invention, the mixture contains substantially no ion forming a poorly water-soluble salt. The ion forming a poorly water-soluble salt may be, for example, a chloride ion (Cl⁻), bromide ion (Br⁻), iodide ion (I⁻), thiocyanate ion (SCN⁻), cyanide ion (CN⁻), or the like. Preferably, the ion forming a poorly water-soluble salt may be chloride ion (Cl⁻). In one embodiment, the mixture may contain the chloride ion (Cl⁻) in the range of 0 to 4 mM. The mixture preferably does not contain any ion forming a poorly water-soluble salt with the platinum complex or contains only such in an amount at which the platinum complex does not form a poorly water-soluble salt even when an ion is present.

In an embodiment, the mixture may contain a buffering agent such as an N-tris(hydroxymethyl)-3-aminopropane sulfonate buffering agent (pH: 8.4), a carbonate buffering agent (pH: 8.5), a phosphate buffering agent (pH: 8.0), a 2-[4-(2-hydroxyethyl)-1-piperadinyl]ethane sulfonate (HEPES) buffering agent (pH: 7.2), a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropane sulfonate) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, 3-cyclohexylaminopropane sulfonate buffering agent or the like, (preferably, an N-tris(hydroxymethyl)-3-aminopropane sulfonate buffering agent (pH: 8.4)).

In another embodiment, in step D) of the production method according to the present invention, the ion forming a poorly water-soluble salt with the platinum complex may be a chloride ion (Cl⁻), bromide ion (Br⁻), iodide ion (I⁻), thiocyanate ion (SCN⁻), or cyanide ion (CN⁻). In a preferable embodiment, the ion forming a poorly water-soluble salt with the platinum complex is a chloride ion (Cl⁻).

In a further embodiment, the chloride ion (Cl⁻) is provided from NaCl, HCl, or CaCl₂.

In another embodiment, in step D) of the production method according to the present invention, the ion forming a poorly water-soluble salt with the platinum complex (e.g., chloride ion (C1⁻)) is provided, for example, from an N-tris(hydroxymethyl)-3-aminopropane sulfonate buffer solution, a carbonate buffer solution, a phosphate buffer solution, a 2-[4-(2-hydroxyethyl)-1-piperadinyl]ethane sulfonate buffer solution, a tris(hydroxy)aminomethane buffer solution, a 3-(N-morpholino)propane sulfonate buffer solution, a N-tris(hydroxymethyl)1-2-aminoethane sulfonate buffer solution, a N-2-hydroxyethylpiperazine-N'-2-ethane sulfonate buffer solution, an N-tris(hydroxymethyl)methyl-2-hydroxy-3-aminopropane sulfonate buffer solution, an piperazine-N,N'-bis(2-hydroxypropane sulfonate) buffer solution, an N-2-hydroxyethylpiperazine-N'-2-hydroxypropane-3-propane sulfonate buffer solution, a tris(hydroxymethylmethylglycine) buffer solution, an N,N-bis(2-hydroxyethyl)glycine buffer solution, a 2-(cyclohexylamino)propylethane sulfonate buffer solution, a 3-N-cyclohexylamino-2-hydroxypropane sulfonate buffer solution, a 3-cyclohexylaminopropane sulfonate buffer solution, or the like, but the source is not limited thereto. Preferably, the chloride ion (Cl⁻) may be provided from an N-tris(hydroxymethyl)-3-aminopropane sulfonate buffer solution (pH: 8.4), a carbonate buffer solution (pH: 8.5), a phosphate buffer solution (pH: 8.0), a 2-[4-(2-hydroxyethyl)-1-piperadinyl]ethane sulfonate buffer solution (pH: 7.2) or the like. The ion forming a poorly water-soluble salt with the platinum complex used in step D) of the production method according to the present invention may be provided from a buffer solution other than those above. Any buffer can be used because any buffer solution providing an ion forming a poor water-soluble salt with the platinum complex can convert the platinum complex into a poor water-soluble salt.

In another embodiment, step D) of the production method may include the steps of: i) subjecting the liposome formed to hydrophilization; ii) binding a target specific substance to the liposome; iii) hydrophilizing the modified target specific substance-bound liposome; and iv) filtering the solution containing the hydrophilized liposome.

In a further embodiment, the target specific substance for use in the present production method may be, for example, an antibody, sugar chain, lectin, complementary nucleic acid, receptor, ligand, aptamer, or antigen. Preferably, the target specific substance may be an antibody or a sugar chain, but is not limited thereto. The target specific substance may be any substance if it provides the liposome with target specificity without destruction of the liposome. Those skilled in the art can determine the target specific substance to be bound to the liposome properly depending on the target substance.

In another embodiment, the ammine platinum complex for use in the present invention may be poorly water-soluble.

In a preferable embodiment, the production method according to the present invention may include the following steps of: (A1) dissolving cis-diammine dinitratoplatinum (II) in N-tris(hydroxymethyl)-3-aminopropane sulfonate buffer solution to give a cis-diammine dinitratoplatinum (II)-containing solution, wherein the N-tris(hydroxymethyl)-3-aminopropane sulfonate buffer solution does not contain a chloride ion (Cl⁻) or contains a chloride ion (Cl⁻) in a range of 0 to 4 mM; and (A2) adjusting the pH of the cis-diammine dinitratoplatinum (II)-containing solution to be from 6 to 10 (preferably 8.4); (B1) mixing dipalmitoyl phosphatidylcholine, cholesterol, ganglioside, dicetyl phosphate, dipalmitoyl phosphatidylethanolamine and sodium cholate (preferably at a molar ratio of 35:40:15:5:5:167) to give a lipid; (B2) suspending the lipid in a methanol-chloroform (preferably, 1:1) solution under agitation, evaporating the agitated solution and vacuum-drying the precipitate to obtain a lipid membrane; and (B3) suspending the lipid membrane in N-tris(hydroxymethyl)-3-aminopropane sulfonate buffer solution (pH: 6 to 10, preferably, pH 8.4) to give a lipid suspension, wherein the N-tris(hydroxymethyl)-3-aminopropane sulfonate buffer solution does not contain a chloride ion (Cl⁻) or contains a chloride ion (Cl⁻) in the range of 0 to 4 mM; (B4) agitating the lipid suspension at 30°C to 40°C and then ultrasonicating the suspension after nitrogen substitution; and (C1) mixing the pH-adjusted cis-diammine dinitratoplatinum (II) solution with the ultrasonicated lipid suspension at a ratio in a range of 1:9 to 9:1 (preferably 7:3) and ultrafiltering the suspension at the molecular weight cutoff of 500 to 300,000 (preferably 10,000).

In another preferable embodiment, the production method according to the present invention may include the following steps of : (A1) dissolving cis-diammine dinitratoplatinum (II) in N-tris(hydroxymethyl)-3-aminopropane sulfonate buffer solution to give a cis-diammine dinitratoplatinum (II)-containing solution, wherein the N-tris(hydroxymethyl)-3-aminopropane sulfonate buffer solution does not contain a chloride ion (Cl⁻) or contains a chloride ion (Cl⁻) in the concentration range of 0 to 4 mM; and (A2) adjusting the pH of the cis-diammine dinitratoplatinum (II)-containing solution to be from 6 to 10 (preferably 8.4); and, as step B), (B1) providing the liposome; and (C1) mixing the pH-adjusted cis-diammine dinitratoplatinum (II) solution with the liposome at a ratio in the range of 1:9 to 9:1 and ultrafiltering the suspension at a molecular weight cutoff of 500 to 300,000 (preferably, 10,000).

In another embodiment, the method for producing the liposome encapsulating an ammine platinum complex according to the present invention includes the following steps of: A) providing a solution containing two ammine group-containing water-soluble platinum complex; B) mixing the water-soluble platinum complex-containing solution with the liposome-forming lipid suspension to give a mixture solution; C) subjecting the mixture solution to the liposome-forming conditions; and D) subjecting the mixture solution to the presence of a solution containing an ion forming a poorly water-soluble salt.

In a further embodiment, the water-soluble platinum complex-containing solution in step A) may be adjusted to a pH in the range of pH 6 to 10 (preferably 8.4).

In an embodiment, the lipid suspension in the step B) may be prepared by the steps of i) suspending the lipid in methanol-chloroform solution under agitation, evaporating the agitated solution and vacuum-drying the precipitate to give a lipid membrane; and ii) suspending the lipid membrane in a suspension buffer solution.

In an embodiment, the methanol-chloroform solution may contain methanol and chloroform at a ratio of 1:1.

In an embodiment, the method may include ultrasonicating the lipid suspension additionally after the step B). Preferably, the ultrasonication step may be agitating the lipid solution at 30° C to 40° C and ultrasonicating the solution after nitrogen substitution.

In an embodiment, in step C), the mixture solution may be subjected to the liposome-forming condition for a period sufficient for preparation of the liposome. Preferably, the subjecting of the mixture solution to the liposome-forming conditions may be, for example, ultrafiltration of the mixture solution, and leaving the mixture solution overnight, or the like. More preferably, the subjecting the mixture solution to the liposome-forming conditions may be ultrafiltration of the mixture solution at a molecular weight cutoff of 10,000.

In an embodiment, step D) is carried out after confirmation of liposome formation in step C).

In another embodiment, the method of producing the liposome encapsulating an ammine platinum complex according to the present invention includes the following steps of: A-1) preparing a solution containing two ammine group-containing water-soluble platinum complexes; A-2) adjusting the pH of the water-soluble platinum complex-containing solution in the range of 6 to 10; B-1) mixing the pH-adjusted cis-diammine dinitratoplatinum (II) solution with the ultrasonicated lipid suspension at a ratio in the range of 1: 9 to 9:1 to give a mixture solution; C-1) ultrafiltering the mixture solution; and D-1) subjecting the mixture solution in the presence of chloride ion (Cl⁻) after ultrafiltration , wherein the ammine platinum complex is poorly water-soluble.

In another embodiment, the method of producing the liposome encapsulating an ammine platinum complex according to the present invention includes the following steps of: (A1) dissolving cis-diammine dinitratoplatinum (II) in N-tris(hydroxymethyl)-3-aminopropane sulfonate buffer solution containing substantially no chloride ion (Cl⁻) to give a cis-diammine dinitratoplatinum (II)-containing solution; (A2) adjusting the pH of the cis-diammine dinitratoplatinum (II)-containing solution to be 8.4; (B1) mixing dipalmitoyl phosphatidylcholine, cholesterol, ganglioside, dicetyl phosphate, dipalmitoyl phosphatidylethanolamine and sodium cholate at a molar ratio of 35:40:15:5:5:167 to prepare a lipid; (B2) suspending the lipid in a methanol-chloroform (1:1) solution under agitation, evaporating the agitated solution and vacuum-drying the precipitate to give a lipid membrane; (B3) suspending the lipid membrane in N-tris(hydroxymethyl)-3-aminopropane sulfonate buffer solution (pH: 8.4) containing substantially no chloride ion (Cl⁻) to give a lipid suspension; (B4) agitating the lipid suspension at 30°C to 40°C and then ultrasonicating the suspension after nitrogen substitution; (B5) mixing the pH-adjusted cis-diammine dinitratoplatinum (II) solution with the ultrasonicated lipid suspension at a ratio in the range of 7:3 to give a mixture solution; (C1) ultrafiltering the mixture solution at a molecular weight cutoff of 10,000; and (D1) subjecting the mixture solution with at least one buffer solution selected from the group consisting of an N-tris(hydroxymethyl)-3-aminopropane sulfonate buffer solution (pH: 8.4), a carbonate buffer solution (pH: 8.5), a PBS (pH: 8.0) and a HEPES buffer solution (pH: 7.2) after ultrafiltration.

### Liposome encapsulating an ammine platinum complex

An aspect of the present invention provides a liposome encapsulating an ammine platinum complex. The liposome encapsulating an ammine platinum complex can be prepared by mixing water-soluble ammine platinum complex raw materials with liposome raw materials and subjecting the mixture under a liposome-forming/maintaining condition.

In an embodiment, the liposome encapsulating an ammine platinum complex according to the present invention may contain two ammonia molecules.

In another embodiment, the liposome encapsulating an ammine platinum complex according to the present invention may be cis-diamine dichloroplatinum (II) in water-soluble form.

In another embodiment, the liposome encapsulating an ammine platinum complex according to the present invention may be cis-diamine dichloroplatinum (II).

In yet another embodiment, the liposome encapsulating an ammine platinum complex according to the present invention may contain, as the lipid: dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dimyristoyl phosphatidylcholine, distearoyl phosphatidylcholine, dioleoyl phosphatidylcholine, dimyristoyl phosphatidylserine, dipalmitoyl phosphatidylserine, distearoyl phosphatidylserine, dioleoyl phosphatidylserine, dimyristoyl phosphatidylinositol, dipalmitoyl phosphatidylinositol, distearoyl phosphatidylinositol, dioleoyl phosphatidylinositol, dimyristoyl phosphatidylethanolamine, distearoyl phosphatidylethanolamine, dioleoyl phosphatidylethanolamine, dimyristoyl phosphatidic acid, dipalmitoyl phosphatidic acid, distearoyl phosphatidic acid, dioleoyl phosphatidic acid, galactosyl ceramide, glycosyl ceramide, lactosyl ceramide, phosphatide, globosides, GM1 (Galβ1, 3GalNAcβ1, 4(NeuAa2,3)Galβ1, 4Glcβ1, 1'Cer), ganglioside GD1a, ganglioside GD1b, dimyristoyl phosphatidylglycerol, dipalmitoyl phosphatidylglycerol, distearoyl phosphatidylglycerol, dioleoyl phosphatidylglycerol, or the like, but the lipid is not limited thereto. Preferably, it may be dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, or dipalmitoyl phosphatidylglycerol. Other lipids than those described above can be used because even when it is a liposome composed of a lipid other than above, the cis-diammine dichloroplatinum (II) and/or the water-soluble form thereof can still remain in the liposome.

In the present invention, liposomes prepared by a plurality of production methods (e.g. improved cholate method and freeze drying method) may be used as the liposome.

In an embodiment, the liposome encapsulating an ammine platinum complex according to the present invention may additionally contain a target specific substance. Examples of the target specific substances include, but are not limited to: antibodies, sugar chains, lectins, complementary nucleic acids, receptors, ligand aptamers and antigens (preferably, antibodies and sugar chains). It is possible to modify the surface of liposomal membrane with any substance using a suitable crosslinking agent.

Another aspect of the present invention provides a liposome encapsulating an ammine platinum complex that contains 0.3 µg of an ammonia-containing ammine platinum complex with respect to 1 mg of the lipid. For example, it provides an liposome encapsulating an ammine platinum complex containing the ammonia-containing ammine platinum complex in an amount of more than 0.3 µg (e.g., 0.3 µg, 0.4 µg, 0.5 µg, 0.6 µg, 0.7 µg, 0.8 µg, 0.9 µg, 1.0 µg, 2.0 µg, 3.0 µg, 4.0 µg, 5.0 µg, 6.0 µg, 7.0 µg, 8.0 µg, 9.0 µg, 10.0 µg, 11.0 µg, 12.0 µg, 13.0 µg, 14.0 µg, 15.0. 16.0 µg, 17. 0 µg, 18.0 µg, 19.0 µg, 20.0), or preferably 9 µg or 17 µg or more.

In another aspect of the present invention provides a liposome encapsulating an ammine platinum complex containing an ammonia-containing ammine platinum complex in an amount of, for example, 10 µg or more, 20 µg or more, 30 µg or more, 40 µg or more, 50 µg or more, 60 µg or more, 70 µg or more, 80 µg or more, 90 µg or more, 100 µg or more, 150 µg or more, 200 µg or more, or 300 µg or more, with respect to 1 mg of the phospholipid.

In another aspect of the present invention provides liposome encapsulating an ammine platinum complex containing an ammonia-containing ammine platinum complex in an amount of, for example, 39 µg/ml, 40 µg/ml, 50 µg/ml, 60 µg/ml, 70 µg/ml, 80 µg/ml, and 90 µg/ml, or 100 µg/ml or more, with respect to 1 ml of the liposome.

Another aspect of the present invention provides a liposome encapsulating an ammine platinum complex containing the ammonia-containing ammine platinum complex in an amount of, for example, 1×10⁻¹⁰ µg or more, 2×10⁻¹⁰ µg or more, 3×10⁻¹⁰ µg or more, 4×10⁻¹⁰ µg or more, 5×10⁻¹⁰ µg or more, or 10⁻⁹ µg or more in a single liposome.

In an embodiment, the liposome encapsulating an ammine platinum complex according to the present invention may contain two ammonia molecules.

In another embodiment, the liposome encapsulating an ammine platinum complex according to the present invention may be cis-diamine dichloroplatinum (II) in water-soluble form.

In another embodiment, the liposome encapsulating an ammine platinum complex according to the present invention may be cis-diamine dichloroplatinum (II).

In another embodiment, the liposome encapsulating an ammine platinum complex according to the present invention may contain, as the lipid: dipalmitoyl phosphatidylcholine, cholesterol, ganglioside, dicetyl phosphate, dipalmitoyl phosphatidylethanolamine, sodium cholate, dimyristoyl phosphatidylcholine, distearoyl phosphatidylcholine, dioleoyl phosphatidylcholine, dimyristoyl phosphatidylserine, dipalmitoyl phosphatidylserine, distearoyl phosphatidylserine, dioleoyl phosphatidylserine, dimyristoyl phosphatidylinositol, dipalmitoyl phosphatidylinositol, distearoyl phosphatidylinositol, dioleoyl phosphatidylinositol, dimyristoyl phosphatidylethanolamine, distearoyl phosphatidylethanolamine, dioleoyl phosphatidylethanolamine, dimyristoyl phosphatidic acid, dipalmitoyl phosphatidic acid, distearoyl phosphatidic acid, dioleoyl phosphatidic acid, galactosyl ceramide, glycosyl ceramide, lactosyl ceramide, phosphatide, globosides, GM1 (Galβ1, 3GalNAcβ1, 4(NeuAa2,3)Galβ1, 4Glcβ1, 1'Cer), ganglioside GD1a, ganglioside GD1b, dimyristoyl phosphatidylglycerol, dipalmitoyl phosphatidylglycerol, distearoyl phosphatidylglycerol, dioleoyl phosphatidylglycerol, or the like, but the lipid is not limited thereto, as long as a liposome of a lipid other than those above can contain cis-diammine dichloroplatinum (II) and/or the water-soluble form thereof, according to the present invention, and allow such to be retained it in the liposome. Preferably, the liposome encapsulating an ammine platinum complex may contain dipalmitoyl phosphatidylcholine, cholesterol, ganglioside, dicetyl phosphate, dipalmitoyl phosphatidylethanolamine and sodium cholate at a molar ratio of 35:40:15:5:5:167.

In an embodiment, the liposome encapsulating an ammine platinum complex according to the present invention may additionally contain a target specific substance. Examples of the target specific substances include antibodies, sugar chains, lectins, complementary nucleic acids, receptors, ligands, aptamers and antigens (preferably, antibodies and sugar chains), but are not limited thereto, because it is possible to modify the surface of liposomal membrane with any substance by using a suitable crosslinking agent.

The liposome encapsulating an ammine platinum complex according to the present invention may be used in any form as described above (production method).

### Composition

An aspect of the present invention provides a composition for the treatment of cancers or tumors. The composition includes a liposome encapsulating an ammonia-containing ammine platinum complex, which is prepared by mixing liposome with water-soluble ammine platinum complex raw materials and liposome raw materials and subjecting the mixture to liposome-forming/maintaining conditions.

Another aspect of the present invention provides a composition for the treatment of cancers or tumors. The composition includes a liposome encapsulating an ammonia-containing ammine platinum complex, wherein the liposome contains the ammine platinum complex in an amount of, for example, 0.3 µg, 0.4 µg, 0.5 µg, 0.6 µg, 0.7 µg, 0.8 µg, 0.9 µg, 1.0 µg, 2.0 µg, 3.0 µg, 4.0 µg, 5.0 µg, 6.0 µg, 7.0 µg, 8.0 µg, 9.0 µg, 10.0 µg, 11.0 µg, 12.0 µg, 13.0 µg, 14.0 µg, 15.0 µg, 16.0 µg, 17.0 µg, 18.0 µg, 19.0 µg, or 20.0 µg; preferably 9 µg or more or 17 µg or more, with respect to 1 mg of the lipid.

Another aspect of the present invention provides a composition of a liposome encapsulating an ammine platinum complex containing the ammonia-containing ammine platinum complex in an amount of, for example, 10 µg or more, 20 µg or more, 30 µg or more, 40 µg or more, 50 µg or more, 60 µg or more, 70 µg or more, 80 µg or more, 90 µg or more, 100 µg or more, 150 µg or more, 200 µg or more, or 300 µg or more, with respect to 1 mg of the phospholipid.

Another aspect of the present invention provides a composition of a liposome encapsulating an ammine platinum complex, containing the ammonia-containing ammine platinum complex in an amount of for example, 39 µg/ml, 40 µg/ml, 50 µg/ml, 60 µg/ml, 70 µg/ml, 80 µg/ml or more, 90 µg/ml, or 100 µg/ml in 1 ml of the liposome. Another aspect of the present invention provides a composition of a liposome encapsulating an ammine platinum complex, containing the ammonia-containing ammine platinum complex in an amount of, for example, 1×10⁻¹⁰ µg or more, 2×10⁻¹⁰ µg or more, 3×10⁻¹⁰ µg or more, 4×10⁻¹⁰ µg or more, 5×10⁻¹⁰ µg or more, or 10⁻⁹ µg or more in a single liposome.

In an embodiment, the ammine platinum complex in the liposome for use in the composition according to the present invention may have two ammonia molecules.

In another embodiment, the ammine platinum complex cis-diamine dichloroplatinum (II) may be in a water-soluble form in the liposome for use in the composition according to the present invention.

In another embodiment, the ammine platinum complex of the liposome for use in the composition according to the present invention may be cis-diamine dichloroplatinum (II).

In another embodiment, the liposome encapsulating an ammine platinum complex for use in the composition according to the present invention may contain, as the lipid, dipalmitoyl phosphatidylcholine, cholesterol, ganglioside, dicetyl phosphate, dipalmitoyl phosphatidylethanolamine, sodium cholate sodium cholate, dicetyl phosphatidylethanolamine-polyglycerin 8G, dimyristoyl phosphatidylcholine, distearoyl phosphatidylcholine, dioleoyl phosphatidylcholine, dimyristoyl phosphatidylserine, dipalmitoyl phosphatidylserine, distearoyl phosphatidylserine, dioleoyl phosphatidylserine, dimyristoyl phosphatidylinositol, dipalmitoyl phosphatidylinositol, distearoyl phosphatidylinositol, dioleoyl phosphatidylinositol, dimyristoyl phosphatidylethanolamine, distearoyl phosphatidylethanolamine, dioleoyl phosphatidylethanolamine, dimyristoyl phosphatidic acid, dipalmitoyl phosphatidic acid, distearoyl phosphatidic acid, dioleoyl phosphatidic acid, galactosyl ceramide, glycosyl ceramide, lactosyl ceramide, phosphatide, globosides, GM1 (Galβ1, 3GalNAcβ1,4 (NeuAa2,3)Galβ1, 4Glcβ1,1'Cer), ganglioside GD1a, ganglioside GD1b, dimyristoyl phosphatidylglycerol, dipalmitoyl phosphatidylglycerol, distearoyl phosphatidylglycerol, dioleoyl phosphatidylglycerol or the like, but the lipid is not limited to those above. Preferably, it may be dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, or dipalmitoyl phosphatidylglycerol. Other lipids than those described above can be used because even when it is a liposome composed of a lipid other than above, the cis-diammine dichloroplatinum (II) and/or the water-soluble form thereof can still remain in the liposome.

In an embodiment, the liposome for use in the composition according to the present invention may contain a target specific substance. Examples of the target specific substances include, but are not limited to, antibodies, sugar chains, lectins, complementary nucleic acids, receptors, ligands, aptamers and antigens (preferably, antibodies and sugar chains). The target specific substance may be any substance if it provides the liposome with target specificity without destruction of the liposome. Those skilled in the art can determine the target specific substance to be bound to the liposome properly depending on the target substance.

The liposome encapsulating an ammine platinum complex for use in the composition according to the present invention may be used in any form, as described above in the sections of (production method) and (liposome encapsulating an ammine platinum complex).

The composition according to the present invention may optionally contain suitable formulation materials and a pharmaceutically acceptable carrier. Suitable formulation materials or pharmaceutically acceptable carriers include, but are not limited to: antioxidants, preservatives, colorants, fluorescent colorants, flavoring agents, diluents, emulsifiers, suspending agents, solvents, fillers, bulking agents, buffering agents, delivery vehicles and/or pharmaceutical adjuvants. Typically, the composition according to the present invention is administered in the form of a composition containing an ammine platinum complex, and optionally other active ingredients with at least one physiologically acceptable carrier, diluent or excipient. For example, the suitable vehicle may be a micelle, injection solution, physiological solution, or artificial cerebrospinal fluid, and such a vehicle may contain other substances commonly used in compositions for parenteral delivery.

The acceptable carrier, excipient or stabilizer for use herein is preferably non-toxic to the recipient and preferably inactive at the dosage and concentration used, and preferable examples thereof include phosphates, citrates or other organic acids; ascorbic acid; a-tocopherol; low-molecular weight polypeptides; proteins (e.g. serum albumin, gelatin and immunoglobulin); hydrophilic polymers (e.g. polyvinylpyrrolidone); amino acids (e.g. glycine, glutamine, asparagine, arginine and lysine); monosaccharides, disaccharides and other carbohydrates (glucose, mannose and dextrin); chelating agents (e.g., EDTA); sugar alcohols (e.g., mannitol and sorbitol); salt-forming counter ions (e.g., sodium); and/or nonionic surfactants (e.g. , Tween, pluronic or polyethylene glycol (PEG)); and the like.

Preferable exemplary carriers include neutrally buffered physiological saline and mixtures of serum albumin and the physiological saline. Preferably, the product is formulated as a freeze-dried agent prepared by using a suitable excipient (e.g., sucrose). Other standard carriers, diluents and excipients may optionally be contained. Another exemplary composition contains a Tris buffering agent at a pH of approximately 7.0 to 8.5 or an acetate buffering agent at a pH of approximately 4.0 to 5.5 and additionally sorbitol or a suitable substitute thereof.

Hereinafter, a general method of producing the composition according to the present invention will be described. It should be noted that animal drug compositions, quasi drugs, aquiculture drug compositions, flood compositions, cosmetics compositions, and the like can also be produced by conventionally known production method.

The composition according to the present invention can be administered parenterally, for example as a liquid preparation such as injection solution, suspension, solution or spray, optionally as formulated with a pharmaceutically acceptable carrier. Examples of the pharmaceutically acceptable carriers include excipients, lubricants, binders, disintegrants, decay inhibitors, absorption accelerators, absorbents, wetting agents, solvents, a dissolving auxiliary, suspending agents, isotonic agents, buffering agents, soothing agents and the like. Additives for preparation, such as antiseptics, antioxidants, colorants, and sweeteners, may optionally be added. In addition, the composition according to the present invention may be formulated of substances other than sialyl Lewis X or lipids. The parenteral administration routes include, but are not limited to, intravenous, intramuscular, subcutaneous administration, intracutaneous administration, mucous membranes administration, intrarectal administration, intravaginal administration, local administration, skin administration, and the like. The medicine for use in the present invention may be a pyrogen-free pharmaceutically acceptable aqueous solution when administered systemically. In the production of such a pharmaceutically acceptable composition, it is within the technical scope of those skilled in the art to consider the pH, isotonicity, stability and other requirements.

Preferable examples in the solvents for liquid preparations include injection solution, alcohol, propylene glycol, macrogol, benne oil, corn oil and the like.

Preferable examples of the dissolving auxiliary in liquid preparations include, but are not limited to: polyethylene glycol, propylene glycol, D-mannitol, benzyl benzoate, ethanol, trisaminomethane, cholesterol, triethanolamine, sodium carbonate, sodium citrate and the like.

Preferable examples of the suspending agents in liquid preparations include surfactants such as stearyltriethanolamine, sodium laurylsulfate, laurylaminopropionic acid, lecithin, benzalkonium chloride, benzethonium chloride, and glycerin monostearate; hydrophilic polymers such as polyvinylalcohol, polyvinylpyrrolidone, sodium carboxymethylcellulose, methylcellulose, hydroxymethylcellulose, hydroxyethylcellulose, hydroxyethylcellulose, and hydroxypropylcellulose; and the like.

Preferable examples of the isotonic agents in liquid preparations include, but are not limited to, sodium chloride, glycerol, D-mannitol and the like.

Preferable examples of the buffering agents in liquid preparations include, but are not limited to, phosphates, acetates, carbonates, citrates and the like.

Preferable examples of the soothing agents in liquid preparations include, but are not limited to, benzyl alcohol, benzalkonium chloride, procaine hydrochloride and the like.

Preferable examples of the antiseptics in liquid preparations include, but are not limited to, paraoxybenzoic esters, chlorobutanol, benzyl alcohol, 2-phenylethyl alcohol, dehydroacetic acid, sorbic acid, and the like.

Preferable examples of the antioxidants in liquid preparations include, but are not limited to, sulfites, ascorbic acid, a-tocopherol, cysteine and the like.

In the production of injection solution, the liquid agents and the suspension agents are preferably sterilized, and the injection solution is preferably isotonic with the blood or the solvent at the injection site for the other purpose. Generally, the injection solution is sterilized, for example, by filtration through a microbe-impermeable filter and the like, formulation of a bactericide, light irradiation or the like. After the treatment, the injection solution may be converted into solid matter for example by a freeze drying method or the like, which is then diluted with sterilized water or sterilized injection diluent (aqueous lidocaine hydrochloride solution, physiological saline, glucose aqueous solution, ethanol or the mixed solution thereof, etc.) immediately before use.

The composition according to the present invention may additionally contain other additives such as colorants, preservatives, flavors, flavoring agents and sweetener, and also other medicines.

The amounts of the substances and the composition for use in the present invention can be determined easily by those skilled in the art, taking into consideration application, treating disease (kind, severity, etc.), age, body weight, sex and anamnesis of the subject, the shape or kind of cell, and the like. The frequency of administering the method according to the present invention to a subject can also be determined easily by those skilled in the art, taking into consideration intended usage, treating disease (kind, severity, etc.), age, body weight, sex and anamnesis of subject, therapeutic process, and the like. For example, the frequency of administration may be once every day to once every several months (e.g. once every week to once every month). Preferably, administration of once a week to once a month is carried out, while the effectiveness of administration is evaluated. The dosage can be determined by estimating the quantity needed for the site to be treated.

### Treatment method

An aspect of the present invention provides a method of treating cancers or tumors. The method includes the step of administering a liposome encapsulating an ammonia-containing ammine platinum complex to a mammal in need of treating cancers or tumors in an effective amount for the treatment thereof, wherein the liposome is obtained by mixing a water-soluble ammine platinum complex raw material and a liposome raw material and subjecting the mixture to liposome-forming/maintaining conditions.

Another aspect of the present invention provides a method of treating cancers or tumors. The method includes the step of administrating a liposome encapsulating an ammonia-containing ammine platinum complex to a mammal in need of treating cancers or tumors in an effective amount for the treatment thereof, wherein the ammine platinum complex is encapsulated in the liposome, for example, in an amount of 0.3 µg, 0.4 µg, 0.5 µg, 0.6 µg, 0.7 µg, 0.8 µg, 0.9 µg, 1.0 µg, 2.0 µg, 3.0 µg, 4.0 µg, 5.0 µg, 6.0 µg, 7.0 µg, 8.0 µg, 9.0 µg, 10.0 µg, 11.0 µg, 12.0 µg, 13.0 µg, 14.0 µg, 15.0 µg, 16.0 µg, 17.0 µg, 18.0 µg, 19.0 µg, or 20.0 µg; preferably 9 µg or 17 µg or more, with respect to 1 mg of the lipid.

In another aspect of the method according to the present invention, the ammonia-containing ammine platinum complex may be contained, for example, in an amount of 10 µg or more, 20 µg or more, 30 µg or more, 40 µg or more, 50 µg or more, 60 µg or more, 70 µg or more, 80 µg or more, 90 µg or more, 100 µg or more, 150 µg or more, 200 µg or more, or 300 µg or more, with respect to 1 mg of the phospholipid.

In another aspect of the method according to the present invention, the ammonia-containing ammine platinum complex may be contained, for example, in an amount of 39 µg/ml, 40 µg/ml, 50 µg/ml, 60 µg/ml, 70 µg/ml, 80 µg/ml or more, 90 µg/ml, or 100 µg/ml, with respect to 1 ml of the liposome.

In another aspect of the method according to the present invention, the ammonia-containing ammine platinum complex may be contained, for example, in an amount of 1×10⁻¹⁰ µg or more, 2×10⁻¹⁰ µg or more, 3×10⁻¹⁰ µg or more, 4×10⁻¹⁰ µg or more, 5×10⁻¹⁰ µg or more, or 10⁻⁹ µg or more in a single liposome.

In an embodiment of the treatment method according to the present invention, the liposome encapsulating an ammonia-containing ammine platinum complex may be administered, for example, by intravenous administration, subcutaneous administration, oral administration, local administration, intraperitoneal administration or the like (preferably, intravenous administration).

In another embodiment of the treatment method according to the present invention, the liposome encapsulating an ammonia-containing ammine platinum complex may be administered as ammine platinum complex in an amount in the range of 10 to 90 mg/m² (e.g., any possible combination in a range of 10 mg/m², 15 mg/m², 20 mg/m², 25 mg/m², 30 mg/m², 35 mg/m², 40 mg/m², 45 mg/m², 50 mg/m², 55 mg/m², 60 mg/m², 65 mg/m², 70 mg/m², 75 mg/m², 80 mg/m², 85 mg/m², or 90 mg/m² or more; and 90 mg/m², 85 mg/m², 80 mg/m², 75 mg/m², 70 mg/m², 65 mg/m², 60 mg/m², 55 mg/m², 50 mg/m², 45 mg/m², 40 mg/m², 35 mg/m², 30 mg/m², 25 mg/m², 20 mg/m², 15 m^{g}/m², or 10 mg/m² or less), preferably, 20 mg/m² to 50 mg/m². The dosage may be, but is not limited to, for example, 15 to 20 mg/m² once a day, 50 mg/m² to 70 mg/m² once a day, 25 mg/m² to 35 mg/m² once a day, 10 mg/m² to 20 mg/m² once a day, 70 mg/m² to 90 mg/m² once a day, or 20 mg/m² once a day.

In another embodiment, the cancers or tumors to be treated by the treatment method according to the present invention include, but are not limited to, for example, testis tumors, bladder cancers, pelvis renalis tumors, ureter tumors, prostate cancers, ovarian cancers, head and neck cancers, non-small cell lung cancers, esophageal cancers, uterine cancers, neuroblastomas, stomach cancers, preferably, prostate cancers, and uterine cancers. Because it is possible to apply the treatment method according to the present invention to various cancers and tumors by adjusting the kind and amount of the platinum complex encapsulated in the liposome, the dosage and the administration form of the liposome, and the like according to the kind and stage of the cancers or tumors.

The liposome encapsulating an ammine platinum complex used in the treatment method according to the present invention may be in any shape, as described above in the sections of production method and liposome encapsulating an ammine platinum complex.

A described herein, the "subject" means an organism to which the treatment according to the present invention is applied, and is also called "patient." The patient or the subject may be, for example, a bird, a mammal, or the like. Preferably, such an animal may be a mammal (e.g., monotreme, marsupial, edentate, dermopteran, chiropteran, carnivores, insectivore, proboscid, perissodactyl, artiodactyla, tubuidentate, pholidota, sirenia, cetacea, primates, rodents, lagomorpha, or the like). Examples of the subjects include, but are not limited to, animals such as human, bovine, pig, horse, chicken, cat and dog. It may be more preferably human.

As described herein, the "effective amount for treating" is a term sufficiently recognizable by those skilled in the art, and is an amount of a medicine giving intended to produce pharmacologic results (e.g., prevention, treatment, recurrence prevention etc.). Thus, the therapeutically effective amount is an amount sufficient for alleviation of the symptoms of a disease to be treated. The effective amount in a particular application (e.g., therapeutically effective amount) can be determined by an assay of determining the recovery of the target disease. The actual dosage depends on the individual to be treated, and is preferably an optimized amount to accomplish desired results without any distinctive adverse reaction. Effective dosage is determined sufficiently by those skilled in the art.

The therapeutically effective amount, the preventively effective amount and the toxicity can be determined according to standard pharmaceutical procedures for testing cell cultures or experimental animals (e.g., ED50, a therapeutically effective amount dosage in 50% of a population; and LD50, a lethal dosage to 50% of a population). The dosage ratio of the therapeutic effect to the toxicity effect, i.e. therapeutic coefficient, is expressed by a ratio of ED50/LD50. Preferable is a drug delivery medium having a large therapeutic coefficient. Data obtained by cell culture assays and animal experiments are used in formulating the dosage for usage in human. Dosage of the compound is preferably within a range of circulation concentration having an ED50 at which there is almost no or completely no toxicity. The dosage varies in the range above, according to the administration form used, patient's sensitivity, and administration route. For an example, the dosage is selected properly according to the conditions of the patient such as age, the kinds of the disease and the cell used, and the like.

### Pharmaceutical application

An aspect of the present invention provides a liposome encapsulating an ammonia-containing ammine platinum complex for use as a medicine. The liposome can be prepared by mixing water-soluble ammine platinum complex raw materials and liposome raw materials and subjecting the mixture to liposome-forming/maintaining conditions.

Another aspect of the present invention provides a liposome encapsulating an ammonia-containing ammine platinum complex for use as a medicine. The liposome contains the ammine platinum complex at any concentration or level described in other sections of the present specification.

The liposome for use as a medicine according to the present invention may be used in any shape, as described in the above sections of production method and liposome encapsulating an ammine platinum complex.

The present invention has been exemplified so far with reference to preferable embodiments of the present invention, but it should not be construed that the present invention is restricted by the embodiments of the present invention. It should be understood that the scope of the present invention should be construed only by the claims. It would be understood that those skilled in the art can perform an invention practically equivalent to the present invention, based on the description of the present specification and technical common sense from the description of typical preferable embodiments of the present invention. It would be understood that the patents, patent applications and literatures cited herein are incorporated herein by reference to the present specification, similarly to the case where the description is described specifically herein.

Hereinafter, the configurations of the present invention will be described more specifically with reference to Examples, but it should be understood that the present invention is not limited thereby. Reagents used below were commercially available products, unless specified otherwise.

### EXAMPLES

### Example 1: Preparation of liposome encapsulating cis-diammine dichloroplatinum (II)

### (Synthesis of cis-diamine dinitratoplatinum (II) (cisplatin-nitrate))

4.15 g (10.0 mmol) of platinum potassium tetrachloride (K₂ PtCl₄) and 6.64 g (40 mmol) of potassium iodide were dissolved completely in 50 ml of sufficiently oxygen-free distilled water; 1.35 ml (22 mmol) of 28% aqueous ammonia solution was added thereto under nitrogen atmosphere; and the mixture was agitated, to give a precipitate. The precipitate was washed with water and ethanol respectively twice and dried at room temperature under vacuum (to 20 mm Hg), to give 4.49 g of an ammine-platinum intermediate cis-[Pt(NH₃)₂I₂]. 2.41 g (5.0 mmol) of the ammine-platinum intermediate cis-[Pt(NH₃)₂I₂] was suspended in 30 ml of distilled water; 1. 68 g (9.9 mmol) of silver nitrate was added thereto; and the mixture was agitated in a dark environment for 24 hours. The silver iodide generated was filtered and the filtrate was concentrated in an evaporator, to give a white crystal. The white crystal was washed with cold distilled water and cold ethanol twice, to give 1.01 g of colorless cisplatin-nitrate, cis-[Pt(NH₃)₂(NO₃)₂].

### (Preparation of liposome encapsulating cis-diamine dinitratoplatinum (II))

Dipalmitoyl phosphatidylcholine (DPPC), cholesterol, ganglioside, dicetyl phosphate (DCP), and dipalmitoyl phosphatidylethanolamine (DPPE) were mixed at a molar ratio of 35:40:5:15:5, to give 45.6 mg of a lipid; 46.9 mg of sodium cholate was added thereto; and the mixture was dissolved in 3 ml of methanol-chloroform solution (1:1). The mixture was agitate at 37°C for 1 hour, and methanol and chloroform were removed by a rotary evaporator; the residue was vacuum-dried. The lipid membrane thus obtained was suspended in 3 ml of NaCl-free N-tris(hydroxymethyl)-3-aminopropane sulfonate buffer solution (pH: 8.4), and the mixture was agitated at 37°C for 1 hour. After nitrogen substitution, the solution was ultrasonicated, to give a transparent micellar suspension. Then, 54.1 mg of cis-diamine dinitratoplatinum (II) was weighed and dissolved in 7 ml of NaCl-free N-tris(hydroxymethyl)-3-aminopropane sulfonate buffer solution (pH: 8.4); the mixture was then mixed with the micellar suspension; the mixture was then ultrafiltered (molecular weight cutoff: 10,000) by using PM10 (Amicon Co., USA) and NaCl-free N-tris(hydroxymethyl)-3-aminopropane sulfonate buffer solution (pH: 8.4), to give 10 ml of a homogeneous liposome (35.5 mg lipid).

### (Confirmation of encapsulation of cis-diamine dinitratoplatinum (II) in liposome)

The presence of cis-diamine dinitratoplatinum (II) in the liposome prepared in the present Example was confirmed by using a flameless atomic absorption spectroscopy (FAAS).

The flameless atomic absorption spectroscopy (FAAS) used was AA-6700 Atomic absorption Flame emission spectrophotometer (Shimadzu). The sample was processed sequentially at 120° C for 30 seconds, at 250°C for 10 seconds, at 700°C for 20 seconds, at 700°C for 5 seconds, and at 2600°C for 3 seconds under the condition of a wavelength of 265.9 nm, a slit width of 0.5, and a lamp current of 14 mA. A platinum standard solution (1 mg-Pt/ml, 1000 ppm) was diluted with purified water, to give solutions at concentrations of 12.5 ng/ml, 25 ng/ml, 50 ng/ml, 100 ng/ml and 200 ng/ml for preparation of a calibration curve. The liposome solution was diluted by 10,000 times with purified water to give a test sample.

### (Result)

The liposome prepared in the present Example encapsulated cis-diamine dinitratoplatinum (II) in an amount of 323.33 µg/ml (91.08 µg/mg lipid). The lipid content of the liposome obtained was 35.5 mg. The encapsulating rate of the cis-diamine dinitratoplatinum (II) in liposome was 5.9%. The solution containing the cis-diamine dinitratoplatinum (II)-containing liposome was colorless.

### (Conversion of cis-diamine dinitratoplatinum (II) to cis-diamine dichloroplatinum (II))

The liposome confirmed to encapsulate cis-diamine dinitratoplatinum (II) is subjected to N-tris(hydroxymethyl)-3-aminopropane sulfonate buffer solution (pH: 8.4), carbonate buffer solution (pH: 8.5), PBS (pH: 8.0), or HEPES buffer solution (pH: 7.2) at 25°C for 96 hours. All of these buffer solutions contain NaCl at a final concentration of 150 mM.

As a result, it is found that the colorless solution turns pale yellow, indicating that the cis-diamine dinitratoplatinum (II) is converted to cis-diamine dichloroplatinum (II). The change can also be detected by absorption spectroscopy, IR method, or ¹⁹⁵Pt-NMR method.

### (Absorption spectroscopy)

(1) NaCl in the cis-diamine dinitratoplatinum (II)-encapsulating liposome processed in a buffer containing NaCl at a final concentration of 150 mM for 96 hours is removed by buffer exchange, and the resulting solution is concentrated 10 times. (2) The solution is heated at 100°C for 30 minutes (for the destruction of liposome) and extracted with chloroform. (3) The aqueous layer is recovered. (4) The absorption spectrum of the recovered aqueous layer in the wavelength range of 200 to 400 nm is obtained. (5) The liposome immediately after incorporation of cis-diamine dinitratoplatinum (II) (before subjection to 150 mM NaCl) is also analyzed in the operations (2) to (4). (6) Comparison of the absorption spectra of the cis-diamine dinitratoplatinum (II) liposome subjected in the buffer for 96 hours and the liposome immediately after incorporation cis-diamine dinitratoplatinum (II) confirms that cis-diamine dinitratoplatinum (II) is converted to cis-diamine dichloroplatinum (II).

### (IR method)

Solvent extraction was performed similarly to absorption spectroscopy. After recovery of the aqueous layer, water is evaporated by a rotary evaporator. IR spectra of the cis-diamine dinitratoplatinum (II)-containing liposome after subjecting in the buffer containing NaCl at a final concentration of 150 mM for 96 hours and liposome immediately after incorporation of cis-diamine dinitratoplatinum (II) (before subjection to 150 mM NaCl) are obtained by the Nujol method, and are compared.

The platinum complex in the cis-diamine dinitratoplatinum (II) form has Pt-O-H bending vibration in the spectrum in the region of 950 to 1100 cm⁻¹. That in the cis-diamine dichloroplatinum (II) form, which has only Pt-Cl bonds, has no band in the region of 950 to 1100 cm⁻¹. In this way, the conversion from cis-diamine dinitratoplatinum (II) to cis-diamine dichloroplatinum (II) can be confirmed.

IR methods concerning platinum complexes are described in detail, for example, in R. Fraggiani, B. Lippert, C. J. Lock and B. Rosenberg, J. Am. Chem. Soc., 99, 777 (1977) and F. D. Rochon, A. Morneau and R. Melason, Inorg. Chem. , 27, 10 (1988).

### (Conversion of nitrate encapsulated in liposome to cisplatin)

### (Analysis by ¹⁹⁵PtNMR spectrum)

Conversion from the nitrate contained in the liposome to cisplatin is analyzed by ¹⁹⁵PtNMR spectrum. The sensitivity of the ¹⁹⁵PtNMR spectrum is 20 times higher than that of natural ^{13C}-NMR spectrum. In addition, the natural abundance ratio of ¹⁹⁵Pt is 33.8%, and the relative sensitivity of naturally present platinum to ¹H is relatively high at 3.4×10⁻³. Further because the peak is observed in an extremely wide frequency region at a chemical shift of 1500 ppm, the analytical tool is extremely useful in studying the structure and bonding characteristics.

### (Sample preparation)

Samples of cisplatin, (cis-diammine dinitratoplatinum (II)) nitrate and a liposome encapsulating (cis-diammine dinitratoplatinum (II)) nitrate are prepared in the following manner.

Cisplatin: 2 mg of cisplatin (M.W.: 300) is dissolved in 1 ml of D₂O, to give a sample (6.6 mM).
(Cis-diammine dinitratoplatinum (II)) nitrate: 5 mg of a nitrate (M.W. : 365) is dissolved in 1 ml of D₂O, to give a sample (13.7 mM).
(Cis-diammine dinitratoplatinum (II)) nitrate-encapsulating liposome (NaCl⁺): the nitrate is used at a Pt concentration of 4.75 mM.

### (Measurement method)

Na₂PtCl₆ (sodium hexachloroplatinate (IV)) is dissolved in D₂O, to give an external standard solution. The NMR apparatus used is INOVA-600 (Varian). The sample is loaded in a measurement tube having a diameter of 5 mm for measurement.

In the measurement method, cisplatin, measured as a control, has a chemical shift of -2160 ppm, while the (cis-diammine dinitratoplatinum (II)) nitrate has a chemical shift of -1620 ppm. Thus, (cis-diammine dinitratoplatinum (II)) nitrate encapsulated in the desirable liposome has a chemical shift of -2160 ppm identical with that of cisplatin, when the external solution contains NaCl. It is possible to confirm whether the (cis-diammine dinitratoplatinum (II)) nitrate encapsulated is substituted by the Cl⁻ derived from sodium chloride in the buffer solution and converted to cisplatin, for example by measuring the chemical shift thereof.

### Comparative Example 1. Influence of sodium chloride ion

DPPC, cholesterol, ganglioside, DCP and DPPE were mixed at a molar ratio of 35:40:5:15:5, to give 45.6 mg of a lipid; 46.9 mg of sodium cholate was added thereto; and the mixture was dissolved in 3 ml of methanol-chloroform solution (1:1). The mixture was agitated at 37° C for 1 hour, and, after removal of methanol and chloroform by vaporization in a rotary evaporator, the residue was dried under vacuum. The lipid membrane obtained was suspended in 3 ml of NaCl-containing N-tris(hydroxymethyl)-3-aminopropane sulfonate buffer solution (pH: 8.4), and the mixture was agitated at 37°C for 1 hour. After nitrogen substitution, the solution was ultrasonicated, to give a transparent micellar suspension. Then, 54.1 mg of cis-diamine dinitratoplatinum (II) was weighed and dissolved in 7 ml of NaCl-containing N-tris(hydroxymethyl)-3-aminopropane sulfonate buffer solution (pH: 8.4); the mixture was additionally mixed with a micellar suspension, and ultrafiltered by using PM10 (Amicon Co., USA) and NaCl-containing N-tris(hydroxymethyl)-3-aminopropane sulfonate buffer solution (pH: 8.4) (molecular weight cutoff: 10,000), to give 10 ml of a homogeneous liposome.

Cis-diamine dinitratoplatinum (II) was not encapsulated into the liposome when NaCl-containing N-tris(hydroxymethyl)-3-aminopropane sulfonate buffer solution (pH: 8.4) was used. There were precipitates several tens of minutes after solubilization in the N-tris(hydroxymethyl)methyl-3-aminopropane sulfonate (TAPS) buffer solution. The precipitates were analyzed by absorbance method. UV2500PC (Shimadzu) absorptometer was used for measurement.

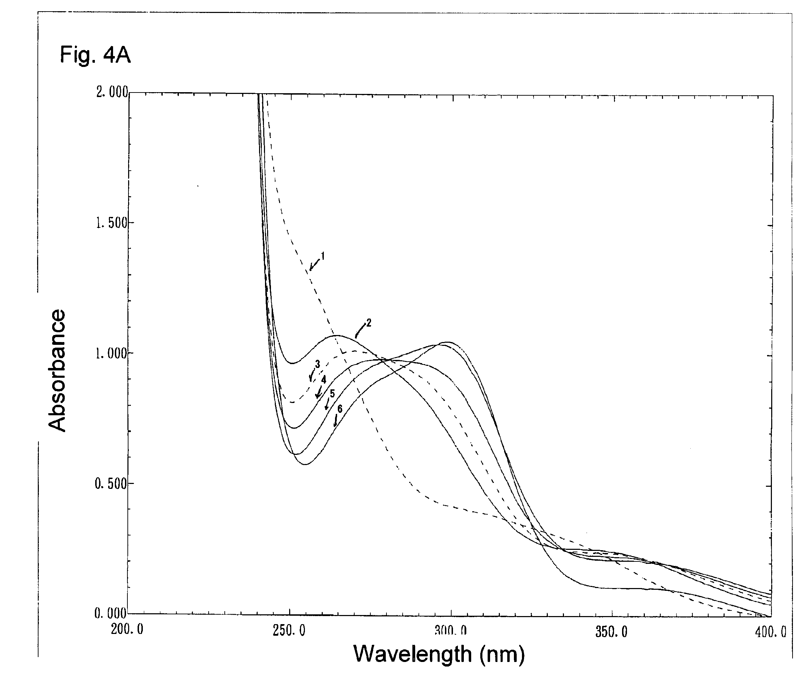

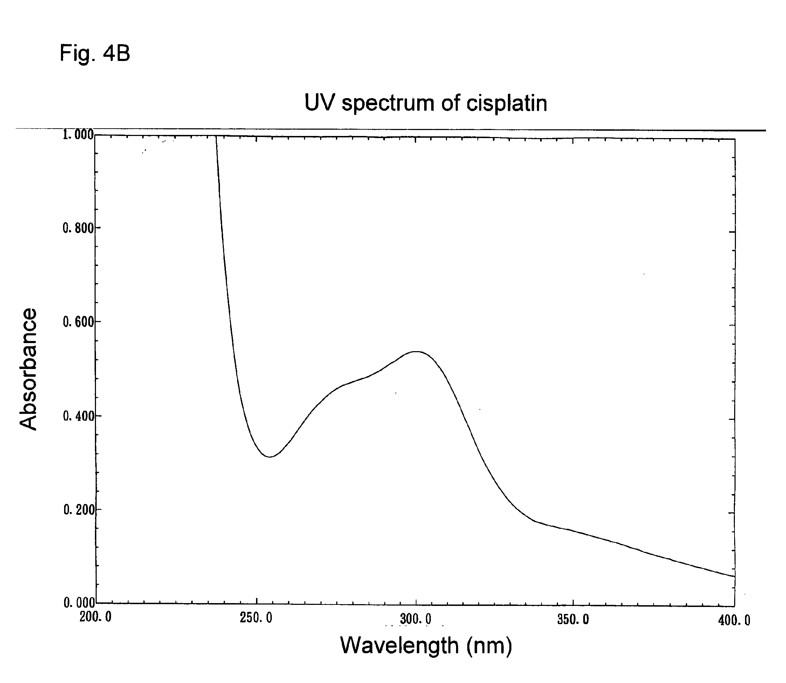

Five mg/ml of cis-diamine dinitratoplatinum (II) was dissolved in a TAPS buffer solution containing 150 mM of sodium chloride (pH: 8.4); the solution was stationarily placed at 25°C; and the UV spectrum of the solution was determined over time (0 minute, 5 minutes, 10 minutes, 15 minutes, 30 minutes and 45 minutes). One mg of cis-diamine dichloroplatinum (II) was dissolved in 1 ml of sodium chloride-containing TAPS buffer solution, to give a control, and the UV spectrum thereof was obtained. As shown in Figs. 4A and 4B, the absorption characteristics of the UV spectrum after 45 minutes were the same as those of cisplatin. There was no change in UV spectrum, when the sample was stored without movement for 45 minutes or more. Thus, it was found that cis-diamine dinitratoplatinum (II) is converted to cis-diamine dichloroplatinum (II) if the aqueous solution of cis-diamine dinitratoplatinum (II) was dissolved in TAPS containing 150 mM NaCl and the mixture was placed without movement at 25° C for 45 minutes. This probably occurs because the presence of NaCl leads to the conversion of cis-diamine dinitratoplatinum (II) to the hardly water-soluble cis-diamine dichloroplatinum (II) and the subsequent precipitation of the latter.

### Example 2. quantitative analysis of liposome

### (I. Quantitative determination of lipid)

The lipid component content of the liposome was calculated by quantitative determination of cholesterol content. For quantitative determination of lipids, Determiner TC555 kit (catalog number: UCC/EAN128) (KYOWA Co. Ltd,) was used. 50 mg/ml of the cholesterol attached to the kit was used as the standard substance.

A standard substance (50 mg/ml: cholesterol) was diluted with PBS buffer solution to give standard solutions containing cholesterol at concentrations of 0 µg, 0.1 µg, 0.25 µg, 0.5 µg, 0.75 µg, 1 µg, 5 µg, 10 µg and 20 µl. The cis-diamine dinitratoplatinum (II)-encapsulating liposome was diluted five times with PBS buffer solution, to give an analyte solution. The standard solutions and the analyte solution were respectively placed in a test tube in an amount of 20 µl. 17 µl of Triton X-100 (10% solution) was added to each test tube, and the mixture was agitated and then left still at 37°C for 40 minutes. 300 µl of the enzyme reagent of Determiner TC555 kit was added thereto, and the mixture was agitated and then left still at 37°C for 20 minutes. The lipid content was obtained by drawing a calibration curve with the standard solutions by measuring the absorbance at 540 nm and measuring the cholesterol content in the liposome.
Conversion formula for obtaining lipid content from cholesterol content:
Lipid content (µg/50 µl) = Cholesterol content (µg/50 µl) × 4.51 (conversion factor)
The lipid content of the obtained liposome was 3.55 mg/ml.

### (II. Particle diameter distribution and particle diameter)

Liposome particles were diluted 50 times with purified water, and the suspension was analyzed by using Zetasizer Nano (Nan-ZS: MALVERN Co., Ltd.).

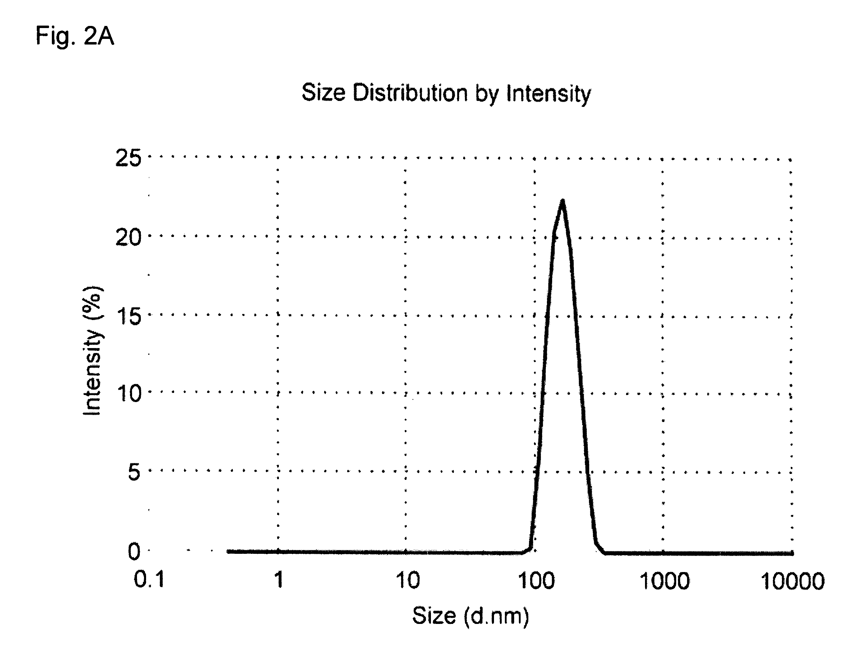

The average particle diameter of the liposome particles was 159 nm (Fig. 2A).

### Example 3. Preparation of sugar chain-bound cis-diamine dinitratoplatinum (II)-containing liposome

A cis-diamine dinitratoplatinum (II)-containing liposome was prepared in a manner similar to the cis-diamine dinitratoplatinum (II)-containing liposome in Example 1. The liposome prepared in the present Example contained cis-diamine dinitratoplatinum (II) in an amount of 323.33 µg/ml (91.08 µg/mg lipid). The lipid content of the obtained liposome was 35.5 mg. The incorporation rate of the cis-diamine dinitratoplatinum (II) into the liposome was 5.9%. The solution containing the cis-diamine dinitratoplatinum (II)-containing liposome was colorless.

### (Hydrophilization of the liposome lipid membrane surface)

Ten ml of the solution of cis-diamine dinitratoplatinum (II)-containing liposome was ultrafiltered by using a XM300 membrane (Amicon Co., USA) and carbonate buffer solution (pH: 8.5) (molecular cutoff rate: 300,000), and the resulting solution was adjusted to pH 8.5. Then, 10 mg of a crosslinking agent bis(sulfosuccinimidyl) suberate (BS3; Pierce Co., USA) was added thereto, and the mixture was agitated at room temperature for 2 hours. Then, the mixture was agitated overnight in a refrigerator, allowing completion of the reaction between the lipid dipalmitoyl phosphatidylethanolamine on the liposomal membrane with BS3. The liposome solution was then ultrafiltered by using a XM300 membrane and carbonate buffer solution (pH: 8.5) (molecular cutoff rate: 300,000). Then, 40 mg of tris(hydroxymethyl)aminomethane dissolved in 1 ml of carbonate buffer solution (pH: 8.5) was added to 10 ml of the liposome solution. The solution was then agitated at room temperature for 2 hours and additionally overnight in a refrigerator; the solution was then ultrafiltered at a molecular cutoff rate of 300,000 for removal of free tris(hydroxymethyl)aminomethane was removed; the carbonate buffer solution was replaced with N-tris(hydroxymethyl)-3-aminopropane sulfonate buffer solution (pH: 8.4); and the BS3 bound to the lipid on the liposomal membrane and the tris(hydroxymethyl)aminomethane were allowed to react with each other completely, to make the lipid dipalmitoyl phosphatidylethanolamine on the liposomal membrane coordinated with the hydroxyl groups of tris(hydroxymethyl)aminomethane and thus to give a membrane surface-hydrated and hydrophilized.

### (Binding of human serum albumin (HSA) onto the liposomal membrane surface)

Binding of the human serum albumin (HSA) onto the liposomal membrane surface was carried out by a known method of a coupling reaction (Yamazaki, N., Kodama, M. and Gabius, H.-J. (1994) Methods Enzymol. 242, 56-65). Specifically, the reaction was carried out by a two-phase chemical reaction. First, 10 ml of the ganglioside present on the liposomal membrane surface obtained in the present Example was added to 43 mg of sodium metaperiodate dissolved in 1 ml of N-tris(hydroxymethyl)-3-aminopropane sulfonate buffer solution (pH: 8.4); and the mixture was agitated overnight in a refrigerator, allowing progression of the periodate oxidation. The mixture was ultrafiltered by using a XM300 membrane and PBS buffer solution (pH: 8.0) (molecular cutoff rate: 300,000) for removal of free sodium periodate; and the N-tris(hydroxymethyl)-3-aminopropane sulfonate buffer solution was replaced with PBS buffer solution (pH: 8.0), to give 10 ml of an oxidized liposome. Twenty mg of human serum albumin (HSA)/PBS buffer solution (pH: 8.0) was added to the liposome solution; the mixture was agitated at room temperature for 2 hours; then, 100 µl of 2 M NaBH₃CN/PBS buffer solution (pH: 8.0) was added thereto; the mixture was agitated at room temperature for 2 hours and additionally overnight in a refrigerator, allowing progression of the coupling reaction between the ganglioside on the liposome and HSA and thus binding of HSA. Then, the mixture was ultrafiltered (molecular cutoff rate: 300,000) for removal of the free sodium cyanoborate and human serum albumin, and the buffer solution was replaced with a carbonate buffer solution (pH: 8.5), to give 10 ml of a HSA-bound liposome solution.

### (Preparation of sugar chain)

The sugar chain used was sialyl Lewis X.

The mass of each sugar chain was determined, and the sugar chain was pretreated before use in the tests below.

### (Binding of sugar chain to human serum albumin (HSA) bound to liposomal membrane surface)

Two mg of each sugar chain prepared in the present Example was dissolved in purified water; the solution was added to 0.5 ml of aqueous solution containing 0.25 g of NH₄ HCO₃; the mixture was agitated at 37° C for 3 days; the mixture was filtered through a 0.45-µm filter; and then, the reductive terminal of the sugar chain was allowed to react in amination reaction, to give 4 mg/ml of a glycosylamine compound of each sugar chain (aminated sugar chain solution). Then, 10 mg of a crosslinking agent 3,3'-dithiobis(sulfosuccinimidyl propionate) (DTSSP; Pierce Co., USA) was added to 10 ml of part of the liposome solution obtained in the present Example; the mixture was agitated at room temperature for 2 hours and additionally overnight in a refrigerator; the mixture was ultrafiltered by using a XM300 membrane and carbonate buffer solution (pH: 8.5) (molecular cutoff rate: 300,000) for removal of free DTSSP, to give a 10 ml of liposome wherein DTSSP is bound to the HSA on the liposome. Subsequently, the glycosylamine compound (aminated sugar chain solution) was added to the liposome solution in an amount of 12.5, 37.5, 125, 250, 500, 1250, or 2500 µl; the mixture was agitated at room temperature for 2 hours; a tris(hydroxymethyl)aminomethane/carbonate buffer solution (pH: 8.5) was added thereto; the mixture was then agitated in a refrigerator overnight, allowing binding of the glycosylated amine compound to the DTSSP on the human serum albumin of the liposomal membrane surface. The mixture was ultrafiltered by using a XM300 membrane and HEPES buffer solution (pH: 7.2) (molecular cutoff rate: 300,000) for removal of free sugar chain and tris(hydroxymethyl)aminomethane, to give 10 ml of each liposome to which both the sugar chain and the human serum albumin are bound.

### (Hydrophilization of human serum albumin (HSA) bound to the liposomal membrane surface)

The HSA protein surface of each of the liposomes to which the sugar chain prepared in the present Example is bound was hydrophilized separately by the following procedure: 26.4 mg of tris(hydroxymethyl)aminomethane was added to 10 ml of each liposome; the mixture was agitated at room temperature for 2 hours and additionally overnight in a refrigerator; the mixture was ultrafiltered by using a XM300 membrane and HEPES buffer solution (pH: 7.2) (molecular cutoff rate: 300,000) for removal of unreacted materials. The mixture was filtered through a 0.45pm filter, to give a final product, 10 ml of each liposome complex to which a hydrophilized sugar chain is bound (total lipid content: 15.2 mg, total protein content: 1100 µg, and average particle diameter: 171 nm).

### (Confirmation of encapsulation of cis-diamine dichloroplatinum (II) in liposome)

The solution containing the sugar chain-bound liposome prepared in the present Example changed form colorless to pale yellow, indicating that the cis-diamine dinitratoplatinum (II) encapsulated in the liposome was converted to cis-diamine dichloroplatinum (II). In addition, the change can be detected by absorption spectroscopy, IR method, or ¹⁹⁵Pt-NMR method.

### (Conversion of nitrate contained in liposome to cisplatin)

### (Analysis by ¹⁹⁵PtNMR spectrum)

The conversion of the nitrate contained in the liposome to cisplatin was analyzed by ¹⁹⁵PtNMR spectrum.

### (Sample preparation)

Samples of cisplatin, (cis-diammine dinitratoplatinum (II)) nitrate and a liposome encapsulating (cis-diammine dinitratoplatinum (II)) nitrate were prepared in the following manner:
Cisplatin: 2 mg of cisplatin (M.W.: 300) was dissolved in 1 ml of D₂O, to give a sample (6.6 mM).
(Cis-diammine dinitratoplatinum (II)) nitrate: 5 mg of a nitrate (M.W.: 365) was dissolved in 1 ml of D₂O, to give a sample (13.7 mM).
(Cis-diammine dinitratoplatinum (II)) nitrate-containing liposome (NaCl⁺):the nitrate was used at a Pt concentration of 4.75 mM.

### (Measurement method)

Na₂ PtCl₆ (sodium hexachloroplatinate (IV)) was dissolved in D₂O, to give an external standard solution. The NMR apparatus used was INOVA-600 (Varian). 600 µl of a sample was placed in a test tube having a diameter of 5 mm for measurement.

### (Results)

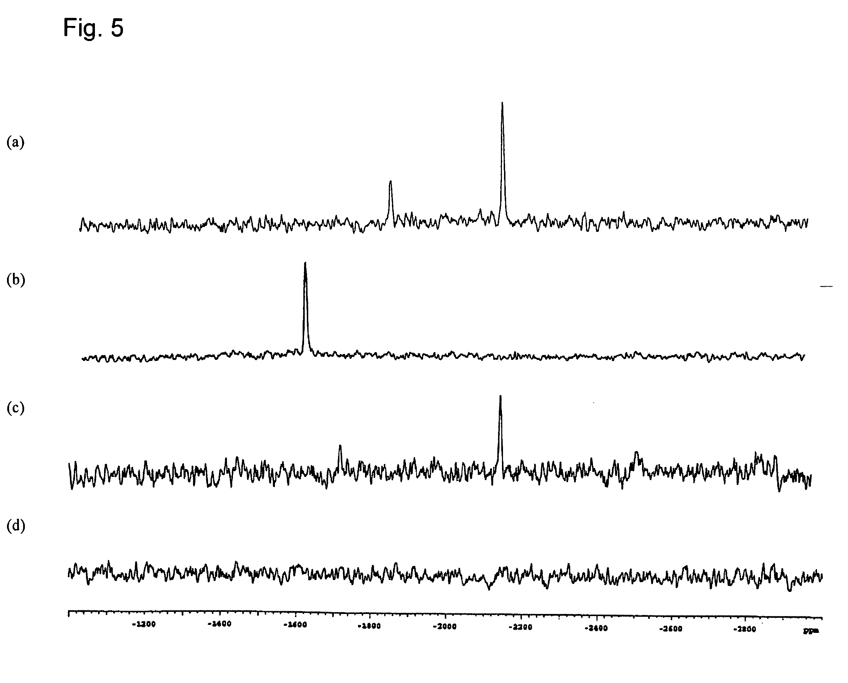

Cisplatin, a comparative compound, had a chemical shift of -2160 ppm, while the (cis-diammine dinitratoplatinum (II)) nitrate, had a chemicals shift of -1620 ppm (Fig.s 5a and 5b). The desired (cis-diammine dinitratoplatinum (II)) nitrate contained in the liposome had a chemical shift of -2160 ppm identical with that of cisplatin, when the external solution contained NaCl (Fig. 5c). The results showed that the nitrate (cis-diammine dinitratoplatinum (II)) contained therein was replaced by the Cl⁻ ion derived from sodium chloride in the buffer solution and converted to cisplatin. In addition, when the liposome-encapsulated cis-diammine dinitratoplatinum (II) was not converted to cis-diamine dichloroplatinum (II), there was no observed peak at the cis-diammine dinitratoplatinum (II) chemical shift of -1620 ppm or at the cis-diamine dichloroplatinum (II) chemical shift of -2160 ppm (Fig. 5d).

Cis-diamine dichloroplatinum (II) was encapsulated in the liposome prepared in the present Example in an amount of 253.98 µg/ml (78.38 µg/mg lipid). The lipid content of the obtained liposome was 32.4 mg. The incorporation rate of cis-diamine dichloroplatinum (II) into the liposome was 4.59%.

The amount of cisplatin encapsulated in the liposome produced by the method described in Patent Document 1 was 8.9 µg/mg-lipid, and the lipid content of the obtained liposome was 200 mg. Thus, the amount of cisplatin encapsulated in the liposome produced by the present method is larger by approximately 9 times than that of the liposome of Patent Document 1. In addition, 54.1 mg of cis-diamine dinitratoplatinum (II) (46.0 mg as cisplatin) is used in preparation of the liposome by the method of the present Example, while cisplatin is used in preparation of the liposome in an amount of 100 mg in Patent Document 1, approximately twice larger than that above. Thus, the incorporation efficiency per lipid content in the liposome produced by the method of the present invention is approximately 18 timers greater, when calculated from the amount of cisplatin contained and the amount of cisplatin used. In addition, the method according to the present invention does not include a heating step, and thus, the liposome produced may be more stable.

Alternatively, the liposome described in Nonpatent Literature 1 contains cisplatin in an amount of 14.0 µg/mg lipid. Thus, the content of cisplatin in the liposome produced by the present method is approximately 5.6 times greater than that of the liposome described in Nonpatent Literature 1.

The results show that the cisplatin-containing liposome prepared by the present method can contain cisplatin in a greater amount than liposome prepared by conventional methods.

### Example 4. Quantitative analysis of liposome

In the present Example, the sugar chain-bound cis-diamine dinitratoplatinum (II)-containing liposome prepared in Example 3 was analyzed quantitatively.

### (I. Quantitative determination of protein)

The total protein content of HSA encapsulated in liposome and HSA bound to the liposome surface was determined by BCA method.

The amount of protein is determined by using Micro BCA^{™} Protein Assay Reagent Kit (catalog number: 23235BN) (PIERCE Co., Ltd.). Two mg/ml of albumin (BSA) attached to the kit was used as the standard substance.

A standard substance (2 mg/ml: albumin) was diluted with PBS buffer solution to give standard solutions at concentrations of 0 µg, 0.25 µg, 0.5 µg, 1 µg, 2 µg, 3 µg, 4 µg and 5 µg/50 µl. The cis-diamine dinitratoplatinum (II)-containing liposome was diluted 20 times with PBS buffer solution, to give an analyte solution. The standard solutions and the analyte solution were respectively placed in test tubes each in an amount of 50 µl. 100 µl of 3% sodium laurylsulfate solution (SDS solution) was added to each test tube. Reagents A, B and C attached to the kit were mixed at a ratio of reagent A:reagent B:reagent C of 48:2:50, and the mixture was added to each test tube in an amount of 150 µl. The test tube was left still at 60°C for 1 hour. After the test tube was cooled to room temperature, the absorbance at 540 nm was determined; a calibration curve was drawn by using the standard solutions; and the protein content in the liposome was determined. The protein content of the liposome was 110 µg/ml.

### (II. Quantitative determination of lipid)

The lipid component content of the liposome was calculated by quantitative determination of cholesterol content. For quantitative determination of lipids, Determiner TC555 kit (catalog number: UCC/EAN128) (KYOWA Co., Ltd.) was used. 50 mg/ml cholesterol attached to the kit was used as the standard substance.

A standard substance (50 mg/ml: cholesterol) was diluted with a PBS buffer solution to give standard solutions containing cholesterol at concentrations of 0 µg, 0.1 µg, 0.25 µg, 0.5 µg, 0.75 µg, 1 µg, 5 µg, 10 µg and 20 µl. The sugar chain-bound cis-diamine dinitratoplatinum (II)-containing liposome prepared in Example 3 was diluted five times with PBS buffer solution, to give an analyte solution. The standard solutions and the analyte solution were respectively placed in a test tube in an amount of 20 µl. 17 µl of Triton X-100 (10% solution) was added to each test tube; the mixture was agitated and then left still at 37° C for 40 minutes. 17 µl of the enzyme reagent of Determiner TC555 kit was added to each test tube; the mixture was agitated and then left still at 37° C for 20 minutes. The lipid content was obtained by drawing a calibration curve with the standard solutions by measuring the absorbance at 540 nm and measuring the cholesterol content in the liposome.
Conversion formula for obtaining lipid content from cholesterol content:
Lipid content (µg/50 µl) = Cholesterol content (µg/50 µl) × 4.51 (conversion factor)
The lipid content of the obtained liposome was 3.24 mg/ml.

### (III. Particle diameter distribution and particle diameter)

The liposome particles were diluted 50 times with purified water and the suspension was analyzed by using Zetasizer Nano (Nan-ZS: MALVERN Co., Ltd.).

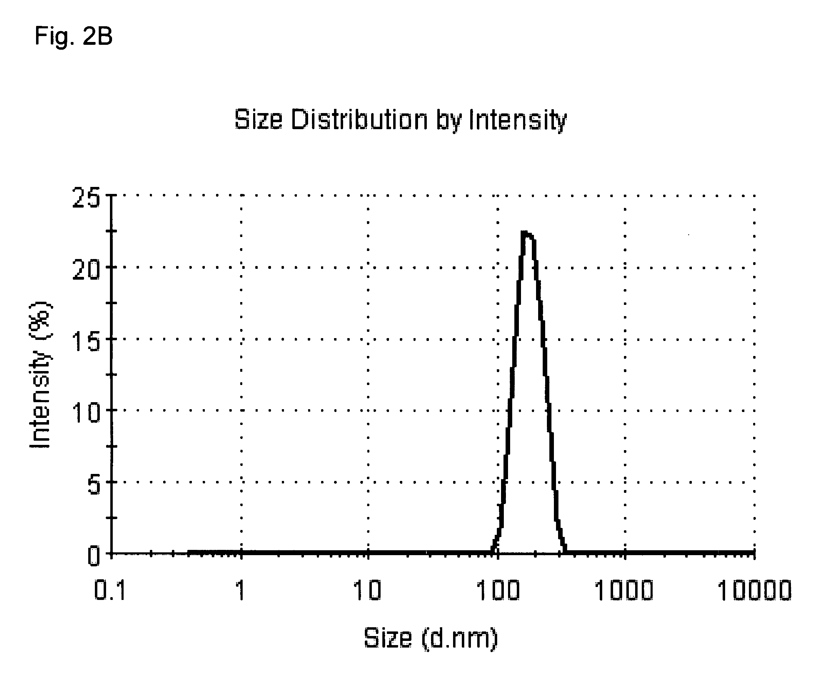

The average particle diameter of the liposome particles was 171 nm (Fig. 2B).

### Example 5: Preparation of antibody-bound liposome encapsulating cis-diamine dichloroplatinum (II)

A cis-diamine dinitratoplatinum (II)-containing liposome was prepared in a manner similar to the "Preparation of cis-diamine dinitratoplatinum (II)-containing liposome" of Example 1. The solution containing the cis-diamine dinitratoplatinum (II)-containing liposome was colorless.

### (Hydrophilization of lipid membrane surface)

The surface of the liposome lipid membrane surface is hydrophilized in a manner similar to Example 1.

### (Preparation of target specific substance)

The target specific substance used was a tumor-specific antibody (E-selectin antibody (AF575) of R&D systems (MN, USA). The substance was pretreated for use as described below. A hybridoma cell line (Number: CRL-2515CL3) producing an anti-human E-selectin mouse monoclonal antibody was purchased from ATCC and purified. The cells were cultured in RPMI1640 (GIBCO code. 11875)/10%FBS/ penicillin 100 unit/ml-streptomycin 100 µg/ml (Antibiotic Antimycotic solution, stabilized SIGMA A5955) (fetal calf serum SIGMA F0926)) at 37°C under 5% CO₂ environment. Pristane (500 µl/mouse) was administered twice (5 day interval) to Balb/c mice (Japan SLC Inc. , female, 6-week old); 5×10⁶ hybridoma cells were suspended in PBS; and 1 ml of the suspension was injected intraperitoneally. The mice were subjected to laparotomy, 14 days after injection for collection of ascites. Ammonium sulfate (0.4 fold of saturation) was added to the ascites; the mixture was centrifuged at 10,000 rpm for 30 minute (4°C); the sediment was redissolved in physiological saline; and the solution was dialyzed (3 days, 4°C). The solution was centrifuged at 10,000 rpm for 30 minute (4°C); two volumes of 0.06 M acetate buffer solution (pH: 4.8) was added to the supernatant; n-caprylic acid was added to a final concentration of 6.8%; and the mixture was agitated at room temperature for 30 minutes. The mixture was centrifuged at 10,000 rpm for 30 minute (0 to 4°C) and dialyzed (3 days, 4°C). The solution was centrifuged at 10,000 rpm for 30 minute (0 to 4°C) and the supernatant recovered.

### (Binding of antibody to human serum albumin (HSA) bound to the liposomal membrane surface)

Ten mg of a crosslinking agent 3,3'-dithiobis(sulfosuccinimidyl propionate) (DTSSP; Pierce Co., USA) was added to 10 ml of part of the liposome solution obtained in the present Example: the mixture was agitated at room temperature for 2 hours and additionally overnight in a refrigerator; the solution was ultrafiltered by using a XM300 membrane and carbonate buffer solution (pH: 8.5) (molecular cutoff rate: 300,000) for removal of DTSSP, to give 10 ml of a liposome carrying HSA bound to DTSSP. Then, 3.26 ml of 4.6 mg/ml E-selectin antibody (AF575) (antibody: 15 mg) prepared in the present Example was added to 10 ml of the liposome solution; the mixture was agitated at 25° C for 2 hours for the reaction; tris(hydroxymethyl)aminomethane/carbonate buffer solution (pH: 8.5) was added thereto and the mixture was left agitated in a refrigerator overnight, allowing the E-selectin antibody (AF575) to bind to the DTSSP on the human serum albumin bound to the liposomal membrane surface. The mixture was ultrafiltered by using a XM300 membrane and HEPES buffer solution (pH: 7.2) (molecular cutoff rate: 300,000) for removal of the free E-selectin antibody (AF575) and tris(hydroxymethyl)aminomethane, to give 10 ml of a liposome to which both the E-selectin antibody (AF575) and human serum albumin were bound.

### (Hydrophilization of human serum albumin (HSA) bound to the liposomal membrane surface)

The liposome carrying the E-selectin antibody (AF575) prepared in the present Example was hydrophilized in a manner similar to Example 3.

### (Confirmation of encapsulation of cis-diamine dichloroplatinum (II) in liposome)

The solution containing the liposome carrying the E-selectin antibody (AF575) prepared in the present Example turned from colorless to pale yellow in color, indicating that the cis-diamine dinitratoplatinum (II) encapsulated in the liposome was converted to cis-diamine dichloroplatinum (II). The change can be detected by a method similar to that in Example 1 by absorption spectroscopy, IR method, or ¹⁹⁵Pt-NMR method.

### (Quantitative analysis of liposome)

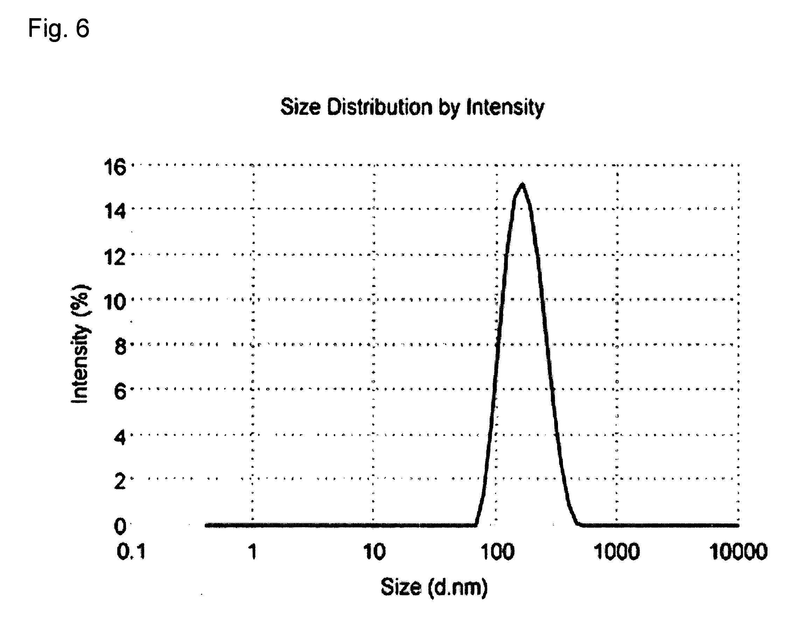

The cis-diamine dinitratoplatinum (II)-containing liposome to which the E-selectin antibody (AF575) is bound prepared in the present Example was analyzed quantitatively (protein quantitative determination, lipid quantitative determination, particle diameter distribution and particle diameter) in a manner similar to Example 1 (Fig. 6).

### Example 6A: Studies on effectiveness in treatment of cancer and tumor

An object of the present Example is to confirm that the cis-diamine dichloroplatinum (II)-containing liposomes prepared in Example 1 (liposome without sugar chain). Example 3 (sugar chain-modified liposome) and Example 5 (antibody-modified liposome) are effective in the treatment of cancers and tumors.

### (Evaluation of the anti-cancer activity of cis-diamine dichloroplatinum (II)-containing liposome)

### (1. Accumulation at cancerous site: evaluation of the cis-diamine dichloroplatinum (II) amount accumulated at cancerous site)

### Example 3

The cis-diamine dichloroplatinum (II)-containing liposome prepared from the (sialyl Lewis X-modified liposome) was used.

1×10⁶ Ehrlich ascites cancer cells (EAT cells) (ATCC Number: CCl⁻77TME (Ehrlich-Letter ascites)) were transplanted subcutaneously into the right femoral region of a 6-week old mouse (Balb/c, female) (n=1) and the following test was performed after 10 days. A cis-diamine dichloroplatinum (II)-containing sialyl Lewis X-modified liposome and a control of pure cis-diamine dichloroplatinum (II) were administered respectively into the caudal vein of a mouse in an amount of 150 pl, i.e. 1.7 mg/kg body weight (226 µg/ml). The resulting tumor was removed, 48 and 96 hours after administration.

0.1 g of the removed tumor tissue (0.2 to 1.0 g) was added to 1 ml of conc. nitric acid, and the mixture was agitated for 5 minutes. After static storage for 1 hour, the mixture was heated at 60° C in a water bath and then cooled to room temperature. 300 µl of distilled water was added to 100 µl of the nitric acid-treated solution. The content of platinum therein was determined by flameless atomic absorption spectroscopy (FAAS: AA-6700 Atomic absorption flame emission spectrophotometer SIMAZU), and the platinum contents in 1 g of tumor were compared between the sample and the control. (wavelength: 265.9 nm, slit width: 0.5, lamp current: 14 mA, 120°C for 30 seconds, 250°C for 10 seconds, 700°C for 5 seconds and 2600°C for 3 second: The calibration curve was prepared by diluting 1 mg/ml platinum standard solution (NAKΔRAI) with distilled water to solutions at concentrations of 12.5 ng/ml, 25 ng/ml, 50 ng/ml, 100 ng/ml and 200 ng/ml). Results are summarized in the following Table.

**[Table 1]**

| | Platinum concentration (ng/g tissue) | |
|---|---|---|
| Period after administration (hours) | Cisplatin alone | SLX-modified |
| 48 | 361 | 579 |
| 96 | 49 | 127 |

Platinum concentration: content of platinum in 1 g of tissue (ng)

Period after administration: time when platinum concentration in tissue was determined after administration of each liposome
Cisplatin alone: only cis-diamine dichloroplatinum (II) was used
SLX-modified: sialyl Lewis X-modified liposome encapsulating cis-diamine dichloroplatinum (II)

### (Discussion)

At the point of 48 hours after liposome administration, the liposome accumulated at the cancerous site more distinctively in the mouse administered with the sialyl Lewis X-modified liposome than in the mouse administered only with cis-diamine dichloroplatinum (II). In addition, administration of cis-diamine dichloroplatinum (II) alone at the point of 96 hours after administration leads to a distinctive decrease in the accumulation at the cancerous site. It was shown that the cis-diamine dichloroplatinum (II) was held in the tumor site in an amount 2.5 times or greater when sialyl Lewis X-modified liposome was used than when only cis-diamine dichloroplatinum (II) was administered (Table 1).

### (2. Confirmation of cell proliferation-inhibiting activity)

In the present Example, the cis-diamine dichloroplatinum

### (II)-containing liposomes prepared in Example 1 (liposome without sugar chain), Example 3 (sugar chain modification liposome) and Example 5 (antibody-modified liposome) were used.

Ehrlich ascites cancer cells (EAT cells) (ATCC Number: CCl⁻77TME (Ehrlich-Letter ascites)) were inoculated at 1×10³ cell/100 µl/well (Falcon3072), and a sample was added after culture for 24 hours. The samples were prepared by diluting the cis-diamine dichloroplatinum (II)-containing unmodified liposome, the cis-diamine dichloroplatinum (II)-containing sialyl Lewis X liposome, and the cis-diamine dichloroplatinum (II)-containing anti-E-selectin antibody-modified liposome, with DMEM buffer solution (Dulbeccos's Ham Modified Eagle liquid medium, low glucose SIGMA D6046)/10% FBS (fetal calf serum SIGMA F0926)/ penicillin 100 unit/ml-streptomycin 100 µg/ml (antibiotic antimycotic solution, stabilized SIGMA A5955)) to a cis-diamine dichloroplatinum (II) amount of 10 µg/ml. A solution of cis-diamine dichloroplatinum (II) alone dissolved in DMEM/10% FBS at a concentration of 10 µg/ml (cisplatin alone) was used as the control. Subsequently, the sample was added to the cultured cell to final concentrations of 8.3 µM and 16.6 µM, and the solutions were cultured at 37° C in a 5% CO₂ incubator for 48 hours. The cell count, 48 hours after addition was determined by the MTT assay of using the formazan amount as an indicator.

In addition, a solution containing only added DMEM/10% FBS was analyzed by the MTT assay as a control containing no sample.

A cell count-measuring reagent WST-8 (Kishida Chemical) was added to each well in an amount of 20 µl, and the absorbance thereof at 450 nm was determined after 2 hours. The absorbance was determined by using GE Healthcare Ultrospec-6300. The test was performed by using four groups of samples (n=3). Results are shown below.

(Table 2. Inhibition of cell proliferation by each sample)

**[Table 2]**

| Sample | Cisplatin alone | | Unmodified | | SLX-modified | | Antibody-modified | |
|---|---|---|---|---|---|---|---|---|
| (µM) | Average | SD | Average | SD | Average | SD | Average | SD |
| No sample | 0.480 | | 0.480 | | 0.480 | | 0.480 | |
| 8.30 | 0.045 | 0.005 | 0.070 | 0.023 | 0.063 | 0.012 | 0.073 | 0.007 |
| 16.60 | 0.027 | 0.003 | 0.043 | 0.007 | 0.032 | 0.008 | 0.023 | 0.007 |

Sample: final concentration (µM) of added sample No sample: average absorbance (450 nm) of the group to which no sample (cis-diamine dichloroplatinum (II)) was added Cisplatin alone: only cis-diamine dichloroplatinum (II) was used.
Unmodified: cis-diamine dichloroplatinum (II)-containing, unmodified liposome
SLX-modified: cis-diamine dichloroplatinum (II)-containing, sialyl Lewis X-modified liposome
Antibody-modified: cis-diamine dichloroplatinum (II)-containing, anti-E-selectin antibody-modified liposome Average: average absorbance (450 nm) of samples after administration.
SD: standard deviation

### (Discussion)

The absorbance (450 nm) in the group of no sample addition was 0.480 (Table 2, No sample).

The results showed that, in the groups respectively administered with unmodified liposome, SLX-modified liposome and antibody-modified liposome, the therapeutic effect at a sample final concentration of 8.3 µM or 16.6 µM was almost as high as that by cis-diamine dichloroplatinum (II) alone. The results showed that cis-diamine dichloroplatinum (II) did not lose its anti-cancer activity when encapsulated in the liposome. It was shown that cisplatin interacted with nucleic acids as it was released from the liposome in the cell.

The present Example confirmed that the cis-diamine dichloroplatinum (II) encapsulated in the liposome showed proliferation-inhibiting activity to the cell, independently of the unmodified liposome, SLX-modified liposome or antibody-modified liposome.

### (3. Confirmation of tumor proliferation-inhibiting activity)

1×10⁶ Ehrlich ascites cancer cells (EAT cells) (ATCC Number: CCl⁻77TME (Ehrlich-Letter ascites)) were transplanted subcutaneously into right femoral region of 6-week old mice (Balb/c, female) (n=3), and the following tests were performed after 10 days. The cis-diamine dichloroplatinum (II)-containing sialyl Lewis X-modified liposome and the anti-E-selectin antibody-modified liposome were administered into the caudal vein of a mouse respectively in an amount of 1.5 mg/kg (200 µg/ml) on the 5th, 12th, and 19th days after transplantation. As a control, cis-diamine dichloroplatinum (II) was dissolved in physiological saline to a concentration of 200 µg/ml. The dissolved solution was administered similarly.

The size, length (A mm) and diameter (B mm), of the tumor was determined by using a digital vernier caliper (Mitsutoyo CD-S15C) on the 12th, 19th, and 29th days after tumor transplantation. The volume of the tumor (mm³) was calculated by the following Formula: (A×B²)×0.4.

In the present animal experiment, encapsulation liposomes that were bound to a known cancer-specific sugar chain (SLX) or antibody were used, and improvement in anti-cancer activity thereof over conventional cisplatin was studied. Results are shown below.

**[Table 3]**

| (Table 3. Tumor-inhibiting activity of each sample) | | | | |
|---|---|---|---|---|
| | Measured time | Day 12 | Day 19 | Day 29 |
| Saline | Average | 113 | 120 | 887 |
| | SD | 61 | 42 | 259 |
| Cisplatin alone | Average | 63 | 63 | 501 |
| | SD | 27 | 8 | 109 |
| SLX-modified | Average | 100 | 94 | 373 |
| | SD | 46 | 52 | 104 |
| Antibody-modified | Average | 57 | 59 | 68 |
| | SD | 19 | 33 | 35 |

Tumor volume (mm³)
Measured time: time of tumor size measured after tumor transplantation
Physiological saline: only physiological saline was used.
Cisplatin alone: only cis-diamine dichloroplatinum (II) was used.
SLX-modified: cis-diamine dichloroplatinum (II)-containing, sialyl Lewis X-modified liposome
Antibody-modified: cis-diamine dichloroplatinum (II)-containing, anti-E-selectin antibody-modified liposome
Average: average tumor volume (mm³) after administration of each sample
SD: standard deviation

### (Results and discussion)

The results showed that, the size of the tumor on the 29th day after tumor cell transplantation was distinctively smaller when cis-diamine dichloroplatinum (II)-containing, sialyl Lewis X-modified liposome or anti-E-selectin antibody-modified liposome was administered than when cis-diamine dichloroplatinum (II) alone or the unmodified liposome was administered.

The accumulation of the action of cis-diamine dichloroplatinum (II) in the tumor seems to be the results of an increase in compatibility of the liposome to the tumor tissue by modification of the cis-diamine dichloroplatinum (II)-containing liposome with sialyl Lewis X or anti-E-selectin antibody.

Shown in the present Example are the advantageous effects of modification with sialyl Lewis X or anti-E-selectin antibody. The results showed that the cis-diamine dichloroplatinum (II)-containing, sialyl Lewis X- or anti-E-selectin antibody-modified liposome inhibited tumor growth in an amount distinctively greater than that by administration with cis-diamine dichloroplatinum (II) alone. Thus, the sialyl Lewis X or anti-E-selectin antibody-modified liposome was effective in treating tumors.

It was also shown that the method allowed significant increase in cisplatin content in the lipid or phospholipid, compared to conventional encapsulation methods, and consequently, distinctive increase in therapeutic effect.

The therapeutic effectiveness is obviously higher than that obtained, for example, with conventional anti-cancer drug-containing liposome (Cancer Chemother. Pharmacol. (1999) 43: 1-7 Comparative pharmacokinetics, tissue distribution, and therapeutic effectiveness of cisplatin encapsulated in long-circulating, pegylated liposome (SPI) in tumor-bearing mice). The SPI077 liposome described in the literature is a liposome clinically used in the U.S. The data on SPI077 liposome are shown in Fig. 1A of the literature, but the dosage thereof is far larger than that in the present Example. Thus, the present invention provides a similar or higher therapeutic effect even with reduced dosage. Because even a probe-unlabelled liposome (SPI077) shows an anti-cancer activity similar to that herein in the case of some cancer species, addition of cisplatin to a liposome carrying a sugar chain or an antibody as the recognition probe would be effective additionally.

### Example 6B: Studies on the effectiveness in treatment of other cancers and tumors

Effectiveness of the cis-diamine dichloroplatinum (II)-containing liposomes prepared in Example 1 (Unmodified liposome), Example 3 (sugar chain-modified liposome) and Example 5 (antibody-modified liposome) in the treatment of testicular tumor, bladder cancer, renal pelvic/ureteral tumor, prostate cancer, ovarian cancer, head and neck cancer, non-small cell lung cancer, esophagus cancer, uterocervical cancer, neuroblastoma, stomach cancer, small cell lung cancer, osterosarcoma, and blastocyte tumor is studied.

Cells (approximately 2×10⁷ pieces) of testicular tumor, bladder cancer, renal pelvic/ureteral tumor, prostate cancer, ovarian cancer, head and neck cancer, non-small cell lung cancer, esophagus cancer, uterocervical cancer, neuroblastoma, stomach cancer, small cell lung cancer, osterosarcoma, or blastocyte tumor were transplanted subcutaneously into the femoral region of a female Balb/c mouse, and a cancer-bearing mouse bearing a cancerous tissue grown to 0.3 to 0.6 g was used in the present experiment.

Each cis-diamine dichloroplatinum (II)-containing liposome (200 µl) was administered orally or caudally to each cancer-bearing mouse.

It was found that the volume of the cancer or tumor decreased or the tumor or cancer disappeared in all cancer-bearing mice administered with the cis-diamine dichloroplatinum (II)-containing liposome.

Thus, the cis-diamine dichloroplatinum (II)-containing liposomes prepared by the present method were shown to be effective in treatment of cancers and tumors.

### Example 7: Test on other water-soluble ammine platinum complexes

### (Preparation of cisplatin sulfate-containing liposome)

### (Preparation of cisplatin sulfate)

4.15 g (10.0 mmol) of platinum potassium tetrachloride (K₂ PtCl₄) and 6.64 g (40 mmol) of potassium iodide were completely dissolved in 50 ml of sufficiently oxygen-free distilled water; and 1.35 ml (22 mmol) of 28% aqueous ammonia solution was added to the mixture while agitating under nitrogen atmosphere, allowing precipitation. The precipitate was washed with water and ethanol respectively twice and dried at room temperature under vacuum (to 20 mm Hg), to give an ammine-platinum intermediate cis-[Pt(NH₃)₂I₂]. 1.45 g (3.0 mmol) of the ammine-platinum intermediate cis-[Pt(NH₃)₂I₂] was suspended in 300 ml of distilled water; 0.933 g (3.0 mmol) of silver sulfate was added thereto, allowing reaction under agitation for 4 hours; the silver chloride generated was removed by filtration, to give a colorless cisplatin sulfate cis-[Pt(NH₃)₂SO₄].

### (Preparation of cisplatin sulfate-containing liposome)

DPPC, cholesterol, ganglioside, DCP, and DPPE were mixed at a molar ratio of 35:40:5:15:5 to a total lipid content of 45.6 mg; 46.9 mg of sodium cholate was added; and the mixture was dissolved in 3 ml of methanol-chloroform solution (1:1) solution. The mixture was agitated at 37° C for 1 hour; methanol and chloroform were evaporated by a rotary evaporator; and the residue was dried under vacuum. The lipid membrane obtained was suspended in 3 ml of NaCl-free N-tris(hydroxymethyl)-3-aminomethane (TAPS) buffer solution (pH: 8.4), and the mixture was agitated at 37° C for 1 hour. Then, the solution was ultrasonicated after nitrogen substitution, to give a transparent micellar suspension. Then, 50 mg of cisplatin sulfate was weighed and dissolved in 7 ml of NaCl-free TAPS buffer solution (pH: 8.4); the solution was mixed with the micellar suspension; the mixture was ultrafiltered by using a PM10 (Amicon Co. , USA) and NaCl-free TAPS buffer solution (pH: 8.4) (molecular cutoff rate: 10,000), to give 10 ml of a uniform liposome.

### (Confirmation of the liposome containing cisplatin sulfate)

Encapsulation of cisplatin sulfate in the liposome prepared in the present Example was examined by a method similar to that in Example 1, by means of flameless atomic absorption spectroscopy (FAAS).

The results showed that cisplatin sulfate was contained in the liposome.

### (Conversion of cisplatin sulfate to cis-diamine dichloroplatinum (II)) (1. Conversion of cisplatin sulfate to cis-diamine dichloroplatinum (II) in buffer solution)

The liposome known to contain cisplatin sulfate was processed in a N-tris(hydroxymethyl)-3-aminopropane sulfonate buffer solution (pH: 8.4), a carbonate buffer solution (pH: 8.5), PBS (pH: 8.0), or a HEPES buffer solution (pH: 7.2) at 25°C for 96 hours. Each of these buffer solutions contains NaCl at a final concentration of 150 mM.

It is found that the colorless solution turns pale yellow, indicating that cisplatin sulfate is converted to cis-diamine dichloroplatinum (II). In addition, the change can be detected by a method similar to that in Example 1 by absorption spectroscopy, IR method, or ¹⁹⁵Pt-NMR method.

### (2. Preparation of sugar chain-bound cisplatin sulfate-containing liposome)

A sugar chain-bound cis-diamine dichloroplatinum (II)-containing liposome is prepared from the liposome known to contain cisplatin sulfate in a similar manner to Example 2. Conversion of cisplatin sulfate to cis-diamine dichloroplatinum (II) in the liposome obtained can be detected by absorption spectroscopy, IR method, or ¹⁹⁵Pt-NMR method by a method similar to that in Example 1.

### (3. Preparation of antibody-bound cis-diamine dichloroplatinum (II)-containing liposome)

In a manner similar to Example 6, an antibody-bound cis-diamine dichloroplatinum (II)-containing liposome is prepared from each solution containing the liposome known to contain cisplatin sulfate. Conversion of cisplatin sulfate to cis-diamine dichloroplatinum (II) in the liposome obtained can be detected by absorption spectroscopy, IR method, or ¹⁹⁵Pt-NMR method by a method similar to that in Example 1.

### (Quantitative analysis of liposome)

Each of the liposomes prepared in the section of "conversion of cisplatin sulfate to cis-diamine dichloroplatinum (II)" is analyzed quantitatively (protein quantitative determination, lipid quantitative determination, particle diameter distribution and particle diameter) in a manner similar to Example 1.

### (Evaluation of the effectiveness in treatment of cancers and tumors)

The effectiveness in tumor/cancer treatment of each of the liposomes prepared in the section of "conversion of cisplatin sulfate to cis-diamine dichloroplatinum (II)" is evaluated in a manner similar to Example 5.

The results show that the size of cancer or tumor declines or the tumor or cancer disappears in all cancer-bearing mice administered with the poor water-soluble medicine-containing liposome.

Thus, the cis-diamine dichloroplatinum (II)-containing liposomes prepared by the present method are obviously effective in treatment of cancers and tumors.

### Example 8: Tests with other platinum complexes

A liposome encapsulating cis-diammine-1,1-cyclobutane-dicarboxylatoplatinum (II), cis-diammine (glucolato)platinum (II), (1R,2R-diammine cyclohexane)oxalatoplatinum (II), or cis-diammine dibromoplatinum (II), replacing the cis-diammine dichloroplatinum (II), is prepared by a method similar to that in Example 1.

### (Confirmation of encapsulation of drug in liposome)

Encapsulation of the medicine in the water-soluble form in liposome is studied in a manner similar to Example 1 by flameless atomic absorption spectroscopy (FAAS).

### (Conversion from water-soluble medicine to poor water-soluble medicine)

### (1. Conversion of water-soluble medicine to poor water-soluble medicine by buffer solution)

In a manner similar to Example 1, a solution of the liposome known to contain a water-soluble pharmaceutical is processed with a solution containing an ion forming a poorly water-soluble salt. As a result, the water-soluble medicine is found to be converted to a poorly water-soluble medicine. In addition, the change can be detected by a method similar to that in Example 1 by absorption spectroscopy, IR method, or ¹⁹⁵Pt-NMR method.

### (2. Preparation of liposome containing a sugar chain-bound poor water-soluble medicine)

In a manner similar to Example 3, a sugar chain-bound poor water-soluble medicine-containing liposome is prepared from each solution of the liposome known to contain a water-soluble pharmaceutical. Conversion of the encapsulated water-soluble medicine to a poor water-soluble medicine in the liposome can be detected by a method similar to that in Example 1 by absorption spectroscopy, IR method, or ¹⁹⁵Pt-NMR method.

### (3. Preparation of liposome containing antibody-bound poor water-soluble medicine)

In a manner similar to Example 6, a sugar chain-bound poor water-soluble medicine-containing liposome is prepared from each solution of the liposome known to contain a water-soluble pharmaceutical. Conversion of the encapsulated water-soluble medicine to a poor water-soluble medicine in liposome can be detected by a method similar to that in Example 1 by absorption spectroscopy, IR method, or ¹⁹⁵Pt-NMR method.

### (Quantitative analysis of liposome)

Each of the liposomes prepared in the section of the (conversion from water-soluble medicine to poor water-soluble medicine) is analyzed quantitatively (protein quantitative determination, lipid quantitative determination, particle diameter distribution and particle diameter) in a manner similar to Example 1.

### (Evaluation of the effectiveness in treatment of cancers and tumors)

The effectiveness in tumor/cancer treatment of each of the liposomes prepared in the section of (conversion of water-soluble medicine to poor water-soluble medicine) is evaluated in a manner similar to Example 5.

The results show that the size of cancer or tumor declines or the tumor or cancer disappears in all cancer-bearing mice administered with the poor water-soluble medicine-containing liposome.

Thus, the poorly water-soluble medicine-containing liposomes prepared by the present method are obviously effective in treatment of cancers and tumors.

### Example 9. Encapsulation of cis-diammine dinitratoplatinum (II) in vacant freeze-dried liposome

In the present Example, for confirmation of the applicability of the encapsulation method according to the present invention to any liposome, cis-diammine dinitratoplatinum (II) was encapsulated by using a liposome prepared by the vacant freeze-dried liposome method (H. Kikuchi, N. Suzuki et al., Biopharm. Drug Dispos., 17, 589-605 (1999)), which is different from the improved cholate method (N. Yamazaki, M. Kodama, H-J. Gabius, Methods Enzymol., 242, 56-65 (1994); N. Yamazaki, J. Membr. Sci., 41, 249-267 (1989); and M. Hirai, H. Minematsu, N. Kondo, K. Oie, K. Igarashi, N. Yamazaki, BBRC., 353, 553-558 (2007)).

### (Preparation of cis-diammine dinitratoplatinum (II) (CDDP-3))

A method similar to that in Example 1 was used. Cis-diammine dinitratoplatinum (II) was prepared according to the method of Dahara (S. G. Dhara, Indian J. Chem., 7, 335 (1970)).

4.15 g (10 mmol) of potassium chloroplatinate was dissolved in distilled water; 6.64 g (40 mmol) of potassium iodide was added thereto, while the solution was protected from light and cooled on ice under nitrogen stream; and the mixture was agitated for 5 minutes. 1.35 mL (22 mmol) of 28% aqueous ammonia solution was added dropwise to the reaction solution, and the mixture was agitated for 3 hours. The yellow crystals generated were collected by filtration, washed with distilled water and ethanol sequentially, and dried at 40° C under reduced pressure for 10 hours, to give 4.49 g of an ammine-platinum intermediate cis-[Pt(NH₃)₂I₂] (cis-diamminediiodeplatinum (II) (CDDP-2)). 2.41 g (5 mmol) of cis-[Pt(NH₃)₂I₂] was suspended in distilled water; 1.68 g (9.9 mmol) of silver nitrate was added thereto; and the mixture was agitated for 24 hours while protected from light. The silver chloride generated was filtered off, and the filtrate was concentrated in an evaporator, to give white crystals. The white crystals were washed with cold distilled water and ethanol sequentially and dried at 40° C under reduced pressure for 10 hours, to give 1.01 g of cis-diammine dinitratoplatinum (II).

### (Preparation of two kinds of liposomes and encapsulation of cis-diamine dichloroplatinum (II) therein)

### (1. Improved cholate salt method (liposome I))

The liposome I was prepared by a method similar to the improved cholate salt method in Example 1. DPPC, cholesterol, ganglioside, DCP and DPPE were mixed at a molar ratio of 35:40:5:15:5 to a total lipid content of 45.6 mg; and 46.9 mg of sodium cholate was added; and the mixture was dissolved in 3 ml of methanol-chloroform (1:1) solution. After agitation at 37°C for 1 hour, methanol and chloroform were evaporated by a rotary evaporator, the residue was vacuum-dried and suspended in 3 mL of sodium chloride-free TAPS (N-tris(hydroxymethyl)-3-aminopropane sulfonate) buffer solution (pH: 8.4). After agitation at 37° C for 1 hour, the suspension was ultrasonicated to give a transparent micellar suspension.

Then, cis-diammine dinitratoplatinum (II) (108.2 mg) was dissolved in 7 ml of sodium chloride-free TAPS buffer solution (pH: 8.4) completely, and the solution was adjusted to pH 8.4 with 1 M sodium hydroxide; and the solution was mixed with the micellar suspension. Ultrafiltration (molecular cutoff rate: 10,000) of the suspension by using PM10 (AMICON) and sodium chloride-free TAPS buffer solution (pH: 8.4) gave 10 mL of a liposome. The liposome external solution was substituted with 150 mM sodium chloride-containing TAPS buffer solution (pH: 8.4) by ultrafiltration by using PM10 (Amicon), thus allowing conversion of cis-diammine dinitratoplatinum (II) encapsulated in the liposome to cis-diamine dichloroplatinum (II).

### (2. Freeze drying method (liposome II)

The vacant freeze-dried liposome used was a COASTOME EL series product (EL-01-PA, NOF Corporation). The COASTOME EL-01-PA (negatively charged liposome) contained dicetyl phosphatidylethanolamine-polyglycerin 8G, dipalmitoyl phosphatidylcholine, cholesterol and dipalmitoyl phosphatidylglycerol at a ratio of 4.2:11.4:15.2:11.4 (µMol/vial). Cis-diammine dinitratoplatinum (II) (40 mg) was dissolved in 2 mL of sodium chloride-free TAPS buffer solution (pH: 8.4); the solution was adjusted to pH 8.4 with 1 M sodium hydroxide; and 2 mL of the solution was added dropwise into the vial containing the freeze-dried liposome. The vial was shaken by rotation five times; subsequent ultrafiltration by using PM10 (AMICON) and sodium chloride-free TAPS buffer solution (pH: 8.4) (molecular cutoff rate: 10,000) gave 2 mL of a liposome. Sodium chloride was added to the liposome solution to a concentration of 150 mM; the mixture was agitated at 4°C for 96 hours, allowing conversion of the encapsulated cis-diammine dinitratoplatinum (II) to cis-diamine dichloroplatinum (II).

### Comparative Example 2. Liposome containing cis-diamine dichloroplatinum (II) encapsulated without use of cis-diammine dinitratoplatinum (II)

In a Comparative Example, a liposome containing cis-diamine dichloroplatinum (II) directly encapsulated, not produced by encapsulation of cis-diammine dinitratoplatinum (II) in liposome and subsequent conversion to cis-diamine dichloroplatinum (II), was prepared.

### (1. Improved cholate salt method)

A liposome containing cis-diamine dichloroplatinum (II) directly encapsulated was prepared by dissolving 28 mg of cis-diamine dichloroplatinum (II) (SIGMA) completely in 7 ml of 150 mM sodium chloride-containing TAPS buffer solution (pH: 8.4), adjusting the solution to pH 8.4 with 1 M sodium hydroxide, and mixing the solution with the micellar suspension. A method similar to the encapsulation of cis-diammine dinitratoplatinum (II) in the present Example was used, except that cis-diammine dinitratoplatinum (II) was replaced with cis-diamine dichloroplatinum (II) and the buffer solution contained sodium chloride.

### (2. Freeze drying method)

The vacant freeze-dried liposome used was a COASTOME EL series product (EL-01-PA, NOF Corporation). A liposome containing cis-diamine dichloroplatinum (II) directly encapsulated was prepared in the following manner: COASTOME EL-01-PA was warmed to room temperature. Four mg of cisplatin was completely dissolved in 2 ml of 150 mM sodium chloride-containing TAPS buffer solution (pH: 8.4); the solution was added to COASTOMEEL-01-PA; the mixture was shaken by rotation thrice. The solution was ultrafiltered by using a membrane having a molecular cutoff rate of 10,000 for removal of free cisplatin.

### (3. Preparation of liposome containing sugar chain-modified cis-diamine dichloroplatinum (II))

A sugar chain-modified liposome I was prepared in a manner similar to Example 3 by using part of the cis-diamine dichloroplatinum (II)-containing liposome I prepared by the improvement cholic acid of the present Example.

### (Hydrophilization of lipid membrane surface)

Ten ml of the solution of the liposome I containing cis-diamine dinitratoplatinum (II) was ultrafiltered by using a XM300 membrane (Amicon Co. , USA) and carbonate buffer solution (pH: 8.5) (molecular cutoff rate: 300,000), and the solution was adjusted to pH 8.5. Then, 10 mg of a crosslinking agent bis(sulfosuccinimidyl) suberate (BS3; Pierce Co., USA) was added thereto, and the mixture was agitated at room temperature for 2 hours. Then, the solution was agitated additionally overnight in a refrigerator, before completion of the chemical binding reaction between lipid dipalmitoyl phosphatidylethanolamine and S3 on the liposomal membrane. The liposome solution was ultrafiltered by using a XM300 membrane and carbonate buffer solution (pH: 8.5) (molecular cutoff rate: 300,000). Then, a solution of 40 mg of tris(hydroxymethyl)aminomethane in 1 ml of carbonate buffer solution (pH: 8.5) was added to 10 ml of the liposome solution. The solution was then agitated at room temperature for 2 hours and additionally overnight in a refrigerator; the solution was ultrafiltered at a molecular cutoff rate of 300,000 for removal of free tris(hydroxymethyl)aminomethane; and the carbonate buffer solution was replaced with N-tris(hydroxymethyl)-3-aminopropane sulfonate buffer solution (pH: 8.4), allowing completion of the chemical binding reaction between BS3 bound to the lipid on the liposomal membrane and tris(hydroxymethyl)aminomethane. In this way, the liposomal membrane was hydrated and hydrophilized by coordination of tris(hydroxymethyl)aminomethane hydroxyl groups to the lipid dipalmitoyl phosphatidylethanolamine on the membrane.

### (Binding of human serum albumin (HSA) onto liposomal membrane surface)

43 mg of sodium metaperiodate dissolved in 1 ml of N-tris(hydroxymethyl)-3-aminopropane sulfonate buffer solution (pH: 8.4) was added to the ganglioside present on the membrane surface of 10 ml of liposome I obtained in the present Example; the mixture was agitated in a refrigerator overnight for periodate oxidation. The mixture was ultrafiltered by using a XM300 membrane and PBS buffer solution (pH: 8.0) (molecular cutoff rate: 300,000) for removal of free sodium periodate; and the N-tris(hydroxymethyl)-3-aminopropane sulfonate buffer solution was replaced with PBS buffer solution (pH: 8.0), to give 10 ml of an oxidized liposome. 20 mg of human serum albumin (HSA)/PBS buffer solution (pH: 8.0) was added to the liposome solution; the mixture was allowed to react at room temperature for 2 hours; 100 µl of 2 M NaBH₃CN/PBS buffer solution (pH: 8.0) was added thereto; and the mixture was agitated at room temperature for 2 hours and additionally overnight in a refrigerator, allowing binding of HSA by coupling reaction between the ganglioside on the liposome and HSA. The mixture was then ultrafiltered (molecular cutoff rate: 300,000) for removal of free sodium cyanoborate and human serum albumin; the buffer solution of the solution was replaced with carbonate buffer solution (pH: 8.5), to give 10 ml of a HSA-bound liposome solution.

### (Preparation of sugar chain)

The sugar chain used was sialyl Lewis X.

Each sugar chain was weighed and pretreated for use in the tests below.

### (Binding of sugar chain to human serum albumin (HSA) bound to the liposomal membrane surface)

Two mg of the sugar chain was dissolved in purified water; the solution was added to 0.5 ml of aqueous solution containing 0.25 g of NH₄ HCO₃; and the mixture was agitated at 37° C for 3 days and filtered through a 0.45-µm filter; amination reaction of the reductive terminal of the sugar chain gave a 4 mg/ml of the glycosylamine compound of each sugar chain (aminated sugar chain solution). Subsequently, 10 mg of a crosslinking agent 3,3'-dithiobis(sulfosuccinimidyl propionate) (DTSSP; Pierce Co., USA) was added to part of the solution of the liposome I obtained in the present Example (10 ml); the mixture was agitated at room temperature for 2 hours and additionally overnight in a refrigerator; the mixture was ultrafiltered by using a XM300 membrane and carbonate buffer solution (pH: 8.5) (molecular cutoff rate: 300,000) for removal of free DTSSP, to give 10 ml of a liposome carrying HSA thereon bound to DTSSP. Then, 37.5 µl of the glycosylamine compound (aminated sugar chain solution) was added to the liposome solution; the mixture was allowed to react at room temperature for 2 hours; tris(hydroxymethyl)aminomethane/carbonate buffer solution (pH: 8.5) was added; the mixture was then agitated in a refrigerator overnight, allowing binding of the glycosylated amine compound to the DTSSP on the human serum albumin bound to the liposomal membrane surface. The mixture was ultrafiltered by using a XM300 membrane and HEPES buffer solution (pH: 7.2) (molecular cutoff rate: 300,000) for removal of free sugar chain and tris(hydroxymethyl)aminomethane, to give 10 ml of each liposome bound to the sugar chain and human serum albumin.

### (Hydrophilization of human serum albumin (HSA) bound to the liposomal membrane surface)

The HSA protein surface on the liposome I bound to the sugar chain prepared in the present Example was hydrophilized. 26.4 mg of tris(hydroxymethyl)aminomethane was added to 10 ml of the liposome; the mixture was agitated at room temperature for 2 hours and additionally overnight in a refrigerator; and the mixture was ultrafiltered by using a XM300 membrane and HEPES buffer solution (pH: 7.2) (molecular cutoff rate: 300,000) for removal of unreacted materials. The mixture was filtered through a 0.45-µm filter, to give 10 mg of the final product, i.e., each liposome complex bound to the hydrophilized sugar chain.

### (Measurement of particle diameter and zeta potential)

Each of the liposome solutions (without sugar chain) prepared in the Examples and Comparative Examples was diluted 50 times with purified water, and the particle diameter was determined by dynamic scattering method by using Zetasizer Nano Nan-ZS (Malvern).

The cis-diamine dichloroplatinum (II)-containing liposome I obtained after encapsulation of cis-diammine dinitratoplatinum (II) and conversion thereof to cis-diamine dichloroplatinum (II) showed uniform particle size distribution and had an average particle diameter of 159 nm (Table 4). The cis-diamine dichloroplatinum (II)-containing liposome II obtained after encapsulation of cis-diammine dinitratoplatinum (II) and conversion thereof to cis-diamine dichloroplatinum (II) showed uniform particle size distribution and had an average particle diameter of 147 nm (Table 4).

**[Table 4]**

| (Table 4. Physical properties of cis-diamine dichloroplatinum (II)-containing liposomes) | | | | |
|---|---|---|---|---|
| Liposome | Improved cholate method | | Freeze-dried liposome method | |
| Liposome No. | - | liposome I | - | liposome II |
| Encapsulated substance | CDDP | CDDP-3 → CDDP | CDDP | CDDP-3 → CDDP |
| Addition amount (mg) | 28.0 | 108.2 | 4.0 | 40.0 |
| Liposome volume (mL) | 10.0 | 10.0 | 2.0 | 2.0 |
| Lipid content (mg/mL) | 3.55 | 3.55 | 7.6 | 7.6 |
| Average particle diameter | 158 | 159 | 147 | 147 |
| CDDP concentration (µg/ml) | 1.1 | 323.3 | 134.6 | 893 |
| CDDP/lipid (µg/mg) | 0.3 | 91.1 | 17.7 | 117.5 |

As shown in Table 4, analysis of the particle diameter and shape of the liposomes I and II revealed that the particle diameter of cis-diamine dichloroplatinum (II)-containing liposome was approximately 150 nm, independently of the kind of the liposome. It is important to adjust the particle diameter of liposome for the accumulation of the liposome in the tumor tissue or inflammatory structure. It is known that there is a gap of 100 to 200 nm between the neogenetic blood vessels at cancer sites or between the blood vessels at inflammatory sites, and thus, the particle size of the above liposome I or II would be suitable for migration thereof from the blood vessel into the desired structure (A. A. Gabizon, Cancer Res., 52, 891-896 (1992); S. K. Huang, F. J. Martin, G. Jay et al., Am. J. Pathol., 143, 10-14 (1993); S. K. Huang, E. Mayhew, S. Gilani et al., Cancer Res., 52, 6774-6781 (1992); and N. Z. Wu, D. Da, T. L. Rudoll et al., Cancer Res., 53, 3765-3770 (1993)).

### (Observation under electron microscope)

The cis-diamine dichloroplatinum (II)-containing liposome I (without sugar chain) prepared in the present Example was negatively stained by using 1% uranium acetate (J. D. Almeida, C. M. Brand, D. C. Edwards and T. D. Heath, Lancet 2 (7941), 899-901 (1975)). Specifically, a drop of the liposome solution was placed on a copper mesh and washed thoroughly with a PBS buffer solution, and then the mesh was immersed in 1% uranium acetate for several seconds. Excess water was wiped off for drying. Then, the shape and size of the particle was observed at a magnification of 80,000 times by using a transmission electron microscope H-7100S (HITACHI).

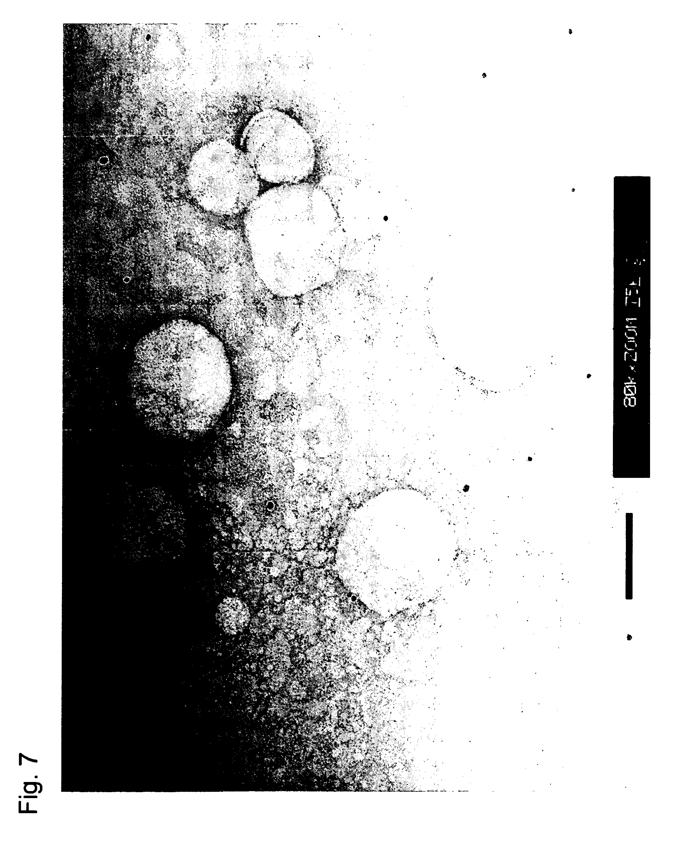

The results showed that the cis-diamine dichloroplatinum (II)-containing liposome I was a uniform spherical liposome (Fig. 7). In addition, the particle diameter of the liposome I, as observed under electron microscope, was similar to that obtained by the dynamic scattering method.

### (Quantitative determination of lipid content)

A method similar to Example 2 was used. The total cholesterol content was determined in the presence of 0.5% TitonX-100 by using Determiner TC555 kit (KYOWA), and the total lipid content was calculated from the molar ratio of the respective lipids. The lipid content, an indicator of liposome amount, was 3.5 mg/mL in the case of liposome I and 7.6 mg/mL in the case of liposome II (Table 4).

### (Quantitative determination of cis-diammine dinitratoplatinum (II))

The amount of the cis-diammine dinitratoplatinum (II) encapsulated in the liposome was determined quantitatively by flameless atomic absorption spectroscopy (FAAS) by using AA-6700 Atomic absorption flame emission spectrophotometer (SHIMAZU)). The test sample was processed at 120°C for 30 seconds, 250°C for 10 seconds, 700° C for 20 seconds, 700° C for 5 seconds, and 2600° C for 3 seconds sequentially, under the condition of a wavelength of 265.9 nm, a slit width of 0.5 and a lamp current of 14 mA. One mg/mL of platinum standard solution (NAKΔRAI) was diluted with purified water, to give solutions at concentrations of 12.5 ng/ml, 25 ng/ml, 50 ng/ml, 100 ng/ml and 200 ng/ml, and a calibration curve was drawn thereby. The liposome solution was diluted with purified water 10,000 times before use as the test sample.

The amount of the cis-diamine dichloroplatinum (II) encapsulated in the liposome was calculated by measuring the platinum amount by flameless atomic absorption spectroscopy (FAAS), showing that the cis-diamine dichloroplatinum (II) concentration in the liposome I solution prepared by encapsulating cis-diammine dinitratoplatinum (II) and converting it into cis-diamine dichloroplatinum (II) by the improved cholate method was 91.08 µg/mg-lipid (323.3 µg/mL) (left column in Table 4). On the other hand, the content of the cis-diamine dichloroplatinum (II), when encapsulated directly into the liposome, was 0.306 µg/mg-lipid (1.07 µg/mL) (left column in Table 4).

The cis-diamine dichloroplatinum (II) concentration in the liposome II solution prepared by encapsulating cis-diammine dinitratoplatinum (II) and converting it into cis-diamine dichloroplatinum (II) by the freeze drying method was 117.5 µg/mg-lipid (893 µg/mL) (right column in Table 4). On the other hand, the concentration of the cis-diamine dichloroplatinum (II), when encapsulated directly therein, was 17.7 µg/mg-lipid (134.6 µg/mL) (right column in Table 4).

The content in 1 mg of lipid, when prepared by encapsulation of cis-diammine dinitratoplatinum (II) and conversion thereof to cis-diamine dichloroplatinum (II) in the liposome I, was approximately 300 times larger than that in the liposome containing cis-diamine dichloroplatinum (II) directly encapsulated, and that of liposome II was 6.6 times larger. The results confirm in particular that the liposome I prepared by the improved cholate method of forming liposome and encapsulating the substance simultaneously gave a significantly larger content.

Cis-diammine dinitratoplatinum (II) is approximately 10 times more soluble in water than cis-diamine dichloroplatinum (II). Thus, the cis-diamine dichloroplatinum (II) content in the liposome at an amount of approximately 300 times larger in 1 mg of lipid, when the liposome is prepared by the method of encapsulating the cis-diammine dinitratoplatinum (II) in liposome and converting it to cis-diamine dichloroplatinum (II), is far higher than the content expected. In addition, the content thereof in liposome II being approximately 6 to 7 times larger indicates that the liposome I prepared by the encapsulation method, in particular the improved cholate method, is more effective.

Generally, in the case of the liposome I prepared by the improved cholate method of forming liposome and encapsulating substance simultaneously, the content of an uncharged low-molecular weight compound such as cis-diamine dichloroplatinum (II) is very low. For that reason, it was not possible make liposome I contain as much as 0.306 µg/mg-lipid of cis-diamine dichloroplatinum (II), by the method of encapsulating cis-diamine dichloroplatinum (II) into the liposome directly. However, it was possible to encapsulate as much as 91.1 µg/mg-lipid of cis-diamine dichloroplatinum (II) by using the encapsulation method according to the present invention. The large difference in cis-diamine dichloroplatinum (II) content between liposome I prepared by the improved cholate method and liposome II prepared by the vacant freeze-dried liposome method is probably due to the fact that it is almost not possible to encapsulate an uncharged low-molecular weight compound by the improved cholate method.

Liposomes I and II in the present Example have negatively charged lipid components making the liposome surface charge negative for prevention of binding with blood proteins, but positively charged components may be used, and those skilled in the art can change the component design properly. Cis-diammine dinitratoplatinum (II), which is positively charged in aqueous solution, may have a molecular shape advantageous for encapsulation in the liposome that consists of negatively charged lipid components. Most of liposomes targeting an inflammatory or cancerous site by the EPR effect (Y. Matsumura and H. Maeda, Cancer Res. , 46, 6387-6392 (1986)) are designed to have negatively charged surface for improvement in in-blood retentivity, and, similarly to many liposomes commonly used, it would be possible to raise the content of the poorly water-soluble pharmaceutical in a liposome by using the encapsulation method according to the present invention.

(Confirmation of the conversion of cis-diammine dinitratoplatinum (II) to cis-diamine dichloroplatinum (II)) Replacement of the nitrate ion of cis-diammine dinitratoplatinum (II) with the chloride ion in the 150 mM sodium chloride solution was studied by absorption spectroscopic method (Comparative Example 1 and Figs. 4A and 4B).

As shown in Figs. 4A and 4B, the absorption spectrum of cis-diammine dinitratoplatinum (II) (Fig. 4A) changes over time from immediately after addition of chloride ion until it become identical with the absorption spectrum of cis-diamine dichloroplatinum (II) shown in Fig. 4B. The results showed that 99% or more of the cis-diammine dinitratoplatinum (II) was converted to cis-diamine dichloroplatinum (II) in the presence of 150 mM sodium chloride.

Separately, conversion of the cis-diammine dinitratoplatinum (II) contained in liposomes I and II to cis-diamine dichloroplatinum (II) in the 150 mM sodium chloride solution was analyzed by ¹⁹⁵Pt-NMR method.

### (¹⁹⁵Pt-NMR method)

A method similar to Example 3 was used. The cis-diammine dinitratoplatinum (II)-encapsulating liposome used was liposome II prepared in the present Example. Conversion of cis-diammine dinitratoplatinum (II) to cis-diamine dichloroplatinum (II) in the liposome was analyzed at 25° C by placing a sample in a test tube having a diameter of 5 mm and by ¹⁹⁵Pt-NMR method using INOVA-600 (Varian).

### (Sample preparation)

Sodium hexachloroplatinate (IV) was dissolved in heavy water to a concentration of 50 mM and used as the external standard solution. Cis-diamine dichloroplatinum (II) and cis-diammine dinitratoplatinum (II) were dissolved respectively in heavy water to give samples at final concentrations of 6.6 mM and 13.7 mM. The liposome samples before and after conversion of the liposome II-encapsulated cis-diammine dinitratoplatinum (II) to cis-diamine dichloroplatinum (II) were freeze-dried, the resulting powders were resuspended in heavy water, to give solutions at a platinum concentration of 4.75 mM.

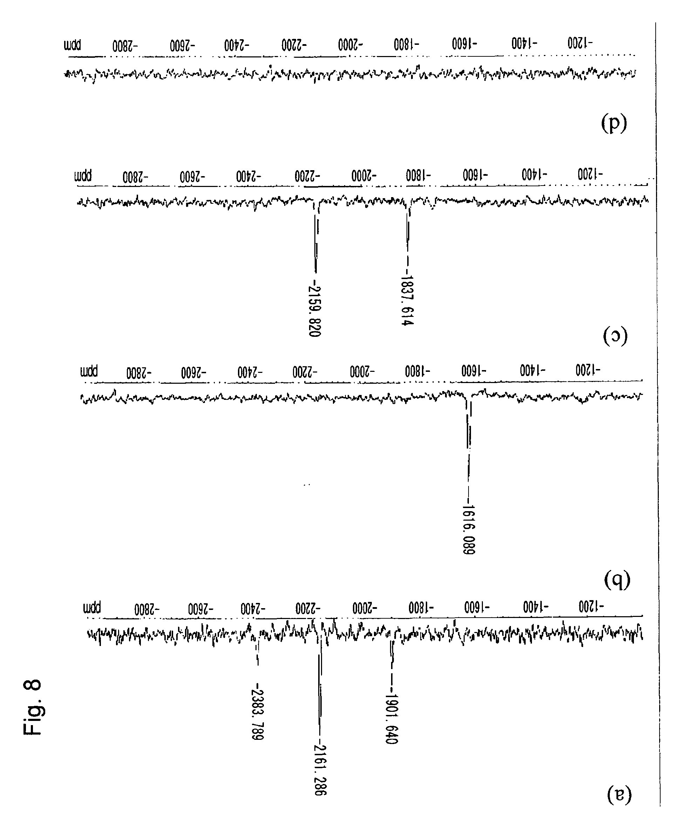

As shown in Figs. 8a and b, the chemical shift of cis-diamine dichloroplatinum (II), when dissolved in heavy water, was -2160 ppm, and that of cis-diammine dinitratoplatinum (II), when dissolved in heavy water, was -1620 ppm. The chemical shift agreed well with the data in literature (B. Rosenberg, Biochimie., 60859 (1978)). As shown in Fig. 8c, the chemical shift of cis-diammine dinitratoplatinum (II)-encapsulating liposome II, when the external solution was replaced with 150 mM sodium chloride-containing TAPS (pH: 8.4) buffer solution and the solution was left at 25° C for 96 hours, was -2160 ppm, which was similar to the chemical shift in the spectrum of cis-diamine dichloroplatinum (II) shown in Fig. 8a. The results demonstrated that cis-diammine dinitratoplatinum (II) contained in the liposome II was converted to cis-diamine dichloroplatinum (II). When cis-diammine dinitratoplatinum (II) was not changed to cis-diamine dichloroplatinum (II), there was no peak at the cis-diammine dinitratoplatinum (II) chemical shift of -1620 ppm or at the cis-diamine dichloroplatinum (II) chemical shift of -2160 ppm (Fig. 8d).

As shown in Figs. 5a to d and Example 3, the liposome I gave similar results, confirming that cis-diammine dinitratoplatinum (II) encapsulated in the liposome was converted to cis-diamine dichloroplatinum (II).

In analysis by ¹⁹⁵Pt-NMR method of liposome I (improved cholate method) or liposome II (freeze drying method), there was no peak at the cis-diammine dinitratoplatinum (II) chemical shift of -1620 ppm, but there was a peak observed at the cis-diamine dichloroplatinum (II) chemical shift of -2160 ppm (Fig.s 5c and 8c). The results confirms that cis-diammine dinitratoplatinum (II) in any liposome was changed in structure to cis-diamine dichloroplatinum (II).

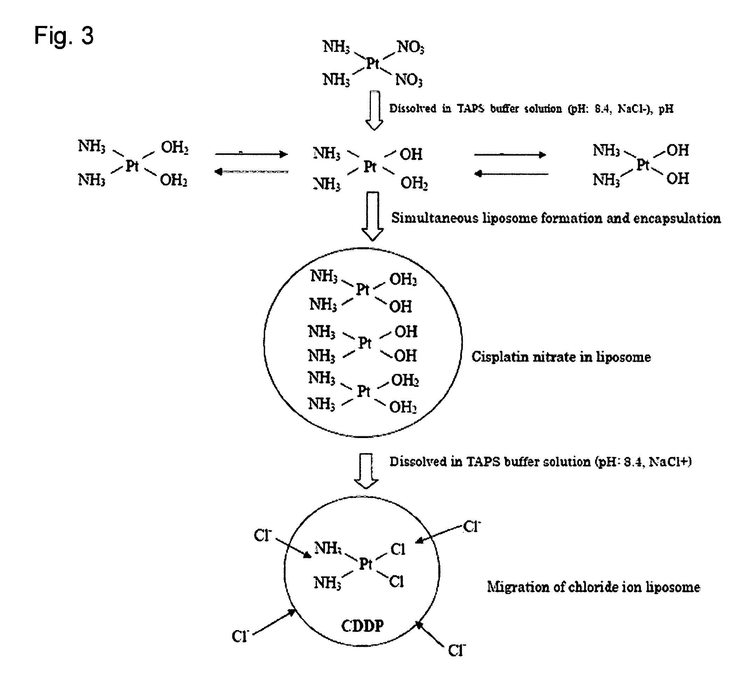

As shown in Figs. 5d and 8d, when the liposome-encapsulated cis-diammine dinitratoplatinum (II) was not converted to cis-diamine dichloroplatinum (II), there was no observed peak at the cis-diammine dinitratoplatinum (II) chemical shift of -1620 ppm or at the cis-diamine dichloroplatinum (II) chemical shift. As shown in Fig. 3, the chemical shift was not detected, probably because the cis-diammine dinitratoplatinum (II) contained was present in a number of molecular species in coordination with water molecules and also in mild interaction with liposome membrane components.

The present invention has been described so far with reference to preferable embodiments, but it should be understood that the scope of the present invention is not restricted by these embodiments but restricted only by the claims. The description in the patents, patent applications and literatures cited herein should be herein incorporated by reference, as described specifically herein.

### INDUSTRIAL APPLICABILITY

The present invention has an advantage that it is possible to encapsulate a poorly water-soluble ammine platinum complex, which could not be encapsulated or only encapsulated at low incorporation efficiency, in liposome efficiently. Thus, the present invention provides an advantage that it is possible to use a poorly water-soluble ammine platinum complex, which prohibited oral administration, as a liposome preparation.

## Claims

1. A method for producing a liposome encapsulating an ammine platinum complex, comprising the following steps of:
A) providing a water-soluble ammine platinum complex, a platinum complex raw material or a combination thereof;
B) providing a liposome, a liposome raw material or a combination thereof; and
C) preparing a mixture of the water-soluble ammine platinum complex, the platinum complex raw material or the combination thereof and the liposome, the liposome raw material or the combination thereof and subjecting the mixture to a liposome-forming/maintaining condition, wherein
a salt formed by the platinum complex is in a water-soluble form at the time point where the liposome exists.

2. The method according to Claim 1, further comprising the step of D) subjecting the mixture obtained in said step C) in the presence of a solution containing an ion forming a poorly water-soluble salt with said platinum complex.

3. The method according to Claim 1, wherein said water-soluble ammine platinum complex has two ammine groups.

4. The method according to Claim 3, wherein said water-soluble ammine platinum complex is cis-diammine dinitratoplatinum (II).

5. The method according to Claim 1, comprising the steps of:
A) dissolving said water-soluble ammine platinum complex in a first buffer solution and thus preparing a platinum complex solution;
B) providing said liposome raw material, comprising the steps of:
B-i) obtaining a lipid membrane by suspending a liposome-forming lipid in a methanol-chloroform solution under agitation, evaporating the agitated solution, and vacuum-drying a precipitate; and
B-ii) suspending the lipid membrane in a second buffer solution and forming a lipid suspension; and
C) mixing the platinum complex solution with the lipid suspension and subjecting the mixture under said liposome-forming/maintaining condition, wherein
the first and second buffer solutions do not contain any ion forming a poorly water-soluble salt with the platinum complex.

6. The method according to Claim 5, further comprising the step of ultrasonicating said lipid suspension after the step B-ii).

7. The method according to Claim 1, wherein the liposome-forming/maintaining condition in step C) is a condition selected from the group consisting of subjecting the mixture of the platinum complex solution and the lipid suspension to ultrafiltration and leaving the mixture to stand overnight.

8. The method according to Claim 1, comprising the steps of:
A) dissolving said water-soluble ammine platinum complex in a first buffer solution and thus preparing platinum complex solution;
B) providing said liposome; and
C) mixing the platinum complex solution with the liposome and subjecting the mixture under the liposome-forming/maintaining condition, wherein
the first buffer solution does not contain any ion forming a poorly water-soluble salt with the platinum complex.

9. The method according to Claim 1, wherein said liposome in said step B) has a composition sufficient for permeating said water-soluble ammine platinum complex through a liposomal membrane into the liposome and remaining therein.

10. The method according to Claim 1, wherein said liposome-forming/maintaining condition is a condition sufficient for preserving said liposome without destruction, and at the same time for penetrating the water-soluble ammine platinum complex through the liposomal membrane into the liposome and remaining therein.

11. The method according to Claim 1, wherein the lipid constituting said liposome or said liposome raw material are selected from the group consisting of dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, dipalmitoyl phosphatidylglycerol and combinations thereof.

12. The method according to Claim 1, wherein said mixture in the step C) is adjusted to a pH in a range of 6 to 10.

13. The method according to Claim 1, wherein the condition in which the salt formed by said platinum complex is in a water-soluble form is that the mixture is subjected in the presence of a solution which does not contain any ion forming a poor water-soluble salt with the platinum complex that is selected from the group consisting of a chloride ion (Cl⁻), a bromide ion (Br⁻), an iodide ion (I⁻), a thiocyanate ion (SCN⁻) and a cyanide ion (CN⁻).

14. The method according to Claim 13, wherein said chloride ion (Cl⁻) is contained in a range of 0 to 4 mM.

15. The method according to Claim 1, wherein said mixture in said step C) contains a buffering agent selected from the group consisting of an N-tris(hydroxymethyl)-3-aminopropane sulfonate buffering agent, a carbonate buffering agent, a phosphate buffering agent, a 2-[4-(2-hydroxyethyl)-1-piperadinyl]ethane sulfonate buffering agent, a tris(hydroxy)aminomethane buffering agent, a 3-(N-morpholino)propane sulfonate buffering agent, an N-tris(hydroxymethyl)1-2-aminoethane sulfonate buffering agent, an N-2-hydroxyethylpiperazine-N'-2-ethane sulfonate buffering agent, an N-tris(hydroxymethyl)methyl-2-hydroxy-3-aminopropane sulfonate buffering agent, a piperazine-N,N'-bis(2-hydroxypropanesulfonic acid) buffering agent, an N-2-hydroxyethylpiperazine-N'-2-hydroxypropane-3-propane sulfonate buffering agent, a tris(hydroxymethylmethylglycine) buffering agent, an N,N-bis(2-hydroxyethyl)glycine buffering agent, a 2-(cyclohexylamino)propylethane sulfonate buffering agent, a 3-N-cyclohexylamino-2-hydroxypropane sulfonate buffering agent, a 3-cyclohexylaminopropane sulfonate buffering agent and combinations thereof.

16. The method according to Claim 1, wherein said water-soluble ammine platinum complex, the platinum complex raw material or the combination thereof and said liposome, the liposome raw material or the combination thereof in said step C) are mixed at a ratio in a range of 1:9 to 9:1.

17. The method according to Claim 2, wherein the ion forming a poorly water-soluble salt with said platinum complex in said step D) is selected from the group consisting of a chloride ion (Cl⁻), a bromide ion (Br⁻), an iodide ion (I⁻), a thiocyanate ion (SCN⁻) and a cyanide ion (CN⁻).

18. The method according to Claim 17, wherein the ion forming a poorly water-soluble salt with said platinum complex is a chloride ion (Cl⁻).

19. The method according to Claim 18, wherein said chloride ion (Cl⁻) is provided from NaCl, HCl or CaCl₂.

20. The method according to Claim 2, wherein the ion forming a poorly water-soluble salt with said platinum complex in said step D) is provided from a buffer solution selected from the group consisting of an N-tris(hydroxymethyl)-3-aminopropane sulfonate buffer solution, a carbonate buffer solution, phosphate buffer, a 2-[4-(2-hydroxyethyl)-1-piperadinyl]ethane sulfonate buffer solution, a tris(hydroxy)aminomethane buffer solution, a 3-(N-morpholino)propane sulfonate buffer solution, an N-tris(hydroxymethyl)methyl-2-aminoethane sulfonate buffer solution, an N-2-hydroxyethylpiperazine-N'-2ethane sulfonate buffer solution, an N-tris(hydroxymethyl)methyl-2-hydroxy-3-aminopropane sulfonate buffer solution, a piperazine-N,N'-bis(2-hydroxypropanesulfonic acid) buffer solution, an N-2-hydroxyethylpiperazine-N'-2-hydroxypropane-3-propane sulfonate buffer solution, a tris(hydroxymethylmethylglycine) buffer solution, an N,N-bis(2-hydroxyethyl)glycine buffer solution, a 2-(cyclohexylamino)propylethane sulfonate buffer solution, a 3-N-cyclohexylamino-2-hydroxypropane sulfonate buffer solution and a 3-cyclohexylaminopropane sulfonate buffer solution.

21. The method according to Claim 18, wherein said chloride ion (Cl⁻) is provided from a buffer solution selected from the group consisting of an N-tris(hydroxymethyl)-3-aminopropane sulfonate buffer solution (pH: 8.4), a carbonate buffer solution (pH: 8.5), phosphate buffer (pH: 8.0) and a 2-[4-(2-hydroxyethyl)-1-piperadinyl]ethane sulfonate buffer solution (pH: 7.2).

22. The method according to Claim 2, wherein said step D) further comprises the steps of:
i) subjecting the liposome formed to hydrophilization treatment;
ii) binding a target specific substance to the liposome;
iii) hydrophilizing the modified target specific substance-bound liposome; and
iv) filtering a solution containing the hydrophilized liposome.

23. The method according to Claim 22, wherein said target specific substance is selected from the group consisting of antibodies, sugar chains, lectins, complementary nucleic acids, receptors, ligands, aptamers and antigens.

24. The method according to Claim 1, wherein said ammine platinum complex is poorly water-soluble.

25. A method for producing a liposome encapsulating cis-diamine dichloroplatinum (II) according to Claim 1, comprising the following steps of:
(A1) dissolving the cis-diammine dinitratoplatinum (II) in an N-tris(hydroxymethyl)-3-aminopropane sulfonate buffer solution to give a solution containing the cis-diammine dinitratoplatinum (II), wherein the N-tris(hydroxymethyl)-3-aminopropane sulfonate buffer solution does not contain a chloride ion (Cl⁻) or contains a chloride ion (Cl⁻) in a range of 0 to 4 mM;
(A2) adjusting the pH of the solution containing the cis-diammine dinitratoplatinum (II) to 6 to 10;
(B1) preparing a lipid by mixing dipalmitoyl phosphatidylcholine, cholesterol, ganglioside, dicetyl phosphate, dipalmitoyl phosphatidylethanolamine and sodium cholate;
(B2) suspending the lipid in a methanol-chloroform solution under agitation, evaporating the agitated solution and vacuum-drying a precipitate to give a lipid membrane;
(B3) suspending the lipid membrane in an N-tris(hydroxymethyl)-3-aminopropane sulfonate buffer solution (pH: 6 to 10) to prepare a lipid suspension, wherein the N-tris(hydroxymethyl)-3-aminopropane sulfonate buffer solution does not contain a chloride ion (Cl⁻) or contains a chloride ion (Cl⁻) in a range of 0 to 4 mM;
(B4) agitating the lipid suspension at 30°C to 40°C and then ultrasonicating the suspension after nitrogen substitution; and
(C1) mixing the pH-adjusted cis-diammine dinitratoplatinum (II) solution and the ultrasonicated lipid suspension at a ratio in a range of 1:9 to 9:1 and subjecting the mixture to ultrafiltration at a molecular weight cutoff of 500 to 300,000.

26. A method for producing a liposome encapsulating cis-diamine dichloroplatinum (II) according to Claim 1, comprising the following steps of:
(A1) dissolving the cis-diammine dinitratoplatinum (II) in an N-tris(hydroxymethyl)-3-aminopropane sulfonate buffer solution to give a solution containing the cis-diammine dinitratoplatinum (II), wherein the N-tris(hydroxymethyl)-3-aminopropane sulfonate buffer solution does not contain a chloride ion (Cl⁻) or contains a chloride ion (Cl⁻) in a range of 0 to 4 mM;
(A2) adjusting the pH of the solution containing the cis-diammine dinitratoplatinum (II) to 8.4;
(B1) providing a liposome; and
(C1) mixing the pH-adjusted cis-diammine dinitratoplatinum (II) solution and the liposome at a ratio in a range of 1:9 to 9: 1 and subjecting the mixture to ultrafiltration at a molecular weight cutoff of 500 to 300,000.

27. A liposome encapsulating an ammonia-containing ammine platinum complex, wherein the liposome can be produced by mixing a water-soluble ammine platinum complex, a platinum complex raw material or a combination thereof and a liposome, a liposome raw material or a combination thereof and subjecting the mixture to the liposome-forming/maintaining condition.

28. The liposome according to Claim 27, wherein the liposome can be produced by mixing said water-soluble ammine platinum complex and said liposome and subjecting the mixture to the liposome-forming/maintaining condition.

29. The liposome according to Claim 27, wherein the liposome can be produced by mixing said water-soluble ammine platinum complex and said liposome raw material and subjecting the mixture to the liposome-forming/maintaining condition.

30. A liposome encapsulating an ammine platinum complex, wherein an ammonia-containing ammine platinum complex is contained in an amount of 0.3 µg or more per mg of the lipid.

31. The liposome according to Claim 30, wherein said ammonia-containing ammine platinum complex is contained in an amount of 9 µg or more per mg of the lipid.

32. The liposome according to Claim 30, wherein said ammonia-containing ammine platinum complex is contained in an amount of 17 µg or more per mg of the lipid.

33. A liposome encapsulating an ammine platinum complex, wherein an ammonia-containing ammine platinum complex is contained in an amount of 100 µg or more per mg of the phospholipid.

34. A liposome encapsulating an ammine platinum complex, wherein an ammonia-containing ammine platinum complex is contained in an amount of 39 µg or more per mg of the liposome.

35. A liposome encapsulating an ammine platinum complex, wherein an ammonia-containing ammine platinum complex is contained in an amount of 3×10⁻¹⁰ µg or more per liposome.

36. The liposome according to Claim 31, wherein said ammine platinum complex has two ammonia molecules.

37. The liposome according to Claim 31, wherein said ammine platinum complex is cis-diamine dichloroplatinum (II) in a water-soluble form.

38. The liposome according to Claim 31, wherein said ammine platinum complex is cis-diamine dichloroplatinum (II).

39. The liposome according to Claim 31, wherein said liposome encapsulating an ammine platinum complex comprises lipids selected from the group consisting of dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, dipalmitoyl phosphatidylcholine, dipalmitoyl phosphatidylglycerol and combinations thereof.

40. The liposome according to Claim 31, wherein said liposome encapsulating an ammine platinum complex further comprises a target specific substance.

41. The liposome according to Claim 40, wherein said target specific substance is selected from the group consisting of antibodies, sugar chains, lectins, complementary nucleic acids, receptors, ligands, aptamers and antigens.

42. A composition for treatment of a cancer or a tumor, comprising a liposome encapsulating an ammonia-containing ammine platinum complex, and the liposome is produced by mixing a water-soluble ammine platinum complex, a platinum complex raw material or a combination thereof and a liposome, a liposome raw material or a combination thereof and subjecting the mixture to the liposome-forming/maintaining condition.

43. A composition for treatment of a cancer or a tumor, comprising a liposome encapsulating an ammonia-containing ammine platinum complex, in which the ammine platinum complex is contained in an amount of 0.3 µg or more per mg of the lipid.

44. The composition according to Claim 43, wherein said ammine platinum complex has two ammonia molecules.

45. The composition according to Claim 44, wherein said ammine platinum complex is cis-diamine dichloroplatinum (II) in a water-soluble form.

46. The composition according to Claim 44, wherein said ammine platinum complex is cis-diamine dichloroplatinum (II).

47. The composition according to Claim 43, wherein said liposome encapsulating an ammine platinum complex comprises lipids selected from the group consisting of dipalmitoyl phosphatidylcholine, cholesterol, ganglioside, dicetyl phosphate, dipalmitoyl phosphatidylethanolamine, sodium cholate, dicetyl phosphatidylethanolamine-polyglycerin 8G, palmitoyl phosphatidylcholine, dipalmitoyl phosphatidylglycerol and combinations thereof.

48. The composition according to Claim 43, wherein said liposome encapsulating an ammine platinum complex further comprises a target specific substance.

49. The composition according to Claim 48, wherein said target specific substance is selected from the group consisting of antibodies, sugar chains, lectins, complementary nucleic acids, receptors, ligands, aptamers and antigens.

50. A method of treating a cancer or a tumor, comprising administering a liposome encapsulating an ammonia-containing ammine platinum complex to a mammal in need of treating a cancer or a tumor in an effective amount of treating thereof, wherein the liposome is prepared by mixing a water-soluble ammine platinum complex, a platinum complex raw material or a combination thereof and a liposome, a liposome raw material or a combination thereof and subjecting the mixture under the liposome-forming/maintaining condition.

51. A method of treating a cancer or a tumor, comprising administering a liposome encapsulating an ammonia-containing ammine platinum complex to a mammal in need of treating a cancer or a tumor in an effective amount of treating thereof, wherein the ammine platinum complex in the liposome is contained in an amount of 0.3 µg or more per mg of the lipid.

52. The method according to Claim 51, wherein said liposome encapsulating an ammonia-containing ammine platinum complex is administered intravenously, subcutaneously, orally, locally, or intraperitoneally.

53. The method according to Claim 51, wherein said liposome encapsulating an ammonia-containing ammine platinum complex is administered at a dose of 1.5 µg/g body weight of the platinum complex.

54. The method according to Claim 51, wherein said cancer or tumor is selected from the group consisting of testis tumors, bladder cancers , pelvis renalis tumors, ureter tumors, prostate cancers, ovarian cancers, head and neck cancers, non-small cell lung cancers, esophageal cancers, uterine cancers, neuroblastoma and stomach cancers.

55. The method according to Claim 51, wherein said liposome encapsulating an ammine platinum complex further comprises a target specific substance.

56. The method according to Claim 55, wherein said target specific substance is selected from the group consisting of antibodies, sugar chains, lectins, complementary nucleic acids, receptors, ligands, aptamers and antigens.

57. A liposome encapsulating an ammonia-containing ammine platinum complex for use as a medicament, wherein the liposome is prepared by mixing a water-soluble ammine platinum complex, a platinum complex raw material or a combination thereof and a liposome, a liposome raw material or a combination thereof and subjecting the mixture to the liposome-forming/maintaining condition.

58. A liposome encapsulating an ammonia-containing ammine platinum complex for use as a medicament, wherein the ammine platinum complex is contained 0.3 µg or more per mg of the lipid.
